# NEUE EUROPÄISCHE PATENTSCHRIFT

(11) **EP 0 309 402 B2**
(45) Veröffentlichungstag und Bekanntmachung des Hinweises auf die Entscheidung über den Einspruch: **12.06.2002**
(45) Hinweis auf die Patenterteilung: 11.01.1995
(21) Anmeldenummer: 88810623.4
(22) Anmeldetag: 13.09.1988
(51) Int. Cl.: C08K 5/34, C08K 5/35, C09D 7/12, C07D 519/00, C07D 491/22, C07D 471/10, C07D 401/12, C07D 401/14, C07D 211/94

(54) **N-substituierte sterisch gehinderte Amin-Stabilisatoren**
N-substituted hindered amine stabilizers
Stabilisateurs à base d'amines avec empêchement stérique N-substituées

(30) Priorität: 21.09.1987 US 99411; 21.09.1987 US 99414; 21.09.1987 US 99420
(43) Veröffentlichungstag der Anmeldung: 29.03.1989
(73) Patentinhaber: Ciba Specialty Chemicals Holding Inc., 4002 Basel (CH)
(72) Erfinder: Winter, Roland A.E., Armonk New York 10504 (US); Galbo, James P., Hartsdale New York 10530 (US); Seltzer, Raymond, New City New York 10956 (US); Behrens, Rudolf A., New Fairfield Connecticut 06812 (US); Mar, Andrew, Norwalk Connecticut 06851 (US); Schirmann, Peter J., Fairfield Connecticut 06430 (US); Malherbe, Roger F., Dr., CH-4058 Basle (CH)

(56) Entgegenhaltungen:
- EP-A- 0 209 127
- US-A- 4 315 848
- US-A- 4 344 876
- US-A- 4 426 471
- US-A- 4 426 472
- US-A- 4 465 757
- US-A- 4 472 547
- US-A- 4 547 537
- US-A- 4 581 429
- US-A- 4 605 743
- US-A- 4 665 185
- US-A- 4 668 721
- JOURNAL OF POLYMER SCIENCE, POLYMER CHEMISTRY EDITION, vol. 22, no. 1, January 1984, pages 277-281, John Wiley & Sons, Inc.; T. KURUMADA et al.: "The effect of N-substituents of hindered amine onphoto-oxidation of polypropylene"
- Developments in Polymer Stabilization -3 Pgs. 130-134 (1980)
- Int. Conf. Advances in the Stabilization Pgs. 227-235 (1986)
- Journal of Polymer Science, Vol. 16, No.10 (1978)
- J. Org. Chem., Vol 43, No.22, 1978
- J. Org. Chem., Vol. 36, No.1, 1971
- Surface Coatings - Vol. 1, 1974, page 95, paragraph 9.7.3
- "American Paint & Coatings Journal", August 14, 1989, pages 40, 41
- "Light and Heat Stabilization of Coatings" published by Ciba-Geigy Corporation in 1985 ( see last page of publication date).
- The paper "Photo-stabilisation and Photo-degradation of Organic Coatings Containing a Hindered Amine Light Stabiliser: Part III - Kinetics of Stabilisation During Free Radical Oxidation" by Bauer & Gerlock in Polymer Degradation and Stability, 14, (1986) pages 97 - 112
- "A Manual of Resins for Surface Coatings", Vol.II, 1987, pages 56-57, 60-61, 65 and 69
- Ullmann, 5. Auflage, A18, S.367;
- CRC Handbook of Chemistry and Physics, 61st. Edn. 1980-1981, F-97;
- Beyer/Walter, Lehrbuch der Organischen Chemie, 20. Auflage 1984, 243-244
- G. Wade, Organic Chemistry, 2nd Edn. 1991, 910;
- L. J. Calbo (Ed.), Coatings Additives Handbook, Marcel Dekker Inc., New York 1986, S. 256-257
- "Plastics Additives Handbook", by Gächter & Müller, 1985, page 150.

## Beschreibung

The instant invention pertains to the stabilization of ambient curing and of acid catalyzed thermosetting resins by use of hindered amine light stabilizers substituted on the hindered nitrogen atom by a variety of OR₁ groups. The invention further pertains to the new compounds of this type of hindered amines.

Hindered amine light stabilizers are well known to be effective in stabilizing a host of organic substrates including polymers from the deleterious effects of light and oxygen.

Such hindered amine light stabilizers have been used in the stabilization of hot-crosslinkable alkyd or acrylic metallic stoving lacquers (US 4,426,472) and in stabilizing acid-catalyzed stoving lacquers based on hot-crosslinkable acrylic polyester or alkyd resins (US 4,344,876 and 4,426,471). None of the hindered amine light stabilizers of these patents possess structures having an OR₁ group substituted directly on the N-atom of the hindered amine.

Related hindered amine stabilizers have been utilized individually and in combination with ultra-violet light absorbers to improve the performance characterisitcs of coating systems. Notwithstanding such improvements, there still exists a need to further retard the photooxidation and photodegradation of such coating systems and thereby provide increased effectiveness by maintaining the physical integrity of the coatings. Such effectiveness can be manifested by prevention of embrittlement, cracking, corrosion, erosion, loss of gloss, chalking and yellowing of the coating.

It has now been determined that the aforementioned improvements can be achieved by substitution on the hindered N-atom of the hindered amines with OR₁ groups and the utilization of such derivatives in ambient curable and acid catalyzed thermosetting coating systems. In particular, the physical integrity of the coatings is maintained to a higher degree with significant reduction in loss of gloss and yellowing. Accordingly, the present invention relates to a stabilized ambient curing or acid catalyzed thermosetting coating composition containing an effective stabilizing amount of a hindered amine compound containing the group 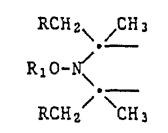 wherein R is hydrogen or methyl and R₁ is C₁-C₁₈ alkyl, C₂-C₁₈ alkenyl, C₂-C₁₈ alkinyl, C₅-C₁₂ cycloalkyl, C₆-C₁₀ bicycloalkyl, C₅-C₈ cycloalkenyl, C₆-C₁₀ aryl, C₇-C₉ aralkyl or C₇-C₉ aralkyl substituted by C₁-C₄ alkyl or phenyl.

More particularly, the instant invention relates to a derivative having one of formulae A to N 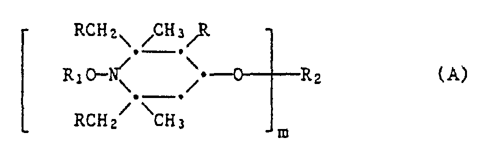 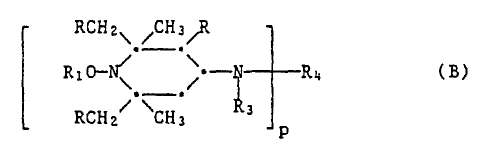 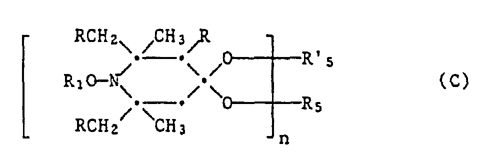 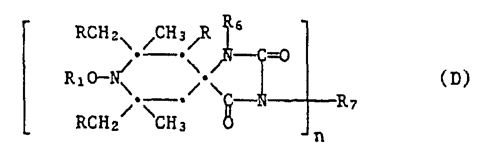 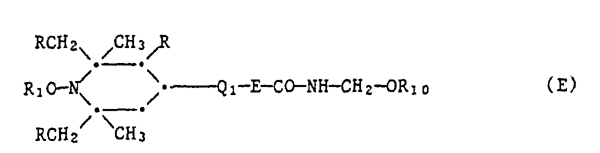 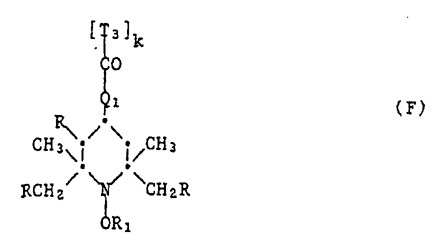 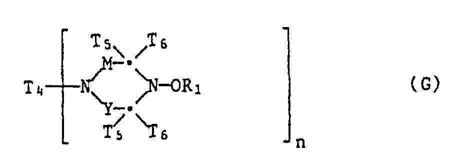 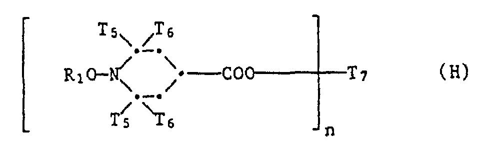 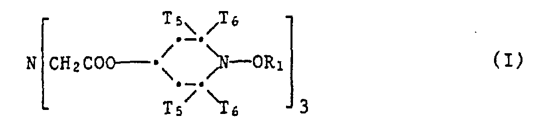 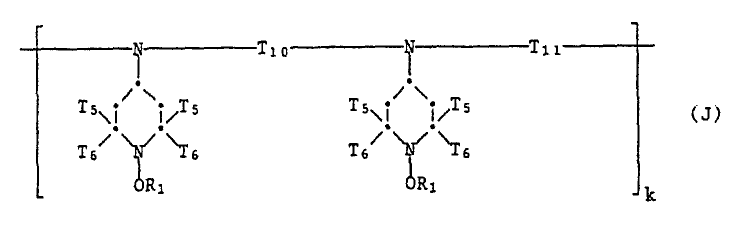 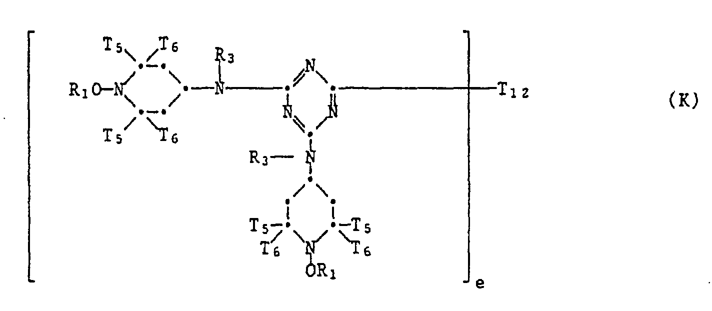 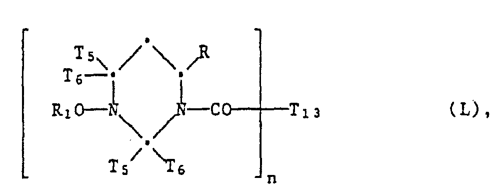 or 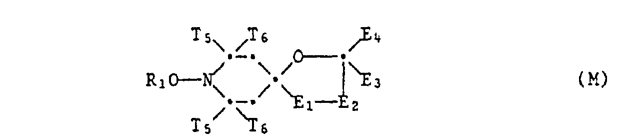 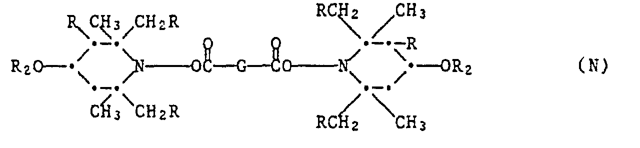 wherein
R is hydrogen or methyl,
R₁ is C₁-C₁₈ alkyl, C₂-C₁₈ alkenyl, C₂-C₁₈ alkinyl, C₅-C₁₂ cycloalkyl, C₆-C₁₀ bicycloalkyl, C₅-C₈ cycloalkenyl, C₆-C₁₀ aryl, C₇-C₉ aralkyl or C₇-C₉ aralkyl substituted by C₁-C₄ alkyl or phenyl;
m is 1-4,
when m is 1,
R₂ is hydrogen, C₁-C₁₈ alkyl optionally interrrupted by one or more oxygen atoms, C₂-C₁₂ alkenyl, C₆-C₁₀ aryl, C₇-C₁₈ aralkyl, glycidyl, a monovalent acyl radical of an aliphatic, cycloaliphatic, araliphatic or aromatic carboxylic acid, or of a carbamic acid, preferably an acyl radical of an aliphatic carboxylic acid having 2-18 C atoms, of a cycloaliphatic carboxylic acid having 5-12 C atoms or of an aromatic carboxylic acid having 7-15 C atoms; or R₂ is a group 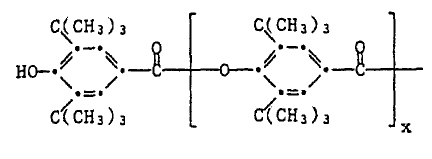 wherein x is 0 or 1, or is a group 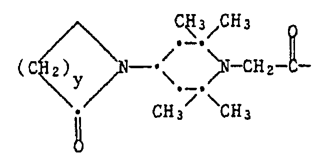 wherein y is 2-4;
when m is 2,
R₂ is C₁-C₁₂ alkylene, C₄-C₁₂ alkenylene, xylylene, a divalent acyl radical of an aliphatic, cycloaliphatic, araliphatic or aromatic dicarboxylic acid or of a dicarbamic acid, preferably an acyl radical of an aliphatic dicarboxylic acid having 2-18 C atoms, of a cycloaliphatic or aromatic dicarboxylic acid having 8-14 C atoms, or of an aliphatic, cycloaliphatic or aromatic dicarbamic acid having 8-14 C atoms; or R₂ is a group 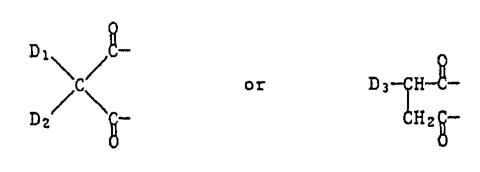 wherein D₁ and D₂ are
independently hydrogen, alkyl containing up to 8 carbon atoms, phenyl, benzyl or 3 5-di-t-butyl-4-hydroxybenzyl, D₃ is an alkyl or alkenyl radical containing up to 18 carbon atoms;
when m is 3, R₂ is a triavalent acyl radical of an aliphatic, cycloaliphatic, or aromatic tricarboxylic acid;
when m is 4, R₂ is a tetravalent acyl radical of an aliphatic or aromatic tetracarboxylic acid including 1,2,3,4-butanetetracarboxylic acid, 1,2,3,4-but-2-enetetracarboxylic acid, and 1,2,3,5- and 1,2,4,5-pentanetetracarboxylic acid;
p is 1, 2 oder 3,
R₃ is hydrogen, C₁-C₁₂ alkyl, C₅-C₇ cycloalkyl, C₇-C₉ aralkyl, C₂-C₁₈ alkanoyl, C₃-C₅ alkenoyl or benzoyl;
when p is 1,
R₄ is hydrogen, C₁-C₁₈ alkyl, C₅-C₇ cycloalkyl, C₂-C₈ alkenyl unsubstituted or substituted by a cyano, carbonyl or carbamide group, or it is aryl, aralkyl, glycidyl, a group of the formula -CH₂-CH(OH)-Z or -CONH-Z wherein Z is hydrogen, methyl or phenyl; or R₄ is a group of formula I 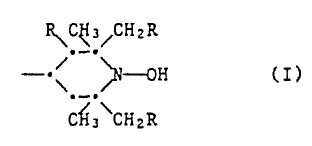 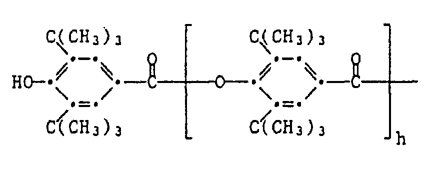 with h as 0 or 1;
or R₃ and R₄ together are alkylene of 4 to 6 carbon atoms or 1-oxo-alkylene or the divalent acyl radical of an aliphatic or aromatic 1,2- or 1,3-dicarboxylic acid,
when p is 2,
R₄ is C₂-C₁₂ alkylene, C₆-C₁₂ arylene, xylylene, a -CH₂CH(OH)-CH₂- group, or a group -CH₂-CH(OH)-CH₂-O-X-O-CH₂-CH(OH)-CH₂- wherein X is C₂-C₁₀ alkylene, C₆-C₁₅ arylene or C₆-C₁₂ cycloalkylene; or, provided that R₃ is not alkanoyl, alkenoyl or benzoyl, R₄ can also be a divalent acyl radical of an aliphatic, cycloaliphatic or aromatic dicarboxylic acid or dicarbamic acid, or can be the group -CO-; or R₄ is a group of formula II 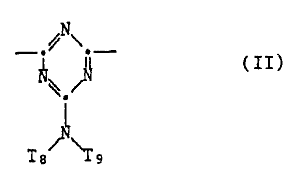 where T₈ and T₉ are independently hydrogen, alkyl of 1 to 18 carbon atoms or a group of formula I, or T₈ and T₉ together are alkylene of 4 to 6 carbon atoms or 3-oxapentamethylene, preferably T₈ and T₉ together are 3-oxapentamethylene;
when p is 3,
R₄ is 2,4,6-triazinetriyl,
n is 1 or 2 and
when n is 1,
R₅ and R'₅ are independently C₁-C₁₂ alkyl, C₂-C₁₂ alkenyl, C₇-C₁₂ aralkyl, or R₅ is also hydrogen, or R₅ and R'₅ together are C₂-C₈ alkylene or hydroxyalkylene or C₄-C₂₂ acyloxyalkylene;
when n is 2,
R₅ and R'₅ together are (-CH₂)₂C(CH₂-)₂;
R₆ is hydrogen, C₁-C₁₂ alkyl, allyl, benzyl, glycidyl or C₂-C₆ alkoxyalkyl;
when n is 1,
R₇ is hydrogen, C₁-C₁₂ alkyl, C₃-C₅ alkenyl, C₇-C₉ aralkyl, C₅-C₇ cycloalkyl, C₂-C₄ hydroxyalkyl, C₂-C₆ alkoxyalkyl, C₆-C₁₀ aryl, glycidyl, a group of the formula -(CH₂)ₜ-COO-Q or of the formula -(CH₂)ₜ-O-CO-Q
wherein t is 1 or 2, and Q is C₁-C₄ alkyl or phenyl; or
when n is 2,
R₇ is C₂-C₁₂ alkylene, C₆-C₁₂ arylene, a group -CH₂CH(OH)-CH₂-O-X-O-CH₂-CH(OH)-CH₂- wherein X is C₂-C₁₀ alkylene, C₆-C₁₅ arylene or C₆-C₁₂ cycloalkylene, or a group -CH₂CH(OZ')CH₂-(OCH₂-CH(OZ')-CH₂)₂- wherein Z' is hydrogen, C₁-C₁₈ alkyl, allyl, benzyl, C₂-C₁₂ alkanoyl or benzoyl;
Q₁ is -N(R₈)- or -O-;
E is C₁-C₃ alkylene, the group -CH₂-CH(R₉)-O- wherein R₉ is hydrogen, methyl or phenyl, or E is the group -(CH₂)₃-NH- or a direct bond;
R₁₀ is hydrogen or C₁-C₁₈ alkyl,
R₈ is hydrogen, C₁-C₁₈ alkyl, C₅-C₇ cycloalkyl, C₇-C₁₂ aralkyl, cyanoethyl, C₆-C₁₀ aryl, the group -CH₂-CH-(R₉)-OH wherein R₉ has the meaning defined above, a group of the formula I or a group of the formula 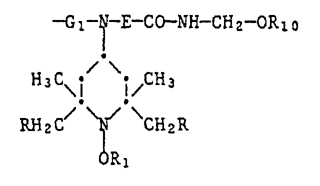 wherein G₁ can be C₂-C₆ alkylene or C₆-C₁₂ arylene, or R₈ is a group -E-CO-NH-CH₂-OR₁₀;
Formula F denotes a recurring structural unit of a polymer where T₃ is ethylene or 1,2-propylene, or is the repeating structural unit derived from an alpha-olefin copolymer with an alkyl acrylate or methacrylate;
preferably a copolymer of ethylene and ethyl acrylate, and where k is 2 to 100;
T₄ has the same meaning as R₄ when p is 1 or 2,
T₅ is methyl,
T₆ is methyl or ethyl, or T₅ and T₆ together are tetramethylene or pentamethylene, preferably T₅ and T₆ are each methyl,
M and Y are independently methylene or carbonyl, preferably M is methylene and Y is carbonyl, and T₄ is ethylene where n is 2;
T₇ is the same as R₇, and T₇ is preferably octamethylene where n is 2,
T₁₀ and T₁₁ are independently alkylene of 2 to 12 carbon atoms, or T₁₁ is a group of formula II;
e is 2, 3 or 4 and
T₁₂ is a group of formula -N(R₅)-(CH₂)_{d}-N(R₅)-
or 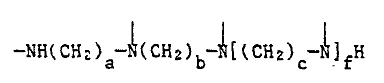 where a, b and c are independently 2 or 3, d is 2 to 10 and f is 0 or 1, preferably a and c are each 3, b is 2 and f is 1;
T₁₃ is the same as R₄ with the proviso that T₁₃ cannot be hydrogen when n is 1;
E₁ and E₂, being different, each are -CO- or -N(E₅)- where E₅ is hydrogen, C₁-C₁₂ alkyl or alkoxycarbonyl of 4 to 22 carbon atoms;
preferably E₁ is -CO- and E₂ is -N(R₅)-.
E₃ is hydrogen, alkyl of 1 to 30 carbon atoms, phenyl, naphthyl, said phenyl or said naphthyl substituted by chlorine or by alkyl of 1 to 4 carbon atoms, or phenylalkyl of 7 to 12 carbon atoms, or said phenylalkyl substituted by alkyl of 1 to 4 carbon atoms, and
E₄ is hydrogen, alkyl of 1 to 30 carbon atoms, phenyl, naphthyl or phenylalkyl of 7 to 12 carbon atoms, or E₃ and E₄ together are polymethylene of 4 to 17 carbon atoms, or said polymethylene substituted by up to four alkyl groups of 1 to 4 carbon atoms, preferably methyl; provided that in formula B' R₃ or R₄ are not hydrogen if R₁ is alkyl
R₂ of formula (N) is as previously defined when m is 1, G is a direct bond, C₁-C₁₂ alkylene, phenylene or -NH-G'-NH wherein G' is C₁-C₁₂ alkylene.

In the structures A to N, if any substituents are C₁-C₁₈ alkyl, they are for example methyl, ethyl, n-propyl, n-butyl, sec-butyl, tert-butyl, n-hexyl, n-octyl, 2-ethylhexyl, n-nonyl, n-decyl, n-undecyl, n-dodecyl, n-tridecyl, n-tetradecyl, n-hexadecyl or n-octadecyl.

If R₂ is a monovalent acyl radical of a carboxylic acid, it is for example an acyl radical of acetic acid, stearic acid, salicylic acid, methacrylic acid, benzoic acid or β-(3,5-di-tert-butyl-4-hydroxyphenyl) propionic acid.

If R₂ is a divalent acyl radical of a dicarboxylic acid, it is for example an acyl radical of adipic acid, succinic acid, suberic acid, sebacic acid, maleic acid, phthalic acid, dibutylmalonic acid, dibenzylmalonic acid or butyl-(3,5-di-tert-butyl-4-hydroxybenzyl)-malonic acid, or bicycloheptenedicarboxylic acid.

If R₂ is a divalent acyl radical of a dicarbamic acid, it is for example an acyl radical of hexamethylenedicarbamic acid or of 2,4-toluylenedicarbamic acid.

As C₇-C₉ aralkyl, R₃ is particularly phenethyl or above all benzyl.

As C₂-C₁₈ alkanoyl, R₃ is for example propionyl, butyryl, octanoyl, dodecanoyl, hexadecanoyl, octadecanoyl, but preferably acetyl; and as C₃-C₅ alkenoyl, R₃ is in particular acryloyl.

If R₄ is C₂-C₈ alkenyl unsubstituted or substituted by a cyano, carbonyl or carbamide group, it is for example 1-propenyl, allyl, methallyl, 2-butenyl, 2-pentenyl, 2-hexenyl, 2-octenyl, 2,2-dicyanovinyl, 1-methyl-2-cyano-2-methoxycarbonyl-vinyl or 2,2-diacetylaminovinyl.

If any substituents are C₂-C₁₂ alkylene, they are for example ethylene, propylene, 2,2-dimethylpropylene, tetramethylene, hexamethylene, octamethylene, decamethylene or dodecamethylene.

If any substituents are C₆-C₁₅ arylene, they are for example o-, m- or p-phenylene, 1,4-naphthylene or 4,4'-diphenylene.

As C₆-C₁₂ cycloalkylene, X is especially cyclohexylene.

If R₅ is C₂-C₈ alkylene or hydroxyalkylene, it is for example ethylene, 1-methyl-ethylene, propylene, 2-ethylpropylene or 2-ethyl-2-hydroxymethylpropylene.

As C₄-C₂₂ acyloxyalkylene, R₅ is for example 2-ethyl-2-acetoxymethylpropylene.

If any substituents are C₂-C₆ alkoxyalkyl, they are for example methoxymethyl, ethoxymethyl, propoxymethyl, tert-butoxyethyl, ethoxyethyl, ethoxypropyl, n-butoxyethyl, tert-butoxyethyl, isopropoxyethyl or propoxypropyl.

If R₇ is C₃-C₅ alkenyl, it is for example 1-propenyl, allyl, methallyl, 2-butenyl or 2-pentenyl.

As C₇-C₉ aralkyl, R₇ is in particular phenethyl or above all benzyl; and as C₅-C₇ cycloalkyl, R₇ is especially cyclohexyl.

If R₇ is C₂-C₄ hydroxyalkyl, it is for example 2-hydroxyethyl, 2-hydroxypropyl, 3-hydroxypropyl, 2-hydroxybutyl or 4-hydroxybutyl.

As C₆-C₁₀ aryl, R₇ is in particular phenyl, or alpha- or β-naphthyl which is unsubstituted or substituted by halogen or C₁-C₄ alkyl.

If R₇ is C₂-C₁₂ alkylene, it is for example ethylene, propylene, 2,2-dimethylpropylene, tetramethylene, hexamethylene, octamethylene, decamethylene or dodecamethylene.

If R₇ is C₆-C₁₂ arylene, it is for example o-, m- or p-phenylene, 1,4-naphthylene or 4,4'-diphenylene.

If Z' is C₂-C₁₂ alkanoyl, it is for example propionyl, butyryl, octanoyl, dodecanoyl or preferably acetyl.

As C₅-C₇ cycloalkyl, R₈ is in particular cyclohexyl.

As C₆-C₁₀ aryl, R₈ is particularly phenyl, or alpha- or β-naphthyl which is unsubstituted or substituted with halogen or C₁-C₄ alkyl.

As C₁-C₃ alkylene, E is for example methylene, ethylene or propylene.

As C₂-C₆ alkylene, G₁ is for example ethylene, propylene, 2,2-dimethylpropylene, tetramethylene or hexamethylene; and as C₆-C₁₂ arylene, G₁ is o-, m- or p-phenylene, 1,4-naphthylene or 4,4'-diphenylene.

The following compounds are examples of hindered amine derivatives applicable for use in the invention.

Preferred are compositions containing a compound of formula A, B, C, D, J, K or M wherein R is hydrogen and T₅ and T₆ are methyl.

Preferred are compositions containing a compound of formula A, B, C, J or K wherein R is hydrogen and R₁ is C₁-C₁₈ alkyl, C₆-C₁₂ cycloalkyl, cyclohexenyl or C₇-C₉ phenylalkyl.

Preferred are further compositions containing a compound of formula A wherein R is hydrogen and R₁ is C₁-C₁₈ alkyl, C₆-C₁₂ cycloalkyl, cyclohexenyl or C₇-C₉ phenylalkyl, m is 1, 2 or 4 and when m is 1, R₂ is C₁-C₁₂ alkyl, allyl, benzyl or an acyl radical of an aliphatic C₂-C₁₈ carboxylic acid, of a cycloaliphatic C₆-C₁₂ carboxylic acid or of an aromatic C₇-C₁₅ carboxylic acid, and when m is 2, R₂ is C₁-C₈ alkylene, butylene, xylylene or is a divalent acyl radical of an aliphatic C₂-C₁₈ dicarboxylic acid cycloaliphaticor of a cycloaliphatic or aromatic C₈-C₁₄ dicarboxylic acid, or of an aliphatic, cycloaliphatic or aromatic C₈-C₁₄ dicarbamic acid, or R₂ is a group 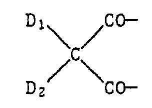 wherein D₁ C₁-C₈ alkyl or 3,5-di-tert.butyl-4-hydroxybenzyl and D₂ is D₁ or hydrogen and when m is 4, R₂ is a tetravalent acyl radical of a butane- or pentanetetracarboxylic acid, especially such compounds of formula A wherein R is hydrogen, R₁ is C₁-C₁₀ alkyl, cycloalkyl, cyclohexyl or C₇-C₉ phenylalkyl, m is 1 or 2, and when m is 1, R₂ is benzyl, C₂-C₁₈ alkanoyl, benzoyl, or 3,5-di-tert.butyl-4-hydroxybenzoyl, and when m is 2, R₂ is xylylene or a divalent acyl radical of an aliphatic C₄-C₁₀ dicarboxylic acid or of a benzene dicarboxylic acid.

Preferred are finally compositions containing a compound of formula B wherein R is hydrogen and R₁ is C₁-C₁₈ alkyl, C₆-C₁₂ cycloalkyl, cyclohexenyl or C₇-C₉ phenylalkyl, p is 1 or 2, R₃ is hydrogen, C₁-C₁₂ alkyl or C₂-C₁₂ alkanoyl, allyl, and when p is 1, R₄ is hydrogen, C₁-C₁₂ alkyl or a group of formula I, and when p is 2, R₄ is C₂-C₈ alkylene or xylylene and if R₃ is not alkanoyl, R₄ may also be a divalent acyl radical of an aliphatic C₄-C₁₀ dicarboxylic acid or of a benzene dicarboxylic acid or is a group of formula II wherein T₈ is hydrogen or C₁-C₄ alkyl and T₉ is C₁-C₁₂ alkyl or a group of formula I.
1. 1,4-dimethoxy-2,2,6,6-tetramethylpiperidine
2. 4-benzoyloxy-1-ethoxy-2,2,6,6-tetramethylpiperidine
3. di-(1-methoxy-2,2,6,6-tetramethylpiperidin-4-yl) sebacate
4. alpha,alpha'-(di-1-ethoxy-2,2,6,6-tetramethylpiperidin-4-yloxy)-p-xylene
5. di-(1-benzyloxy-2,2,6,6-tetramethylpiperidin-4-yl) phthalate
6. di-(1-benzyloxy-2,2,6,6-tetramethylpiperidin-4-yl) diethylmalonate
7. poly-{[6-1,1,3,3-tetramethylbutyl)-imino]-1,2,5-triazine-2,4-diyl] [2-(1-cyclohexyloxy-2,2,6,6-tetramethylpipendyl)-imino]-hexamethylene-[4-(1-cyclohexyloxy-2,2,6,6-tetramethylpiperidyl)-imino]}
8. (1-cyclohexyloxy-2,2,6,6-tetramethylpiperidin-4-yl) 3,5-di-t.butyl-4-hydroxybenzoate
9. 1-cyclohexyloxy-4-octadecanoyloxy-2,2,6,6-tetramethylpiperidine
10. di-(1-methoxy-2,2,6,6-tetramethylpiperidin-4-yl) succinate
11. di-(1-methoxy-2,2,6,6-tetramethylpiperidin-4-yl) isophthalate
12. di-(1-cyclohexyloxy-2,2,6,6-tetramethylpiperidin-4-yl) sebacate
13. di-(1-cyclohexyloxy-2,2,6,6-tetramethylpiperidin-4-yl) isophthalate
14. 4-benzyloxy-1-(alpha-methylbenzyloxy)-2,2,6,6-tetramethylpiperidine
15. di-[1-(alpha-methylbenzyloxy)-2,2,6,6-tetramethylpiperidin-4-yl] sebacate
16. di-(1-heptyloxy-2,2,6,6-tetramethylpiperidin-4-yl) sebacate
17. di-[1-(alpha-methylbenzyloxy)-2,2,6,6-tetramethylpiperidin-4-yl] terephthalate
18. di-(1-ethoxy-2,26,6-tetramethylpiperidin-4-yl) sebacate
19. di-(1-cumyloxy-2,2,6,6-tetramethylpiperidin-4-yl) sebacate
20. 3,15-di-alpha-methylbenzyloxy-2,2,4,4,14,14,16,16-octamethyl-7,11,18,21-tetraoxa-3,15-diazatrispiro-[5.2.2.5.2.2]heneicosane
21. 3,15-dicyclohexyloxy-2,2,4,4,14,1416,16-octamethyl-7,11,18,21-tetraoxa-3,15-diazatrispiro-[5.2.2.5.2.2]heneicosane
22. di-(1-cyclohexyloxy-2,2,6,6-tetramethylpiperidin-4-yl) succinate
23. di-[1-(alpha-methylbenzyloxy)-2,2,6,6-tetramethylpiperidin-4-yl] succinate
24. di-(1-octyloxy-2,2,6,6-tetramethylpiperidin-4-yl) sebacate
25. di-(1-octadecyloxy-2,2,6,6-tetramethylpiperidin-4-yl) sebacate
26. di-(1-nonyloxy-2,2,6,6-tetramethylpiperidin-4-yl) succinate
27. di[1-(1-methylcyclohexyloxy)-2,2,6,6-tetramethylpiperidin-4-yl] sebacate
28. di-[1-(3-cyclohexen-1-yloxy)-2,2,6,6-tetramethylpiperidin-4-yl] sebacate
29. di-(1-tert.butoxy-2,2,6,6-tetramethylpiperidin-4-yl) sebacate
30. di-[1-(bicyclo-[4.4.0]-decyl-1-oxy)-2,2,6,6-tetramethylpiperidin-4-yl] sebacate
31. 4-benzoyloxy-1-benzyloxy-2,2,6,6-tetramethylpiperidine
The coating compositions of this invention may be ambient curing or acid catalyzed hot curable systems, depending from the type of binder resins of the composition.

Resins for ambient curing coatings may be, for example, alkyd resins, thermoplastic acrylic resins, acrylic alkyd resins, polyurethane resins or polyester resins, said resins may be modified with silicones, isocyanates, epoxides, isocyanurates, ketimines or oxazolidines. The resins may be esters of cellulose such as nitrocellulose or cellulose acetobutyrate or the resins may be epoxide resins hardenable with polyamines or other hardeners.

Applicable alkyd, acrylic, polyester and epoxide resins are described in S. Paul's "Surface Coatings: Science and Technology" (1985) at pages 70-310. The unmodified and modified alkyd resins which can be stabilized in accordance with the invention, are the conventional resins which are used in trade sales, maintenance and automotive refinish coatings. For example, such coatings are based on alkyd resins, alkyd/acrylic resins and alkyd/silicon resins optionally crosslinked by isocyanates or epoxy resins.

Resins for acid catalyzed thermosetting coatings may be, for example, hot crosslinkable acrylic, polyester, polyurethane, polyamide or alkyd resins. This implies mixtures of those resins or mixtures with crosslinking agents such as melamine resins.

The acrylic resin lacquers are the conventional acrylic resin stoving lacquers or thermosetting resins including acrylic/melamine systems which are described, for example, in H. Kittel's "Lehrbuch der Lacke und Beschichtungen", Vol. 1, Part 2, on pages 735 and 742 (Berlin 1972), "Lackkunstharze" (1977), by H. Wagner and H.F. Sarx, on pages 229-238, and in S. Paul's "Surface Coatings: Science and Technology" (1985).

The polyester lacquers are the conventional stoving lacquers described e.g. in H. Wagner and H.F. Sarx, op. cit., on pages 86-99.

The alkyd resin lacquers which can be stabilized in accordance with the invention, are the conventional stoving lacquers which are used in particular for coating automobiles (automobile finishing lacquers), for example lacquers based on alkyd/melamine resins and alkyd/acrylic/melamine resins (see H. Wagner and H.F. Sarx, op. cit., pages 99-123). Other crosslinking agents include glycoluril resins, blocked isocyanates or epoxy resins.

In their industrial uses, enamels with high solids content based on crosslinkable acrylic, polyester, urethane or alkyd resins are cured with an additional acid catalyst. Light stabilizers containing a basic nitrogen group are generally less than satisfactory in this application. Formation of a salt between the acid catalyst and the light stabilizer leads to incompatibility or insolubility and precipitation of the salt and to a reduced level of cure and to reduced light protective action and poor resistance to moisture.

The ambient curing coatings as well as the acid catalyzed hot curable coatings stabilized in accordance with the invention are suitable both for metal finish coatings and solid shade finishes, especially in the case of retouching finishes. The lacquers stabilized in accordance with the invention are preferably applied in the conventional manner by two methods, either by the single-coat method or by the two-coat method. In the latter method, the pigment-containing base coat is applied first and a covering coat of clear lacquer applied over it.

The amount of hindered amine derivative employed is 0.1 to 10 % by weight, based on the solvent-free binder, preferably 0.5 to 5 % by weight. The binders can be dissolved or dispersed in customary organic solvents or in water or can be solvent-free.

When used in two-coat finishes, the hindered amine derivative can be incorporated in the clear coat or both in the clear coat and in the pigmented base coat. In the manufacture of acrylic modified alkyd resins or acrylic resins, polymerizable hindered amine derivatives can be polymerized into the resin. The incorporation into the lacquer binder can also, however, be effected via polycondensation in the manufacture of the alkyd or polyester resins. In these cases, there is the additional advantage that the light stabilizers cannot be removed by extraction or migration so that their action is very prolonged.

To attain maximum light stability, the concurrent use of other conventional light stabilizers can be advantageous. Examples are UV absorbers of the benzophenone, benzotriazole, acrylic acid derivative, or oxalanilide type, or aryl-s-triazines or metal-containing light stabilizers, for example organic nickel compounds. In two-coat systems, these additional light stabilizers can be added to the clear coat and/or the pigmented base coat.

If such combinations are employed, the sum of all light stabilizers is 0.2 to 20 % by weight, preferably 0.5 to 5 % by weight, based on the film-forming resin.

Examples of the UV absorbers which may be used in the instant compositions in conjunction with the aforementioned hindered amine compounds are:
(a) 2-(2'-Hydroxyphenyl)-benzotriazoles, for example the 5'-methyl-, 3',5'-di-tert-butyl-, 5'-tert-butyl-, 5'-(1,1,3,3-tetramethylbutyl)-, 5-chloro-3',5'-di-tert-butyl-, 5-chloro-3'-tert-butyl-5'-methyl-, 3'-sec-butyl-5'-tert-butyl-, 4'-octoxy-, 3',5'-di-tert-amyl derivative.
(b) 2-Hydroxy-benzophenones, for example, the 4-hydroxy-, 4-methoxy-, 4-octoxy-, 4-decyloxy-, 4-dodecyloxy-, 4-benzyloxy, 4,2',4'-trihydroxy- and 2'-hydroxy-4,4'-dimethoxy derivative.
(c) Acrylates, for example, alpha-cyano-β,β-diphenyl-acrylic acid ethyl ester or isoctyl ester, alpha-carbomethoxy-cinnamic acid methyl ester, alphacyano-β-methyl-p-methoxy-cinnamic acid methyl ester or butyl ester, alpha-carbomethoxy-p-methoxy-cinnamic acid methyl ester, N-(β-carbomethoxy-β-cyanovinyl)-2-methyl-indoline.
(d) Nickel compounds, for example, nickel complexes of 2,2'-thiobis-[4-(1,1,3,3-tetramethylbutyl)-phenol], such as the 1:1 or 1:2 complex, optionally with additional ligands such as n-butylamine, triethanolamine or N-cyclohexyl-di-ethanolamine, nickel dibutyldithiocarbamate, nickel salts of 4-hydroxy-3,5-di-tert-butyl-benzylphosphonic acid monoalkyl esters, such as of the methyl, ethyl or butyl ester, nickel complexes of ketoximes such as of 2-hydroxy-4-methyl-phenyl undecyl ketonoxime, nickel complexes of 1-phenyl-4-lauroyl-5-hydroxy-pyrazol, optionally with additional ligands.
(e) Oxalic acid diamides, for example, 4,4'-di-octyl-oxyoxanilide, 2,2'-di-octyloxy-5,5'-di-tert-butyl-oxanilide, 2,2'-di-dodecyloxy-5,5'-di-tert-butyl-oxanilide, 2-ethoxy-2'-ethyl-oxanilide, N,N'-bis-(3-dimethylaminopropyl)-oxalamide, 2-ethoxy-5-tert-butyl-2'-ethyl-oxanilide and its mixture with 2-ethoxy-2'-ethyl-5,4'-di-tert-butyl-oxanilide, and the mixtures of ortho- and paramethoxy- as well as of o- and p-ethoxy-disubstituted oxanilides.
(f) Hydroxyphenyl-5-triazines such as 2,6-bis-(2,4-di-methylphenyl)-4(2-hydroxy-4-octyloxyphenyl)-s-triazine or the corresponding 4(2,4-dihydroxyphenyl) derivatives.

Of particular value in the instant compositions are the benzotriazoles of high molecular weight and low volatility such as 2-[2-hydroxy-3,5-di(alpha,alpha-dimethylbenzyl)-phenyl]-benzotriazole, 2-(2-hydroxy-3,5-di-tert-octylphenyl]-benzotriazole, 2-(2-hydroxy-3-alpha,alpha-dimethyl-benzyl-5-tert-octylphenyl)-benzotriazole, 2-(2-hydroxy-3-tert-octyl-5-alpha,alpha-dimethylbenzylphenyl)-benzotriazole, 2-(2-hydroxy-3,5-di-tert-amylphenyl)-benzotriazole, 2-[2-hydroxy-3-tartbutyl-5-(2-(omega-hydroxy-octa-(ethylerieoxy)-carbonyl)-ethylphenyl]-benzotriazole, 5-chloro-2-[2-hydroxy-3,5-di(alpha,alpha-dimethylbenzyl)-phenyl]-benzotriazole, 5-chloro-2-(2-hydroxy-3,5-di-tert-butylphenyl)-benzotriazole, 5-chloro-2-[2-hydroxy-3-tert-butyl-5-(2-octyloxycarbonylethyl)-phenyl]-benzotriazole, 2-(2-hydroxy-3-sec-dodecyl-5-methyl-phenyl)-benzotriazole and hexamethylene di[β-(3-tert-butyl-4-hydroxy-5-[2-benzotriazolyl]-phenyl)-propionate].

Most preferably the benzotriazoles useful in the instant compositions are 2-[2-hydroxy-3,5-di-(alpha,alpha-dimethylbenzyl)-phenyl]-benzotriazoleand 2-[2-hydroxy-3-tert-butyl-5-(2-(omega-hydroxy-octa-(ethyleneoxy)-carbonyl)-ethylphenyl]-benzotriazole,
Further ingredients which the enamels or coatings can contain are antioxidants, for example those of the sterically hindered phenol derivatives, phosphorus compounds, such as phosphites, phosphines or phosphonites, plasticizers, levelling assistants, hardening catalysts, thickeners, dispersants or adhesion promoters.

Typical phosphite and phosphonites include triphenyl phosphite, diphenylalkyl phosphites, phenyldialkyl phosphites, tri-(nonylphenyl)phosphite, trilauryl phosphite, trioctadecyl phosphite, di-stearyl-pentaerythritol diphosphite, tris-(2,4-di-tert.butylphenyl) phosphite, di-isodecylpentaerythritol diphosphite, di-(2,4-di-tert.-butylphenyl)pentaerythritol diphosphite, tristearyl-sorbitol triphosphite, tetrakis-(2,4-di-tert.butylphenyl)-4,4'-diphenylylenediphosphonite.

The stabilizers are needed to impart greater retention of durability to the cured enamels (as measured by 20 ° gloss, distinction of image, cracking or chalking); the stabilizers must not retard cure (normal bake for auto finishes at 121°C and low bake repair at 82°C) as measured by hardness, adhesion, solvent resistance and humidity resistance, the enamel should not yellow on curing and further color change on exposure to light should be minimized; the stabilizers should be soluble in the organic solvents normally used in coating applications such as methyl amyl ketone, xylene, n-hexyl acetate, alcohol and the like.

The instant hindered amine light stabilizers substituted on the N-atom by an OR₁ group fulfill each of these requirements and provide alone or in combination with a UV-absorber outstanding light stabilization protection to the cured coatings.

The following examples describe the inventive use of the hindered amine derivatives in various ambient curable and acid catalyzed thermosetting coatings. Parts and percentages are by weight.

### Example 1: Stabilization of an Aromatic Urethane Varnish

Pieces of 1.27 cm x 20.32 cm x 30.48 cm western red cedar panels having a fine radial cut are used to test a commercially aromatic urethane varnish (Flecto-Varathane 90). One half of each panel is coated with two coats of the unstabilized varnish. An equal amount of varnish containing 7 % (by weight based on resins solids) of light stabilizers is applied to the other half of the panel in two coats. After storage for 2 weeks at ambient temperature, the wood panels are exposed outdoors at an angle of 45°S for a period of 5 months. The 60° gloss of each half of the panel is measured at the top, middle, and bottom portion of the panel and averaged (ASTM D 523). Due to the lack of homogeneity of wood substrates, the gloss retention of the same varnish tends to differ slightly from panel to panel. Thus, the application of an unstabilized control varnish to every panel allows for a more meaningful measurement of the improvement in gloss due to the presence of the light stabilizer.

| Compound | Conc. (% by wt.) | 60 ° Gloss Retention % | | |
|---|---|---|---|---|
| | | Unstabilized | Stabilized | Gloss Improvement |
| 2 | 7 | 52.5 | 63.6 | 11.1 |
| 3 | 7 | 42.5 | 62.1 | 19.6 |
| A/2 | 3.5/3.5 | 44.5 | 64.0 | 19.5 |
| A/3 | 3.5/3.5 | 45.6 | 65.6 | 20.5 |
| A is 2-(2-hydroxy-3,5-di-tert-amylphenyl)-benzotriazole | | | | |

### Example 2: Stabilization of Acrylic Alkyd Refinish Enamel

A commercially available acrylic alkyd enamel pigmented with non-leafing aluminium pigment and tinted a light blue is stabilized with the indicated amount of ultraviolet light absorber and hindered amine derivative (by weight on resin solids) and then spray applied onto Bonderite 40 panels primed with an alkyd primer. After the coating is allowed to cure at room temperature for 14 days, the panels are exposed outdoors at an angle of 5°S for a period of 8 months. The 20° gloss of the panels is measured, as reported below.

| Stabilizer | Conc. (% by wt.) | 20 ° Gloss |
|---|---|---|
| B/15 | 3/2 | 31 |
| B/2 | 3/2 | 31 |
| B/12 | 3/2 | 36 |
| B = 2-[2-hydroxy-3-tert.butyl-5-(2-(omega-hydroxy-octa-(ethyleneoxy)-carbonyl-ethylphenyl]-benzotriazole | | |

### Example 3: Stabilization of a Medium Oil Alkyd Enamel

A medium oil alkyd enamel pigmented with non-leafing aluminium pigment and tinted light blue is stabilized with the indicated amounts of ultraviolet light absorber and hindered amine derivative, and then spray applied onto cold rolled steel panels primed with an epoxy primer. After the coating is allowed to cure at room temperature for 2 weeks, the panels are exposed for accelerated weathering in a Xenon Arc Weatherometer for 840 hours. The 20° gloss values of the panels are determined before and after exposure and indicated below in terms of % gloss retention

| Stabilizer | Conc. (% by wt.) | 20° Gloss Retention % |
|---|---|---|
| B/1 | 3/2 | 28.8 |
| B/12 | 3/2 | 31.7 |
| B/14 | 3/2 | 42.9 |

### Example 4: Stabilization of a Thermoplastic Acrylic Lacquer

A commercially available light blue metallic thermoplastic acrylic lacquer is stabilized with 2 % each of UV absorber and hindered amine (by weight on total resin solids) and then spray applied onto Bonderite 40 panels primed with an alkyd primer. After storage at ambient temperature for 2 weeks, the panels are exposed in an Xenon Arc Weatherometer for 1250 hours. The 20° gloss retention of the panels are reported below.

| Stabilizer | 20° Gloss Retention % |
|---|---|
| C/1 | 21 |
| C/5 | 23 |
| C/15 | 21 |
| C/11 | 24 |
| C/12 | 27 |
| C = 2-(2-hydroxy-3,5-di(alpha,alpha-dimethylbenzyl)phenyl)-benzotriazole | |

### Example 5: Stabilization of Acrylic Alkyd Refinish Enamel

The acrylic alkyd enamel of example 2 pigmented with non-leafing aluminium pigment is stabilized with the indicated amount of light stabilizers (by weight on resin solids) and then spray applied onto Bonderite 40 panels primed with an alkyd primer. After the coating is allowed to cure at ambient temperature for 14 days, the panels are exposed in a QUV weathering apparatus. The 60° gloss values of the samples at various invervals are listed below.

| Stabilizer | 60° Gloss after Exposure of | | | | |
|---|---|---|---|---|---|
| | 0 | 208 | 433 | 593 | 712 hours |
| none | 87 | 38 | 25 | 19 | 13 |
| 2 % 16 | 89 | 69 | 45 | 37 | 31 |
| 2 % 24 | 85 | 62 | 43 | 32 | 24 |
| 2 % 16 + 2 % C | 87 | 77 | 62 | 54 | 45 |
| 2 % 24 + 2 % C | 91 | 79 | 53 | 46 | 41 |

### Example 6: Stabilization of a Thermoset Acrylic Enamel

Pieces of steel sheetings coated with a primer based on-polyester/epoxy resin are coated with a silver metallic base coat in a thickness of about 0.02 mm and air dried for 3 minutes. Thereon a clear top coat is sprayed in a tickness of about 0.038 mm. The clear coat is a thermoset acrylic enamel consisting of 70 % of a copolymer of hydroxyethyl acrylate, styrene, acrylonitrile, butyl acrylate and acrylic acid and 30 % of a melamine resin. It contains further 0.5 % of p-toluenesulfonic acid and the stabilizers indicated in the following table. After 15 minutes air-drying the coated sheets are baked for 30 minutes at 121°C. The hardened samples are exposed in a QUV apparatus and the time to 50 % loss of 20° gloss is determined.

| Stabilizer | Concentration (% by wt. of dry resin) | Time of 50 % loss of 20 ° Gloss (hours) |
|---|---|---|
| none | - | 900 |
| 1/B | 1.5/3.5 | 3900 |
| 2/B | 1.5/3.5 | 4200 |
| 4/B | 1.5/3.5 | 4800 |
| 3/B | 1/3 | 4000 |
| 5/B | 1/3 | 4100 |
| 11/B | 1/3 | 4100 |
| 12/B | 1/3 | 4100 |
| 13/B | 1/3 | 4300 |
| 14/B | 1/3 | 3900 |
| 15/B | 1/3 | 4800 |
| 16/B | 1/3 | 4300 |
| 17/B | 1/3 | 3800 |
| 31/B | 1/3 | 3700 |

### Example 7: Stabilization of a Thermoset Acrylic Enamel

A clear thermoset acrylic enamel based on a binder consisting of 70 % of a copolymer form hydroxyethyl acrylate, butyl acrylate, butyl methacrylate, styrene and acrylic acid and 30 % of a melamine resin is formulated with 0.5 % of p-toulenesulfonic acid and the stabilizers indicated in the following table.

Commercially available steel sheetings coated with a primer are used as substrate. The sheets are coated with a silver metallic base coat which is stabilized with 1 % of a hindered amine (Tinuvin® 440) and 1 % of an UV absorber (Compound B) and is sprayed onto the panel to a thickness of about 0.015 to 0.020 mm. After 3 minutes the clear coat is sprayed onto the base coat in a thickness of 0.04 to 0.05 mm. After 10 minutes of air-drying the samples are baked for 30 minutes at 121 °C. The baked samples are exposed in a QUV weathering apparatus and the distinction of image (Dl) is determined in certain intervals.

| Stabilizer | DI after QUV Exposure of | | | |
|---|---|---|---|---|
| | 0 | 610 | 1250 | 2164 hours |
| none | 85 | 21 | 13 | -- |
| 1.5 % 17 + 3 % B | 83 | 87 | 83 | 84 |
| 1.5 % 24 + 3 % B | 81 | 83 | 85 | 82 |

### Example 8: Stabilization of an Acrylate-Ketimine Enamel

An acrylic ketimine basecoat/clearcoat system is stabilized in the clearcoat with the indicated amount of ultraviolet light absorber and hindered amine derivative. The basecoat is spray applied to a thickness of 0.02 mm onto a ketimine-acetoacetate primed cold rolled steel panel. It is clearcoated with 0.06 mm of an unsaturated acrylate-ketimine enamel (wet on wet). The panels are baked for 45 minutes at 60 °C and are then exposed in a QUV exposure apparatus. In this apparatus, the samples are subjected to weathering in repeated cycles for 4 hours in a humid atmosphere at 50°C and then for 4 hours under UV light at 60 ° C. The 20 gloss of the panels are reported at different exposure intervals.

| Stabilizer | Hours of QUV Exposure | | | | | |
|---|---|---|---|---|---|---|
| | 0 | 400 | 800 | 1200 | 1600 | 2000 |
| Unstabilized | 94 | 93 | 87 | 30* | | |
| 1 % 16 + 1.5 % B | 94 | 93 | 89 | 56 | 31 | 34* |

| | | | | | | |
|---|---|---|---|---|---|---|
| * Indicates Cracking | | | | | | |

### Example 9: Stabilization of a Polyester-Melamine Enamel

A polyester-melamine coil coating catalyzed with p-toluenesulfonic acid is formulated to include a benzotriazole UV absorber and a hindered amine light stabilizer of the invention. The material was applied using a wire-wound rod and an automatic drawdown apparatus onto coil primed panels to a dry thickness of 0.02 mm. The panels were baked in a 500 ° F (260° C) oven for 45 seconds at which time the peak metal temperature was 435°F (225°C). Two colored systems, a phthalo blue and a bronze oxide pigmented system were tested. The panels were exposed in South Florida at an angle of 45°S to the sun for 17 months. The color change (ΔE) of the panels are reported.

| Compound | ΔE of brown panels |
|---|---|
| Unstabilized | 6.7 |
| 3 % 5 + 3 % B | 4.7 |
| | ΔE of blue |
| Unstabilized | 7.0 |
| 3 % 16 + 3 % B | 5.3 |

### Example 10: Stabilization of a Thermoset Acrylic Enamel

The thermoset acrylic enamel of Example 6 is formulated to include a hindered amine light stabilizer of the invention. Coil coated aluminium panels primed with an epoxy primer are coated with about 0.02 mm of a silver metallic basecoat and finally with about 0.06 mm of the clear finishing enamel. After 5-10 minutes of air-drying, the coated panels are baked for 30 minutes at 30 ° C.

The coated panels are exposed in the QUV expoxure apparatus and the 20° gloss of the samples are determined at various invervals.

| Compound | 20° Gloss | | | | | |
|---|---|---|---|---|---|---|
| | 0 | 800 | 1600 | 2000 | 2400 | 2800 |
| Unstabilized | 92 | 69 | 45* | | | |
| 1 % 5 | 92 | 84 | 64 | 53 | 48 | 14 |
| 1 % 16 | 92 | 79 | 49 | 49 | 43 | 20 |
| 1 % 12 | 92 | 90 | 79 | 73 | 63 | 31 |

| | | | | | | |
|---|---|---|---|---|---|---|
| * Indicates Cracking | | | | | | |

Various N-alkoxy hindered amine derivatives containing a single piperidine ring are known. For example, O-alkyl derivatives with hydrogen in the 4-position are disclosed in Kurumada et al, J. Polym. Sci, Polym. Chem. Ed. 23, 1477-91 (1985); Bolsman et al, Rec. Trav. Chim. Pays-Bas 97, 313-19 (1978); and Sholle et al, Dokl. Akad. Nauk SSSR, Chem. Sect. 200, 137-9 (1971). Similar derivatives with benzoyloxy in the 4-position are noted in Kurumada et al, J. Polym. Sci., Polym. Chem. Ed. 22, 277-81 (1984). US 4,547,537 disclose N-alkoxy piperidyl compounds with tetrahydro-1,4-oxazine-2-one group linked to the piperidine ring. N-aralkoxy substituents on hindered piperidine rings are also disclosed in Keana et al, J. Org. Chem. 36, 209-11 (1971) and Howard et al, J. Org. Chem. 43, 4279-83 (1978). N-alpha-hydroxy-alkoxy substituents on piperidinones are noted in Wilson, Trans. Far. Soc. 67, 3008-19 (1971). Moad et al, Aust. J. Chem. 36, 1573-88 (1983) disclose various O-substituents having unsaturation and/or carboxyl groups in the chain. Finally, Fujita et al, J. Polym. Sci., Polym. Lett. Ed. 16, 515-18 (1978) disclose di-piperidinoxy dioxospiro compounds which are able to prevent degradation of several synthetic polymers.

A further object of the invention are the new N-substituted hindered amine compounds having one of formulae A' to M' 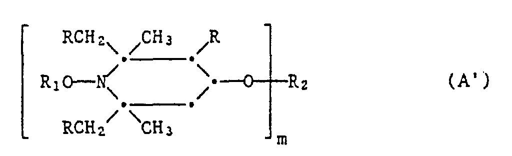 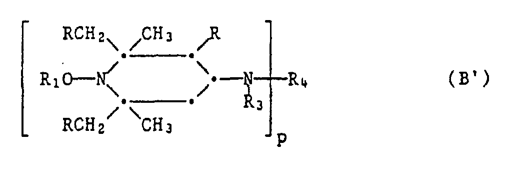 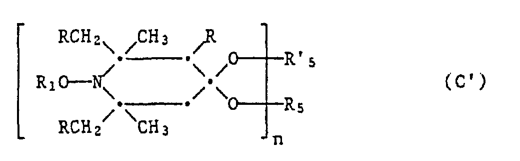 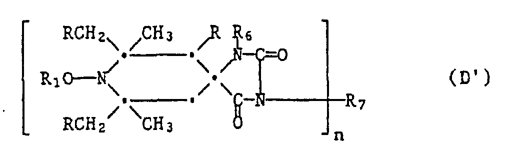 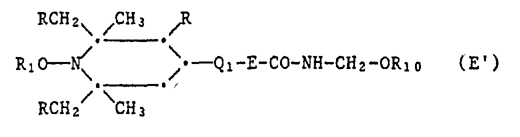 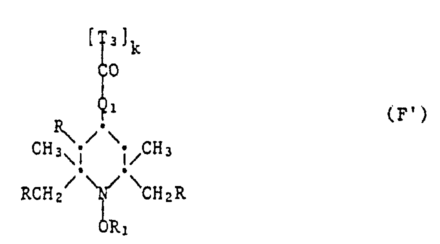 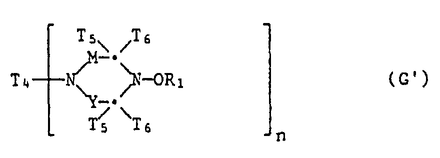 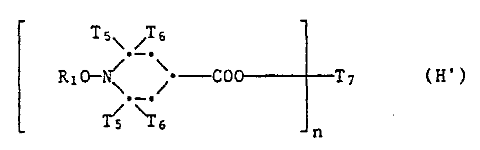 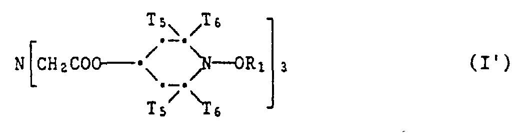 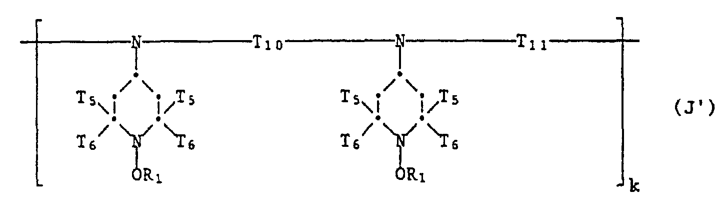 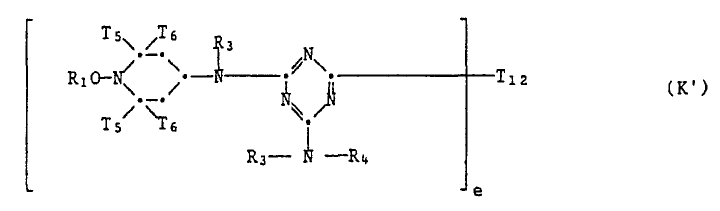 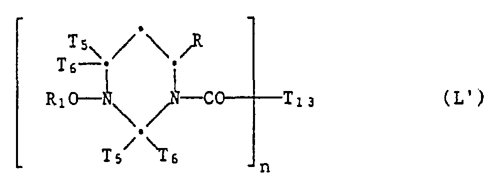 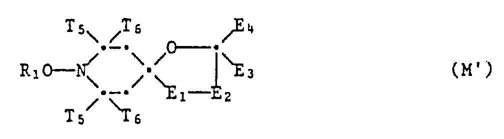 wherein
R is hydrogen or methyl, preferably R is hydrogen;
R₁ is C₁-C₁₈ alkyl, C₂-C₁₈ alkenyl, C₂-C₁₈ alkynyl, C₅-C₈ cycloalkenyl, C₅-C₁₂ cycloalkyl, C₆-C₁₀ bicycloalkyl, C₆-C₁₀ aryl, C₇-C₉ aralkyl, or C₇-C₉ aralkyl substituted by alkyl or aryl;
m is 2-4,
when m is 2
R₂ is C₁-C₁₂ alkylene, C₄-C₁₂ alkenylene, xylylene, a divalent acyl radical of an aliphatic, cycloaliphatic, araliphatic or aromatic dicarboxylic or dicarbamic acid having up to 20 C atoms, preferably an acyl radical of an aliphatic dicarboxylic acid having 2-12 C atoms, of a cycloaliphatic or aromatic dicarboxylic acid having 8-12 C atoms or of an aliphatic, cycloaliphatic or aromatic dicarbamic acid having 8-12 C atoms; or is a group of formula 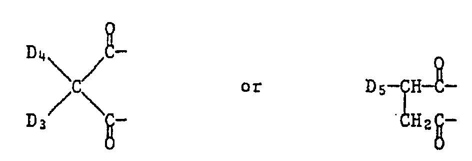 wherein D₃ and D₄ are independently hydrogen, C₁-C₆ alkyl, phenyl, benzyl or 3,5-di-t-butyl-4-hydroxybenzyl and D₅ is alkyl or alkenyl containing up to 18 carbon atoms;
when m is 3, R₂ is a trivalent acyl radical of an aliphatic, cycloaliphatic, or aromatic tricarboxylic acid having up to 12 C atoms;
when m is 4, R₂ is a tetravalent acyl radical of an aliphatic or aromatic tetracarboxylic acid having up to 18 C atoms, including 1,2,3,4-butane-tetracarboxylic acid, 1,2,3,4-but-2-enetetracarboxylic acid, and 1,2,3,5-and 1,2,4,5-pentanetetracarboxylic acid;
p is 1, 2 or 3,
R₃ is hydrogen, C₁-C₁₂ alkyl, C₅-C₈ cycloalkyl, C₇-C₉ aralkyl, C₂-C₁₈ alkanoyl, C₃-C₅ alkenoyl or benzoyl;
when p is 1,
R₄ is hydrogen, C₁-C₁₈ alkyl, C₅-C₈ cycloalkyl, C₂-C₈ alkenyl unsubstituted or substituted by a cyano, carbonyl or carbamide group, or it is C₆-C₁₀ aryl, C₇-C₉ aralkyl, glycidyl, a group of the formula -CH₂-CH-(OH)-Z or -CONH-2 wherein Z is hydrogen, methyl or phenyl; or R₄ is a group of the formula 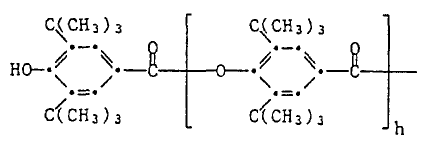 with h as 0 or 1;
or a group of the formula I 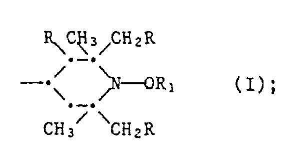 or R₃ and R₄ together are alkylene of 4 to 6 carbon atoms or 1-oxo-alkylene or the divalent acyl radical of an aliphatic or aromatic 1,2- or 1,3-dicarboxylic acid; when p is 2,
R₄ is C₁-C₁₂ alkylene, C₆-C₁₂ arylene, xylylene, a -CH₂CH(OH)-CH₂- group, or a group -CH₂-CH(OH)-CH₂-O-X-O-CH₂-CH(OH)-CH₂- wherein X is C₂-C₁₀ alkylene, C₆-C₁₅ arylene or C₆-C₁₂ cycloalkylene; or, provided that R₃ is not alkanoyl, alkenoyl or benzoyl, R₄ can also be a divalent acyl radical of an aliphatic, cycloaliphatic or aromatic dicarboxylic acid or dicarbamic acid, or can be the group -CO-; or R₄ is a group of formula II 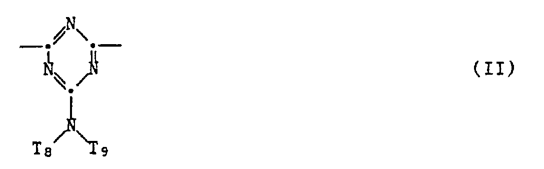 where T₈ and T₉ are independently hydrogen, alkyl of 1 to 18 carbon atoms, or T₈ and T₉ together are alkylene of 4 to 6 carbon atoms or 3-oxapentamethylene, preferably T₈ and T₉ together are 3-oxapentamethylene;
when p is 3,
R₄ is 2,4,6-triazinetriyl,
n is 1 or 2 and
when n is 1,
R₅ and R'₅ are independently C₁-C₁₂ alkyl, C₂-C₁₂ alkenyl, C₇-C₁₂ aralkyl, or R₅ is also hydrogen, or R₅ and R'₅ together are C₂-C₈ alkylene or hydroxyalkylene or C₄-C₂₂ acyloxyalkylene;
and when n is 2,
R₅ and R'₅ together are (-CH₂)₂C(CH₂-)₂;
R₆ is hydrogen, C₁-C₁₂ alkyl, allyl, benzyl, glycidyl or C₂-C₆ alkoxyalkyl;
when n is 1,
R₇ is hydrogen, C₁-C₁₂ alkyl, C₃-C₅ alkenyl, C₇-C₉ aralkyl, C₅-C₇ cycloalkyl, C₂-C₄ hydroxyalkyl, C₂-C₆ alkoxyalkyl, C₆-C₁₀ aryl, glycidyl, a group of the formula -(CH₂)ₜ-COO-Q or of the formula -(CH₂)ₜ-O-CO-Q
wherein t is 1 or 2, and Q is C₁-C₄ alkyl or phenyl;
and when n is 2,
R₇ is C₂-C₁₂ alkylene, C₆-C₁₂ arylene, a group -CH₂CH(OH)-CH₂-O-X-O-CH₂-CH(OH)-CH₂- wherein X is C₂-C₁₀ alkylene, C₆-C₁₅ arylene or C₆-C₁₂ cycloalkylene, or a group -CH₂CH(OZ')CH₂-(OCH₂-CH(OZ')-CH₂)₂- wherein Z' is hydrogen, C₁-C₁₈ alkyl, allyl, benzyl, C₂-C₁₂ alkanoyl or benzoyl;
Q₁ is -N(R₈)- or -O-;
E is C₁-C₃ alkylene, the group -CH₂-CH(R₉)-O- wherein R₉ is hydrogen, methyl or phenyl, the group -(CH₂)₃-NH- or a direct bond;
R₁₀ is hydrogen or C₁-C₁₈ alkyl, R₈ is hydrogen, C₁-C₁₈ alkyl, C₅-C₇ cycloa(kyl C₇-C₁₂ aralkyl, cyanoethyl, C₆-C₁₀ aryl, the group -CH₂-CH(R₉)-OH wherein R₉ has the meaning defined above; a group of the formula I
or a group of the formula 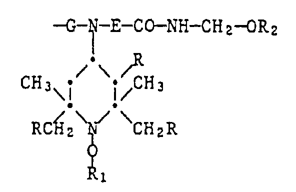 wherein G is C₂-C₆ alkylene or C₆-C₁₂ arylene; or R₈ is a group -E-CO-NH-CH₂-OR₁₀;
Formula F denotes a recurring structural unit of a polymer where T₃ is ethylene or 1,2-propylene, or is the repeating structural unit derived from an alpha-olefin copolymer with an alkyl acrylate or methacrylate;
preferably a copolymer of ethylene and ethyl acrylate, and where k is 2 to 100;
T₄ has the same meaning as R₄ when p is 1 or 2,
T₅ is methyl,
T₆ is methyl or ethyl, or T₅ and T₆ together are tetramethylene or pentamethylene or mixture of said hydroxylamine derivatives, preferably T₅ and T₆ are each methyl,
M and Y are independently methylene or carbonyl, preferably M is methylene and Y is carbonyl, and T₄ is ethylene where n is 2;
T₇ is the same as R₇, and is preferably octamethylene when n is 2,
T₁₀ and T₁₁ are independently alkylene of 2 to 12 carbon atoms, or T₁₁ is a group of formula II,
e is 2, 3 or 4,
T₁₂ is a group -N(R⁴)-(CH₂)_{d}-N(R⁴)-
or 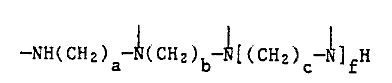 where a, b and c are independently 2 or 3, d is 2-10 and f is 0 or 1, preferably a and c are each 3, b is 2 and f is 1;
when n is 1, T₁₃ is C₂-C₁₈ alkyl, C₂-C₁₈ alkenyl, C₆-C₈ cycloalkyl, C₆-G₁₂ aryl or phenyl substituted by C₁-C₄ alkyl, hydroxy or halogen, and when n is 2, T₁₃ has the same meaning as R₂;
E₁ and E₂, being different, each are -CO- or -N(E₅)-, there E₅ is hydrogen, C₁-C₁₂ alkyl or alkoxycarbonylalkyl of 4 to 22 carbon atoms, preferably E₁ is -CO- and E₂ is -N(E₅).
E₃ is hydrogen, alkyl of 1 to 30 carbon atoms, phenyl, naphthyl, said phenyl or said naphthyl substituted by chlorine or by alkyl of 1 to 4 carbon atoms, or phenylalkyl of 7 to 12 carbon atoms, or said phenylalkyl substituted by alkyl of 1 to 4 carbon atoms, and
E₄ is hydrogen, alkyl of 1 to 30 carbon atoms, phenyl, naphthyl or phenylalkyl of 7 to 12 carbon atoms, or E₃ and E₄ together are polymethylene of 4 to 17 carbon atoms, or said polymethylene substituted by up to four alkyl groups of 1 to 4 carbon atoms, preferably methyl.

In the structures A' to M', if any substituents are C₁-C₁₈ alkyl, they are for example methyl, ethyl, n-propyl, n-butyl, sec-butyl, tert-butyl, n-hexyl, n-octyl, 2-ethylhexyl, n-nonyl, n-decyl, n-undecyl, n-dodecyl, n-tridecyl, n-tetradecyl, n-hexadecyl or n-octadecyl. Typical cycloalkyl groups include cyclopentyl and cyclohexyl; typical cycloalkenyl groups include cyclohexenyl; while typical aralkyl groups include benzyl, alpha-methyl-benzyl, alpha,alpha-dimethylbenzyl or phenethyl.

If R₂ is a divalent acyl radical of a dicarboxylic acid, it is for example an acyl radical of adipic acid, succinic acid, suberic acid, sebacic acid, phthalic acid, isophthalic acid, terephthalic acid, dibutylmalonic acid, dibenzylmalonic acid, (3,5-di-tert-butyl-4-hydroxybenzyl)-malonic acid or bicycloheptenedicarboxylic acid.

If R₂ is a divalent acyl radical of a dicarbamic acid, it is for example an acyl radical of hexamethylenedicarbamic acid or of 2,4-toluylenedicarbamic acid.

The following compounds are examples of polyalkylpiperidine starting materials useful in making the hindered amine derivatives of formula A'. (Relates to the selected preparative procedure).
di-(2,2,6,6-tetramethylpiperidin-4-yl) adipate
di-(2,2,6,6-tetramethylpiperidin-4-yl) sebacate
di-(2,2,6,6-tetramethyipiperidin-4-yl) phthalate
alpha,alpha'-(di-2,2,6,6-tetramethylpiperidine-4-oxy)-p-xylene
di-(2,2,6,6-tetramethylpiperidin-4-yl) succinate
di-(2,2,6,6-tetramethylpiperidin-4-yl) malonate
di-(1-oxyl-2,2,6,6-tetramethylpiperidin-4-yl) isophthalate
4-hydroxy-1-methoxy-2,2,6,6-tetramethylpiperidine
di-(1-hydroxy-2,2,6,6-tetramethylpiperidin-4-yloxy)-p-xylene
1-ethoxy-4-hydroxy-2,2,6,6-tetramethylpiperidine
(2,2,6,6-tetramethylpiperidin-4-yl)-[4-(2-oxoazepin-1-yl)-2,2,6,6-tetramethylpiperidin-4-yl] acetate.

As C₂-C₁₈ alkanoyl, R₃ is for example propionyl, butyryl, octanoyl, dodecanoyl, hexadecanoyl, octadecanoyl, but preferably acetyl; and as C₃-C₅ alkenoyl, R₃ is in particular acryloyl.

If R₄ is C₂-C₈ alkenyl unsubstituted or substituted by a cyano, carbonyl or carbamide group, it is for example 1-propenyl, allyl, methallyl, 2-butenyl, 2-pentenyl, 2-hexenyl, 2-octenyl, 2,2-dicyanovinyl, 1-methyl-2-cyano-2-methoxycarbonyl-vinyl or 2,2-diacetylaminovinyl.

If any substituents are C₂-C₁₂ alkylene, they are for example ethylene, propylene, 2,2-dimethylpropylene, tetramethylene, hexamethylene, octamethylene, decamethylene or dodecamethylene.

If any substituents are C₆-C₁₅ arylene, they are for example o-, m- or p-phenylene, 1,4-naphthylene or 4,4'-di-phenylene.

As C₆-C₁₂ cycloalkylene, X is especially cyclohexylene.

The following compounds are examples of polyalkylpiperidine starting materials useful in making the hindered amine derivatives of formula B'.
N,N'-bis-(2,2,6,6-tetramethylpiperidin-4-yl)-hexamethylene-1,6-diamine,
N,N'-bis-(2,2,6,6-tetramethylpiperidin-4-yl)-hexamethylene-1,6-diacetamide,
4-benzylamino-2,2,6,6-tetramethylpiperidine,
N-n-butyl-N-(2,2,6,6-tetramethylpiperidin-4-yl)-4-hydroxy-3,5-di-tert.butylbenzamide,
N,N'-bis-(2,2,6,6-tetramethylpiperidin-4-yl)-N,N'-dibutyl-adipamide,
N,N'-bis-(2,2,6,6-tetramethylpiperidin-4-yl)-N,N'-dicyclohexyl-2-hydroxypropylenediamine,
N,N'-bis-(2,2,6,6-tetramethylpiperidin-4-yl)-p-xylylenediamine,
4-(3-methyl-4-hydroxy-5-tert-butyl-benzoyl acetamido)-2,2,6,6-tetramethylpiperidine,
alpha-cyano-β-methyl-β-[N-(2,2,6,6-tetramethylpiperidin-4-yl)-amino]-acrylic acid methyl ester,
1-oxyl-22,6,6-tetramethylpiperidin-4-one.

If R₅ is C₂-C₈ alkylene or hydroxyalkylene, it is for example ethylene, 1-methyl-ethylene, propylene, 2-ethylpropylene or 2-ethyl-2-hydroxymethylpropylene.

As C₄-C₂₂ acyloxyalkylene, R₅ is for example 2-ethyl-2-acetoxymethylpropylene.

The following compounds are examples of polyalkylpiperidine starting materials useful in making the hindered amine derivatives of formula C'.
9-aza-8,8,10,10-tetramethyl-1,5-dioxaspiro[5.5]undecane,
9-aza-8,8,10,10-tetramethyl-3-ethyl-1,5-dioxaspiro[5.5]undecane,
2,2,6,6-tetramethylpiperidine-4-spiro-2'-(1',3'-dioxane)-5'-spiro-5"-(1",3"-dioxane)-2"-spiro-4"'-(2''',2''',6''',6'''-tetramethylpiperidine).

If any substituents are C₂-C₆ alkoxyalkyl, they are for example methoxymethyl, ethoxymethyl, propoxymethyl, tert-butoxyethyl, ethoxyethyl, ethoxypropyl, n-butoxyethyl, tert-butoxyethyl, isopropoxyethyl or propoxypropyl.

If R₇ is C₃-C₅ alkenyl, it is for example 1-propenyl, allyl, methallyl, 2-butenyl or 2-pentenyl.

As C₇-C₉ aralkyl, R₇ is in particular phenethyl or above all benzyl; and as C₅-C₇ cycloalkyl, R₇ is especially cyclohexyl.

If R₇ is C₂-C₄ hydroxyalkyl, it is for example 2-hydroxyethyl, 2-hydroxypropyl, 3-hydroxypropyl, 2-hydroxybutyl or 4-hydroxybutyl.

As C₆-C₁₀ aryl, R₁ and R₇ are in particular phenyl, or alpha- or β-naphthyl which is unsubstituted or substituted by halogen or C₁-C₄ alkyl.

If R₇ is C₂-C₁₂ alkylene, it is for example ethylene, propylene 2,2-dimethylpropylene, tetramethylene, hexamethylene, octamethylene, decamethylene or dodecamethylene.

If R₇ is C₆-C₁₂ arylene, it is for example o-, m- or p-phenylene, 1,4-naphthylene or 4,4'-diphenylene.

If Z' is C₂-C₁₂ alkanoyl, it is for example propionyl, butyryl, octanoyl, dodecanoyl or preferably acetyl.

The following compounds are examples of polyalkylpiperidine starting materials useful in making hindered amine derivatives of formula D'.
3-benzyl-1,3,8-triaza-7,7,9,9-tetramethylspiro[4.5]-decane-2,4-dione,
3-n-octyl-1,3,8-triaza-7,7,9,9-tetramethylspiro[4.5]-decane-2,4-dione,
3-allyl-1,3,8-triaza-1,7,7,9,9-pentamethylspiro[4.5]-decane-2,4-dione, or the compounds of the following formulae: 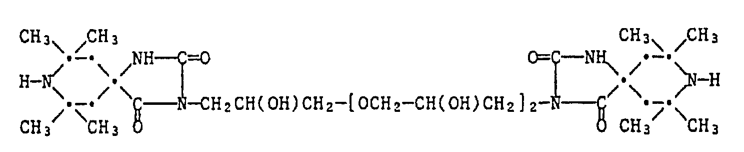 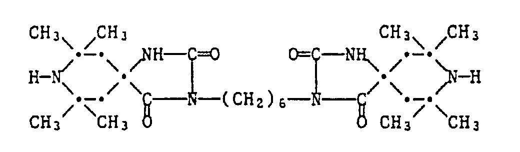 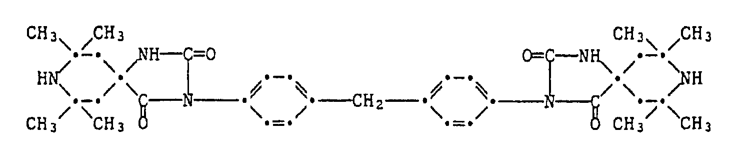 As C₅-C₇ cycloalkyl, R₈ is in particular cyclohexyl.

As C₆-C₁₀ aryl, R₈ is particularly phenyl, or alpha- or β-naphthyl which is unsubstituted or substituted with halogen or C₁-C₄ alkyl. As C₁-C₃ alkylene, E is for example methylene, ethylene or propylene.

As C₂-C₆ alkylene, G is for example ethylene, propylene, 2,2-dimethylpropylene, tetramethylene or hexamethylene; and as C₆-C₁₂ arylene, G is o-, m- or p-phenylene, 1,4-naphthylene or 4,4'-diphenylene.

The following compounds are examples of polyalkylpiperidine starting materials useful in making the hindered amine derivatives of formula E'.
N-hydroxymethyl-N'-2,2,6,6-tetramethylpiperidin-4-yl-urea,
N-methoxymethyl-N'-2,2,6,6-tetramethylpiperidin-4-yl-urea,
N-methoxymethyl-N'-n-dodecyl-N'-2,2,6,6-tetramethylpiperidin-4-yl-urea,
O-(2,2,6,6-tetramethylpiperidin-4-yl)-N-methoxymethyl-urethane.

When the instant hindered amine derivative is of formula F', the following polymeric compounds are examples of starting materials useful in preparing said derivatives. 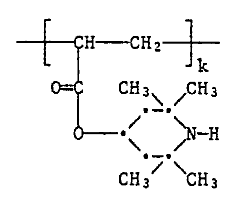 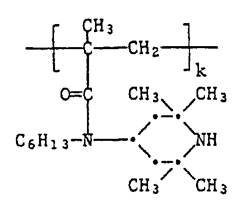 Additional starting hindered amine derivatives include for formula J':
poly-{[6-[(1,1,3,3-tetramethylbutyl)-imino]-1,3,5-triazine-2,4-diyl][2-(1-oxyl-2,2,6,6-tetramethylpiperidyl)-imino]-hexamethylene-4[4-(1-oxyl-2,2,6,6-tetramethylpiperidyl]-imino]}.

Preferred are the compounds of formula A' to M' wherein R is hydrogen, R₁ is C₁-C₁₈ alkyl, C₂-C₆ alkenyl, C₅-C₈ cycloalkyl, cyclohexyl, phenyl or C₇-C₉ aralkyl; m is 2-4 and when m is 2, R₂ is C₂-C₈ alkylene, C₄-C₈ alkenylene, xylylene, a divalent acyl radical of an aliphatic, cycloaliphatic or aromatic dicarboxylic acid having up to 12 carbon atoms or of an aliphatic or aromatic dicarbamic acid having up to 12 carbon atoms, or is a group of formula 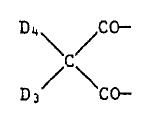 wherein D₃ and D₄ are independently hydrogen, C₁-C₆ alkyl, benzyl or 35-di-t-butyl-4-hydroxybenzyl, and when m is 3, R₂ is a trivalent acyl radical of an aliphatic or aromatic tricarboxylic acid having up to 12 carbon atoms, and when m is 4, R₂ is a tetravalent acyl radical of an aliphatic or aromatic tetracarboxylic acid having up to 12 carbon atoms;
p is 1, 2 or 3, R₃ is hydrogen, C₁-C₁₂ alkyl, C₅-C₈ cycloalkyl, C₇-C₉ aralkyl, C₂-C₁₈ alkanoyl or benzoyl, and when p is 1, R₄ is hydrogen, C₁-C₁₈ alkyl, C₅-C₈ cycloalkyl, phenyl, benzyl or a group of the formula 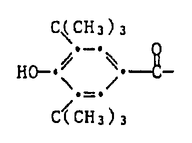 or of the formula 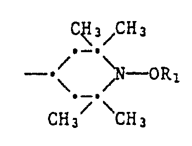 and when p is 2, R₄ is C₂-C₁₂ alkylene, C₆-C₁₂ arylene, xylylene, or provided that R₃ is not alkanoyl or benzoyl, R₄ can also be a divalent acyl radical of an aliphatic or aromatic dicarboxylic acid having up to 12 carbon atoms, of an aliphatic or aromatic dicarbamic acid having up to 12 carbon atoms, or can be a group of formula II wherein T₈ and T₉ are independently hydrogen or C₁-C₁₂ alkyl or T₈ and T₉ together are C₄-C₆ alkylene or 3-oxapentamethylene, and when p is 3, R₄ is 2,4,6-triazinetriyl; n is 1 or 2, and when n is 1, R₅ and R'₅ are C₁-C₁₂ alkyl or benzyl, or R₅ and R'₅ together are C₂-C₈ alkylene or hydroxyalkylene and when n is 2, R₅ and R'₅ together are (-CH₂)₂C(CH₂-)₂;
R₆ is hydrogen, C₁-C₁₂ alkyl, allyl or benzyl, and when n is 1, R₇ is hydrogen, C₁-C₁₂ alkyl, allyl, benzyl, cyclohexyl, 2-hydroxyethyl or a group of the formula -CH₂CH₂-COOQ wherein Q is C₁-C₄ alkyl, and when n is 2, R₇ is C₂-C₁₂ alkylene or C₆-C₁₂ arylene;
Q₁ is -N(R₈)- or -O-; E is C₁-C₃ alkylene or a direct bond; R₁₀ is hydrogen or C₁-C₁₄ alkyl; R₈ is hydrogen, C₁-C₁₂ alkyl, cyclohexyl, benzyl, cyanoethyl or a group 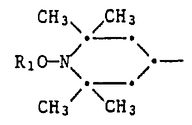 T₃ is ethylene or 1,2-propylene, k is 2 to 100;
T₄ has the same meaning as R₄ when p is 1 or 2,
T₅ and T₆ are methyl, M and Y are independently -CH₂- or -CO-;
T₇ is the same as R₇;
T₁₀ and T₁₁ are independently C₂-C₈ alkylene or T₁₁ is a group of formula I,
e is 3 or 4,
T₁₂ is a group 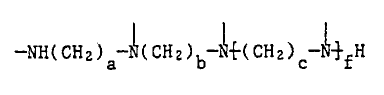 wherein a, b and c are independently 2 or 3, and f is 0 or 1;
when n is 1, T₁₃ is C₂-C₁₈ alkyl, cyclohexyl or phenyl and when n is 2, T₁₃ has the same meaning as R₂;
E₁ is -CO- and E₂ is -N(E₅)-, wherein E₅ is hydrogen, C₁-C₁₂ alkyl or C₄-C₁₈ alkoxycarbonylalkyl, E₃ and E₄ are independently C₁-C₁₂ alkyl or phenyl or E₃ and E₄ together are C₄-C₁₂ polymethylene.

Especially preferred are the compounds of formula (A'), (B'), (C'), (J'), (K') or (M') wherein R is hydrogen, R₁ is C₁-C₁₈ alkyl, cyclohexyl, cyclohexenyl, methylcyclohexyl or C₇-C₉ phenylalkyl;
m is 2, R₂ is C₂-C₈ alkylene, xylylene or a group -CO-R₁₁-CO-, wherein R₁₁ is C₂-C₈ alkylene, cyclohexylene or phenylene or a group 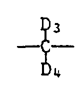 wherein D₃ and D₄ are independently hydrogen, C₁-C₄ alkyl, benzyl or 3,5-di-t-butyl-4-hydroxybenzyl;
p is 1 or 2, R₃ is hydrogen, C₁-C₁₂ alkyl or C₂-C₈ alkanoyl, and when p is 1, R₄ is hydrogen, C₁-C₁₂ alkyl, and when p is 2, R₄ is C₂-C₈ alkylene or is -CO-R₁₁-CO-;
n is 1 or 2, and when n is 1, R₅ and R'₅ together are C₂-C₈ alkylene, and when n is 2, R₅ and R'₅ together are (-CH₂)₂C(CH₂-)₂;
k is 5-20, T₁₀ is C₂-C₈ alkylene and T₁₁ is a group of formula II,
wherein T₈ and T₉ are independently hydrogen or C₁-C₁₂ alkyl or T₈ and T₉ together are pentamethylene or 3-oxapentamethylene, T₅ and T₆ are methyl; e is 4, T₁₂ is a group 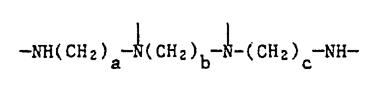 wherein a, b and c independently are 2 or 3;
E₁ is -CO- and E₂ is -N(E₅)-, wherein E₅ is hydrogen, C₁-C₁₂ alkyl or C₄-C₁₅ alkoxycarbonylalkyl, and E₅ and E₄ are independently C₁-C₁₂ alkyl or E₃ and E₄ together are C₅-C₁₂ polymethylene.

The hindered amine derivatives of the instant invention are generally prepared by oxidizing the corresponding hindered amine with an appropriate peroxy compound such as hydrogen peroxide or tert-butyl hydroperoxide in the presence of a metal carbonyl or metal oxide catalyst followed by reduction of the oxyl intermediate formed to the desired N-hydroxy derivative, preferably by catalytic hydrogenation.

Thereafter, the O-alkyl derivatives can be synthesized by several routes. For example, the N-hydroxy derivative can be alkylated with sodium hydride and halogenated hydrocarbons such as benzyl bromide and ethyl iodide. N-methoxy variants can be prepared by thermolysis of a chloro-benzene solution of nitroxyl radical and di-tert-butyl peroxide. The product is formed by a coupling reaction between the nitroxyl radical and the methyl radical generated from β-scission of a t-butoxy radical.

Other N-alkoxy variants can be synthesized by coupling nitroxyl radicals with hydrocarbon radicals generated from thermal decomposition of di-tert-butyl peroxide in the presence of hydrocarbon solvents such as cyclohexane, toluene, and ethylbenzene.

A preferred approach is the preparation of N-alkoxy hindered amines directly from hindered amines. For example, a mixture of 4-benzoyloxy-2,2,6,6-tetramethylpiperidine, aqueous t-butyl hydroperoxide, molybdenum oxide, and ethylbenzene gives a 90 % yield of the N-alpha-methylbenzyloxy piperidine. Molybdenum (VI) has been shown to increase the efficiency of both the oxidation of hindered amine to nitroxyl radical and the reaction of nitroxyl radicals with hydrocarbons.

Although these procedures have been referenced in terms of N-alkoxy substituents, they are meant to equally apply to all OR₁ groups.

The hindered amine precursors are largely commercially available or can be prepared by the application of known methods.

Reference is made to Kurumada et al, J. Polym. Sci., Poly. Chem. Ed. 23, 1477-91 (1985), Moad et al, Aust. J. Chem. 36, 1573-88 (1983) and US 4,547,537 in this regard.

The derivatives are particularly effective in stabilizing organic materials against the degradative effects of acitinic stimuli. Such organic materials include polymeric materials such as the following polymers.
1. Polymers of monoolefins and diolefins, for example polypropylene, polyisobutylene, polybutene-1, polymethylpentene-1, polyisoprene or polybutadiene, as well as polymers of cycloolefins, for instance of cyclopentene or norbornene, polyethylene (which optioanlly can be crosslinked), for example high density polyethylene (HDPE), low density polyethylene (LDPE) and linear low density polyethylene (LLDPE).
2. Mixtures of the polymers mentioned under 1), for example mixtures of polypropylene with polyisobutylene, polypropylene with polyethylene (for example PP/HDPE, PP/LDPE) and mixtures of different types of polyethylene (for example LDPE/HDPE).
3. Copolymers of monoolefines and diolefines with each other or with other vinyl monomers, such as, for example, ethylene/propylene, linear low density polyethylene (LLDPE) and its mixtures with low density polyethylene (LDPE), propylene/butene-1, ethylene/hexene, ethylene/ethylpentene, ethylene/heptene, ethylene/octene, propylene/isobutylene, ethylene/butene-1, propylene/butadiene, isobutylene/isoprene, ethylene/alkyl acrylates, ethylene/alkyl methacrylates, ethylene/vinyl acetate or ethylene/ acrylic acid copolymers and their salts (ionomers) and terpolymers of ethylene with propylene and a diene, such as hexadiene, dicyclopentadiene or ethylidene-norbornene; as well as mixtures of such copolymers and their mixtures with polymers mentioned in 1) above, for example polypropylene/ethylene-propylene-copolymers, LDPE/EVA, LDPE/EAA, LLDPE/EVA and LLDPE/EAA.
   3a. Hydrocarbon resins (for example C₅-C₉) and hydrogenated modifications thereof (for example tackyfiers).
4. Polystyrene, poly-(p-methylstyrene), poly-(α-methylstyrene).
5. Copolymers of styrene or α-methylstyrene with dienes or acrylic derivatives, such as, for example, styrene/butadiene, styrene/ acrylonitrile, styrene/alkyl methacrylate, styrene/maleic anhydride, styrene/butadiene/ethyl acrylate, styrene/acrylonitrile/methyl acrylate; mixtures of high impact strength from styrene copolymers and another polymer, such as, for example, from a polyacrylate, a diene polymer or an ethylene/propylene/diene terpolymer; and block copolymers of styrene, such as, for example, styrene/butadiene/ styrene, styrene/ isoprene/styrene, styrene/ethylene/butylene/ styrene or styrene/ethylene/propylene/styrene.
6. Graft copolymers of styrene or α-methylstyrene such as, for example, styrene on polybutadiene, styrene on polybutadiene-styrene or polybutadiene-acrylonitrile; styrene and acrylonitrile (or methacrylonitrile) on polybutadiene; styrene and maleic anhydride or maleimide on polybutadiene; styrene, acrylonitrile and maleic anhydride or maleimide on polybutadiene; styrene, acrylonitrile and methyl methacrylate on polybutadiene, styrene and alkyl acrylates or methacrylates on polybutadiene, styrene and acrylonitrile on ethylene/propylene/diene terpolymers, styrene and acrylonitrile on polyacrylates or polymethacrylates, styrene and acrylonitrile on acrylate/butadiene copolymers, as well as mixtures thereof with the copolymers listed under 5), for instance the copolymer mixtures known as ABS-, MBS-, ASA- or AES-polymers.
7. Halogen-containing polymers, such as polychloroprene, chlorinated rubbers, chlorinated or sulfochlorinated polyethylene, epichlorohydrin homo- and copolymers, polymers from halogen-containing vinyl compounds,as for example, polyvinylchloride, polyvinylidene chloride, polyvinyl fluoride, polyvinylidene fluoride, as well as copolymers thereof, as for example, vinyl chloride/vinylidene chloride, vinyl chloride/vinyl acetate or vinylidene chloride/vinyl acetate copolymers.
8. Polymers which are derived from α,β-unsaturated acids and derivatives thereof, such as polyacrylates and polymethacrylates, polyacrylamide and polyacrylonitrile.
9. Copolymers from the monomers mentioned under 8) with each other or with other unsaturated monomers, such as, for instance, acrylonitrile/butadiene, acrylonitrile/alkyl acrylate, acrylonitrile/ alkoxyalkyl acrylate or acrylonitrile/vinyl halogenide copolymers or acrylonitrile/alkyl methacrylate/butadiene terpolymers.
10. Polymers which are derived from unsaturated alcohols and amines, or acyl derivatives thereof or acetals thereof, such as polyvinyl alcohol, polyvinyl acetate, polyvinyl stearate, polyvinyl benzoate, polyvinyl maleate, polyvinyl butyral, polyallyl phthalate or polyallylmelamine; as well as their copolymers with olefins mentioned in 1) above.
11. Homopolymers and copolymers of cyclic ethers, such as polyalkylene glycols, polyethylene oxide, polypropylene oxide or copolymers thereof with bis-glycidyl ethers.
12. Polyacetals, such as polyoxymethylene and those polyoxymethylenes which contain ethylene oxide as a comonomer; polyacetals modified with thermoplastic polyurethanes, acrylates or MBS.
13. Polyphenylene oxides and sulfides, and mixtures of polyphenylene oxides with polystyrene or polyamides.
14. Polyurethanes which are derived from polyethers, polyesters or polybutadienes with terminal hydroxyl groups on the one side and aliphatic or aromatic polyisocyanates on the other side, as well as precursors thereof (polyisocyanates, polyols or prepolymers).
15. Polyamides and copolyamides which are derived from diamines and dicarboxylic acids and/or from aminocarboxylic acids or the corresponding lactams, such as polyamide 4, polyamide 6, polyamide 6/6, 6/10, 6/9, 6/12 and 4/6, polyamide 11, polyamide 12, aromatic polyamides obtained by condensation of m-xylene diamine and adipic acid; polyamides prepared from hexamethylenediamine and isophthalic or/and terephthalic acid and optionally an elastomer as modifier, for example poly-2,4,4,-trimethylhexamethylene terephthalamide or poly-m-phenylene isophthalamide. Further copolymers of the aforementioned polyamides with polyolefins, olefin copolymers, ionomers or chemically bonded or grafted elastomers; or with polyethers, such as for instance, with polyethylene glycols, polypropylene glycols or polytetramethylene glycols. Polyamides or copolyamides modified with EPDM or ABS. Polyamides condensed during processing (RIM-polyamide systems).
16. Polyureas, polyimides and polyamide-imides.
17. Polyesters which are derived from dicarboxylic acids and diols and/or from hydroxycarboxylic acids or the corresponding lactones, such as polyethylene terephthalate, polybutylene terephthalate, poly-1,4-dimethylolcyclohexane terephthalate, poly-[2,2,-(4-hydroxyphenyl)-propane] terephthalate and polyhydroxybenzoates as well as block-copolyether-esters derived from polyethers having hydroxyl end groups.
18. Polycarbonates and polyester-carbonates.
19. Polysulfones, polyether-sulfones and polyether-ketones.
20. Crosslinked polymers which are derived from aldehydes on the one hand and phenols, ureas and melamines on the other hand, such as phenol/formaldehyde resins, urea/formaldehyde resins and melamine/formaldehyde resins.
21. Drying and non-drying alkyd resins.
22. Unsaturated polyester resins which are derived from copolyesters of saturated and unsaturated dicarboxylic acids with polyhydric alcohols and vinyl compounds as crosslinking agents, and also halogen-containing modifications thereof of low inflammability.
23. Thermosetting acrylic resins, derived from substituted acrylic esters, such as epoxy-acrylates, urethane-acrylates or polyester-acrylates.
24. Alkyd resins, polyester resins or acrylate resins in admixture with melamine resins, urea resins, polyisocyanates or epoxide resins as crosslinking agents.
25. Crosslinked epoxide resins which are derived from polyepoxides, for example from bis-glycidyl ethers or from cycloaliphatic diepoxides.
26. Natural polymers, such as cellulose, rubber, gelatine and derivatives thereof which are chemically modified in a polymer-homologous manner, such as cellulose acetates, cellulose propionates and cellulose butyrates, or the cellulose ethers, such as methylcellulose; rosins and their derivatives.
27. Mixtures of polymers as mentioned above, for example PP/EPDM, Polyamide 6/EPDM or ABS, PVC/EVA, PVC/ABS, PVC/MBS, PC/ABS, PBTP/ABS, PC/ASA, PC/PBT, PVC/CPE, PVC/acrylates, POM/thermoplastic PUR, PC/thermoplastic PUR, POM/acrylate, POM/MBS, PPE/HIPS, PPE/PA 6.6 and copolymers, PA/HDPE, PA/PP, PA/PPE.

The instant compounds are added to the polymers in a concentration of 0.05 to 5 % by weight, calculated relative to the material to be stabilized. Preferably, 0.1 to 2.5 % by weight of the stabilizer calculated relative to the material to be stabilized, is incorporated into the latter.

Incorporation can be effected during the polymerization or after polymerization, for example by mixing the compounds and, if desired, further additives into the melt by the methods customary in the art before or during shaping, or by applying the dissolved or dispersed compunds to the polymer.

Further additives used in combination with the instant compounds may be other stabilizers such as phenolic antioxidants, metal desactivators, phosphites, thiodipropionic diesters, fatty acid salts, UV-absorbers or nickel complex salts. Further additives may be pigments, fillers, plasticizers, flame retardants or antistatica.

In general, the stabilizers of this invention are employed from about 0.05 to about 5 % by weight of the stabilized composition, although this will vary with the particular substrate and application. An advantageous range is from about 0.1 to about 2.5 %.

The compounds of the invention further can be used - alone or together with phenols - in photographic layers as yellow dye light stabilizers, as cyan dye dark stabilizers, as antistain agents in magenta layers (especially for two-equivalent magenta couplers) and as thermal stabilizers for magenta couplers.

The following examples will further illustrate the embodiments of this invention.

### Example 11 Di-(1-methoxy-2,2,6,6-tetramethylpiperidin-4-yl) Isophthalate

A solution of 30.0 g (73 mmol) of di-(1-oxyl-2,2,6,6-tetramethylpiperidin-4-yl) isophthalate and 27.8 g (190 mmol) of di-tert-butyl peroxide in 70 ml of chlorobenzene is heated for 6 hours in a nitrogen atmosphere in a Fisher-Porter bottle (bath temp. 145-50°C). The crude reaction mixture is chromatographed on silica gel (98:2 heptane: ethyl acetate) to obtain solid, which is recrystallized from methanol to afford the title compound, a white crystalline solid, m.p. 99-101 °C.

| | | | |
|---|---|---|---|
| Anal. Calcd. for C₂₈H₄₄N₂O₆ | C, 66.6; | H, 8.8; | N, 5.55. |
| Found | C, 66.4; | H, 8.7; | N, 5.5. |

### Example 12 Di-(1-methoxy-2,2,6,6-tetramethylpiperidin-4-yl) Sebacate

4-Ben2oyloxy-1-methoxy-2,2,6,6-tetramethylpiperidine (8.0 g, 32 mmol) is stirred for 2 hr. at 60-70 ° C (nitrogen atmosphere) with 2.2 g (39 mmol) of potassium hydroxyde in 300 ml of 1:1 (v/v) methanol:water. Solvent is removed under reduced pressure to obtain a white solid, which is partitioned between water (100 ml) and dichloromethane (150 ml). The aqueous layer is washed with dichloromethane (2 x 150 ml). The organic layers are combined and washed with water (100 ml) and saturated sodium chloride (100 ml), then dried over magnesium sulfate and concentrated to afford 5,7 g of crude 4-hydroxy-1-methoxy-2,2,6,6-tetramethylpiperidine, a white solid with m.p. 92.5-93.5 C. IR: 3250 cm⁻¹.

A solution of 5.4 g (29 mmol) of 4-hydroxy-1-methoxy-2,2,6,6-tetramethylpiperidine, 3.2 g (13.9 mmol) of dimethyl sebacate, and 200 ml of toluene is distilled for 45 minutes to azeotrope any water present. The solution is allowed to cool and 150 mg of lithium amide is added. The reaction mixture is slowly distilled for 5 hr. to remove methanol along with some of the toluene. The remaining toluene is then removed at reduced pressure. The reaction mixture is cooled to 5°C and water (20 ml) is added. The organic material is dissolved in ethyl acetate (200 ml). The aqueous layer is extracted with ethyl acetate (2 x 100 ml). The combined organic layers are washed with water (2 x 50 ml) and saturated sodium chloride (50 ml), then dried over magnesium sulfate and concentrated under reduced pressure. The crude liquid is chromatographed on silica gel (95:5 heptane: ethyl acetate) to obain 5.4 g (68 % overall yield) of the title compound, a colorless liquid. IR: 1750 cm⁻¹.

| | | | |
|---|---|---|---|
| Anal. Calcd. for C₃₀H₅₆N₂O₆ | C, 66.6; | H, 10.4; | N, 5.2. |
| Found | C, 66.7; | H, 10.4; | N, 5.0. |

### Example 13 alpha,alpha'-(Di-1-ethoxy-2,2,6,6-tetramethylpiperidin-4-yloxy)-p-xylene

A mixture of 9.0 g (20.1 mmol) of alpha,alpha'-(di-1-hydroxy-2,2,6,6-tetramethylpiperidin-4-yloxy)-p-xylene, 1.8 g (44.2 mmol) of sodium hydride, and 100 ml of tetrahydrofuran (THF) is refluxed under nitrogen for 2 hr. The reaction mixture is cooled to 50°C and excess ethyl iodide (7.5 g, 48.2 mmol) is added. The reaction mixture is refluxed for 2 hr. Additional sodium hydride (1.8 g), ethyl iodide (7.5 g) and 1.0 ml of t-butyl alcohol are then added, and the reaction mixture is refluxed for 16 hr. The reaction mixture is cooled and methanol is added. The reaction mixture is partitioned between water (600 ml) and diethyl ether (200 ml). The aqueous layer is extracted with ether (200 ml). The combined organic layers are washed with water (200 ml) and saturated sodium chloride (200 ml), then dried over magnesium sulfate and concentrated to obtain a yellow oil. The oil is chromatographed on silica gel (4:1 hexane: ethyl acetate) to obtain a crude solid which is successively recrystallized from cold methanol and hexane. The yield is 7.9 g (78 %) of a white solid, m.p. 99-110°C.

| | | | |
|---|---|---|---|
| Anal. Calcd. for (C₃₀H₅₂N₂O₄) | C, 71.4; | H, 10.4; | N, 5.5. |
| Found | C, 71.6; | H, 10.8; | N, 5.5. |

### Example 14 Di-(1-cyclohexyloxy-2,2,6,6-tetramethylpiperidin-4-yl) Sebacate

A mixture of 20.0 g (41.6 mmol) of di-(-2,2,6,6-tetramethylpiperidin-4-yl) sebacate, 43 g (334 mmol) of 70 % aqueous t-butyl hydroperoxide, 1.3 g (9.0 mmol) of molybdenum trioxide, and 125 ml of cyclohexane is heated at reflux for 2.3 hours. Water is collected in a Dean-Stark trap. The red reaction mixture is cooled and transferred to a Fischer-Porter bottle. Fresh cyclohexane (25 ml) is used to thoroughly rinse the flask, and rinsings are added to the pressure bottle. The pressure bottle is immersed in an oil bath (140°C) for 3 hours whereupon the colorless reaction mixture is cooled to room temperature and filtered. The filtrate is stirred with 10 g of sodium sulfite in 90 ml of water for 2 hours to decompose unreacted hydroperoxide, then diluted with ethyl acetate (200 ml) and water (100 ml). The organic layer is washed with 10 % sodium sulfite (100 ml), water (100 ml), saturated sodium chloride (100 ml), then dried over magnesium sulfate and concentrated at reduced pressure. The crude product is purified by flash chromatography (silica gel, 100:2 heptane: ethyl acetate) to afford 17.8 g (68 % yield) of a white solid, m.p. 56-9°C.

| | | | |
|---|---|---|---|
| Anal. Calcd. for C₄₀H₇₂N₂O₆ | C, 71.0; | H, 10.7; | N, 4.1. |
| Found | C, 71.0; | H, 10.2; | N, 4.2. |

### Example 15 Di-(1-cyclohexyloxy-2,2,6,6-tetramethylpiperidin-4-yl) Isophthalate

A solution of 34.2 g of di-(1-oxyl-2,26,6-tetramethylpiperidin-4-yl) isophthalate and 54 ml of di-tert-butyl peroxide in 250 ml of cyclohexane is heated for 22 hours in a nitrogen atmosphere in a Fisher-Porter bottle (bath temperature of 140°C). Solvent is evaporated under reduced pressure. The product is recristallized from pentane to give a white solid, mp 140-42°C.

| | | | |
|---|---|---|---|
| Anal. Calcd. for C₃₈H₅₆N₂O₆ | C, 71.2; | H, 9.4; | N, 4.4. |
| Found | C, 71.4; | H, 9.1; | N, 4.2. |

### Example 16 alpha,alpha'-(Di-1-benzyloxy-2,2,6,6-tetramethylpiperidin-4-yloxy)-p-xylene

A mixture of 27.7 g (61.7 mmol) of alpha,alpha'-(di-1-hydroxy-2,2,6,6-tetramethylpiperidine-4-yloxy)-p-xylene, 44.4 g (18.5 mmol) of 97 % sodium hydride and 200 ml of tetrahydrofuran is gently refluxed under hydrogen evolution ceases. Benzyl bromide 31.6 g (185 mmol) is then added dropwise, and the reaction mixture is heated at reflux for 3 hours, then stirred overnight at room temperature. Excess sodium hydride is decomposed with methanol. Toluene (500 ml) is added, and the reaction mixture is filtered to remove salts. The filtrate is washed with water (3 x 1000 ml) and saturated sodium chloride (500 ml), then dried over magnesium sulfate and concentrated to give an oil. The oil is crystallized from methanol to give 29.7 g (77 % yield) of a white solid, m.p. 126-29°C.

| | | | |
|---|---|---|---|
| Anal. Calcd. for C₄₀H₅₆N₂O₄ | C, 76.4; | H, 9.0; | N, 4.5. |
| Found | C, 76.0; | H, 9.1; | N, 4.4. |

### Example 17 Di-(1-benzyloxy-2,2,6,8-tetramethylpiperidin-4-yl) Sebacate

A solution of 40.0 g (83 mmol) of di-(-2,2,6,6-tetramethylpiperidin-4-yl) sebacate in 130 ml of toluene is warmed to 80°C. Molybdenum hexacarbonyl (1.0 g) is added and a 5.0M solution of t-butyl hydroperoxide (266 ml, 1.33 mol) is also added over 15 minutes. The reaction mixture is irradiated for 24 hr. with a UV lamp while the internal temperature is maintained at 85-90 °C. The reaction mixture is filtered and the filtrate is evaporated until the volume is approximately 100 ml. The solution is chromatographed on silica gel (9:1 heptane: ethyl acetate) to obtain an oil which is crystallized from methanol. Recrystallization from ethanol gives 16.8 g (29 % yield) of a white solid, m.p. 64-68°C.

| | | | |
|---|---|---|---|
| Anal. Calcd. for C₄₂H₆₄N₂O₆ | C, 72.8; | H, 9.3; | N, 4.0. |
| Found | C, 72.7; | H, 9.2; | N, 4.0. |

### Example 18 Di-(1-benzyloxy-2,2,6,6-tetramethylpiperidin-4-yl) Phthalate

The compound is prepared according to the procedure given in Example 7, except that molybdenum hexacarbonyl is added prior to heating the reaction mixture. m.p. 141-43°C.

| | | | |
|---|---|---|---|
| Anal. Calcd. for C₄₀H₅₂N₂O₆ | C, 73.1; | H, 8.0; | N, 4.3. |
| Found | C, 73.0; | H, 7.7; | N, 4.2. |

### Example 19 Di-(1-benzyloxy-2,2,6,6-tetramethylpiperidin-4-yl) Isophthalate

A mixture of 35.0 g (78.7 mmol) of di-(2,2,6,6-tetramethylpiperidin-4-yl) isophthalate, 1.0 g of molybdenum hexacarbonyl, and 75 ml of toluene is heated to 90 C in a nitrogen atmosphere. A 4.2M solution of t-butyl hydroperoxide in toluene (225 ml, 945 mmol) is added over 5 min. The reaction mixture turns red. After the addition, the reaction mixture is irradiated for 6 hours (internal temp. 85°C) with a UV lamp. Another 1.0 g portion of molybdenum hexacarbonyl is added, and the reaction mixture is irradiated for 16 hours. The mixture is then filtered and concentrated. The crude residue is chromatographed on silica gel (9:1 hexane: ethyl acetate). The less polar of the two major products is recrystallized from 9:1 ethanol: dichloromethane to obtain 14.8 g (29 % yield) of a white solid, m.p. 135-141, which is the title compound.

| | | | |
|---|---|---|---|
| Anal. Calcd. for C₄₀H₅₂N₂O₆ | C, 73.1; | H, 8.0; | N, 4.3. |
| Found | C, 72.9; | H, 7.7; | N, 4.6. |

### Example 20 Di-(1-benzyloxy-2,2,6,6-tetramethylpiperidin-4-yl) Diethylmalonate

The compound is prepared from di-(2,26,6-tetramethylpiperidin-4-yl) diethylmalonate, t-butyl-hydroperoxide, toluene, and molybdenum hexacarbonyl according to the procedure given in Example 19. m.p. 122-23°C.

| | | | |
|---|---|---|---|
| Anal. Calcd. for C₃₉H₅₈N₂O₆ | C, 72.0; | H, 9.0; | N, 4.3. |
| Found | C, 72.0; | H, 9.3; | N, 4.7. |

### Example 21 Di[1-(alpha-methylbenzyloxy)-2,2,6,6-tetramethylpiperidin-4-yl] Phthalate

The compound is prepared from 40.0 g of di-(1-oxyl-2,2,6,6-tetramethylpiperidin-4-yl) phthalate, 200 ml of ethylbenzene, and 2.0 g of molybdenum oxide which are heated to 110°C (nitrogen atmosphere). Thereafter, 65 g of 70 % t-butyl hydroperoxide in water is added dropwise over one hour. The reaction mixture is refluxed for 3 hours after the hydroperoxide addition is complete. The crude product is chromatographed on silica gel (9:1 hexane: ethyl acetate) to give 51.0 g (88 % yield) of a soft glassy product.

| | | | |
|---|---|---|---|
| Anal. Calcd. for C₄₂H₅₆N₂O₆ | C, 73.7; | H, 8.2; | N, 4.1. |
| Found | C, 74.1; | H, 8.4; | N, 4.1. |

### Example 22 Di-(1-(α-methyl-benzyloxy)-2,2,6,6-tetramethylpiperidin-4-yl) Sebacate

A mixture of 40.0 g (83 mmol) of di-(2,2,6,6-tetramethylpiperidin-4-yl) sebacate, 2.0 g of molybdenum oxide, and 250 ml of ethylbenzene is heated to 110°C (nitrogen atmosphere). A commercially available solution of 70 % t-butyl hydroperoxide in water (64.3 g, 499 mmol) is added dropwise over 30 min. Water is collected in a Dean-Stark trap. Heating is continued for 90 minutes after the addition. The reaction mixture is filtered and evaporated. The resulting crude oil is dissolved in heptane (300 ml), and this solution is passed through a short column of silica gel. The first 350 ml of filtrate, nearly pure by TLC, is evaporated to give 41.7 g (70 % yield) of the title compound, a viscous oil.

| | | | |
|---|---|---|---|
| Anal. Calcd. for C₄₄H₆₈N₂O₆ | C, 72.8; | H, 9.6; | N, 3.9. |
| Found | C, 72.9; | H, 9.7; | N, 3.8. |

### Example 23 Di-(1-heptyloxy-2,2,6,6-tetramethylpiperidin-4-yl) Sebacate

A mixture of 35.0 g (72.8 mmol) of di-(2,2,6,6-tetramethylpiperidin-4-yl) sebacate, 58.3 g (582 mmol) of 90 % aqueous t-butyl hydroperoxide, 2.0 g of molybdenum trioxide, and 250 ml of heptane is heated at 140°C in a Fscher-Porter bottle. The pressure is maintained at 40-50 psi by occasional venting. Heating is discontinued after 7 hours. An additional portion (20.0 g) of 90 % t-butyl hydroperoxide is added and the reaction mixture is heated for one hour at 140°C. The reaction is nearly colorless by this time. The reaction mixture is cooled and filtered to remove the catalyst. The organic phase is separated, dried over magnesium sulfate, and concentrated to 100 ml total volume. This solution is passed through silica gel with heptane as the eluent. The filtrate is evaporated to yield 36.9 g (72 % yield) of the title compound, a nearly colorless oil.

| | | | |
|---|---|---|---|
| Anal. Calcd. for C₄₂H₈₀N₂O₆ | C, 71.1; | H, 11.4; | N, 3.95. |
| Found | C, 71.3; | H, 11.8; | N, 3.9. |

### Example 24 Di-(1-alpha-methylbenzyloxy-2,2,6,6-tetramethylpiperidin-4-yl) Terephthalate

A suspension of 40.0 g (90.0 mmol) of di-(2,2,6,6-tetramethylpiperidin-4-yl) terephthalate, 2.0 g of molybdenum trioxide, and 250 ml of ethylbenzene is heated to 110°C. t-butyl hydroperoxide (70 %, 69.5 g, 540 mmol) is rapidly added. No reaction is visible until water is removed by azeotropic distillation and the internal temperature reaches 115°C. Heating is continued for 6 hours. The nearly colorless reaction mixture is allowed to cool, then filtered and evaporated to yield a pink solid. The solid is recrystallized (9:1 2-propanol:methylene chloride) to yield 48.4 g of the title compound, a white solid, m.p. 150-152°C. A second crop of 5.3 g is obtained from the mother liquor. Total yield 53.7 g (87 % yield).

| | | | |
|---|---|---|---|
| Anal. Calcd. for C₄₂H₅₆N₂O₆ | C, 73.7; | H, 8.2; | N, 4.1. |
| Found | C, 74.0; | H, 8.2; | N, 4.0. |

### Example 25 Di-(1-alpha-methylbenzyloxy-2,2,6,6-tetramethylpiperidin-4-yl) Isophthalate

A mixture of 40.0 g (90.0 mmol) of di-(2,2,6,6-tetramethylpiperidin-4-yl) isophthalate, 2.0 g of molybdenum trioxide, and 250 ml of ethylbenzene is heated to 110°C. t-butyl hydroperoxide (70 %, 69.5 g, 540 mmol) is added dropwise over a 45 min. period. The reaction mixture turns red during the addition. Water is removed by azeotropic distillation. The mixture is refluxed for 4 hours after the addition is complete. The catalyst is filtered, and the filtrate is evaporated to obtain a yellow oil. A kugelrohr distillation (110°C, 0.1 mm Hg) is performed to remove volatile by-products. The residue, a viscous oil, is dissolved in hexane and passed through silica gel. Evaporation yields a crude solid which is recrylstallized from ethanol to yield 39.8 g (65 % yield) of the title compound, a white powder, m.p. 118-34°C.

| | | | |
|---|---|---|---|
| Anal. Calcd. for C₄₂H₅₆N₂O₆ | C, 73.7; | H, 8.2; | N, 4.1. |
| Found | C, 73.4; | H, 8.3; | N, 4.1. |

### Example 26 Di-(1-ethoxy-2,2,6,6-tetramethylpiperidin-4-yl) Sebacate

A solution of 7.8 g (32.5 mmol) of sebacoyl chloride in 20 ml of dichloromethane is added dropwise over 15 min. to a solution of 13.1 g (65.1 mmol) of 1-ethoxy-4-hydroxy-2,2,6,6-tetramethylpiperidine, 7.0 g of triethylamine and 100 ml of dichloromethane. The reaction mixture begins to reflux during the addition. The reaction is gently refluxed for an additional hour. Ether (500 ml) is added, the precipitate filtered and the filtrate washed with 1N HCI (2 x 100 ml), water (200 ml), and saturated sodium bicarbonate (300 ml). The organic solution is dried over magnesium sulfate and evaporated to obtain an oil. Purification by chromatography (silica gel, 19:1 hexane: ethyl acetate) affords 12.7 g (69 % yield) of the title compound, a colorless oil.

| | | | |
|---|---|---|---|
| Anal. Calcd. for C₃₂H₅₀N₂O₆ | C, 67.6; | H, 10.6; | N 4.9. |
| Found | C, 67.3; | H, 10.8; | N, 4.8. |

### Example 27 Di(1-cumyloxy-2,2,6,6-tetramethylpiperidin-4-yl) Sebacate

The title compound is prepared according to the procedure in Example 26 except that the 1-cumyloxy reactant is utilized. The reaction temperature reaches 33°C during the addition, and the reaction mixture is then stirred for 1 hour at ambient temperature. The product is a white solid, m.p. 94-6 °C.

| | | | |
|---|---|---|---|
| Anal. Calcd. for C₄₆H₇₂N₂O₆ | C, 73.76; | H, 9.69; | N, 3.74. |
| Found | C, 74.0; | H, 9.8; | N, 3.8. |

### Example 28 8-alpha-Methylbenzyloxy-7,7,9,9-tetramethyl-8-aza-1,4-dioxaspiro[4.5]decane

A mixture of 38.1 g (191 mmol) of 7,7,9,9-tetramethyl-8-aza-1,4-dioxaspiro[4.5]decane, 73.8 g (574 mmol) of 70 % aq. t-butyl hydroperoxide, 2.0 g of molybdenum trioxide, and 130 ml of ethylbenzene is refluxed for 6 hours. Water is collected in a Dean-Stark trap. The catalyst is filtered and the filtrate concentrated at reduced pressure. The residue is dissolved in heptane and passed through silica gel. A Kugelrohr distillation (120°C, 0.1 mm Hg) is used to remove volatile by-products. The title compound crystallizes on standing.

| | | | |
|---|---|---|---|
| Anal. Calcd. for C₁₉H₂₉NO₃ | C, 71.4; | H, 9.1; | N, 4.4. |
| Found | C, 70.3; | H, 9.2; | N, 4.4. |

### Example 29 3,15-Di-alpha-methylbenzyloxy-2,2,4,4,14,14,16,16-octamethyl-7,11,18,21-tetraoxa-3,15-diazatrispiro-[5.2.2.5.2.2.]-heneicosane

The title compound is prepared from 2,2,4,4,14,14,16,16-octamethyl-7,11,18,21-tetraoxa-3,15-diazatrispiro-[5.2.2.5.2.2.]heneicosane according to the procedure given in Example 28. The catalyst is filtered and the filtrate is concentrated to yield an oil which is cristallized from ethanol to give 19.6 g (65 % yield) of a white powder, m.p. 150-53°C.

### Example 30 3,15-Dicyclohexyloxy-2,2,4,4,14,14,16,16-octamethyl-7,11,18,21-tetraoxa-3,15-diazatrispiro-[5.2.2.5.2.2.]-heneicosane

A mixture of 16.7 g (37.9 mmol) of 3,15-dioxyl 2,2,4,4,14,14,16,16-octamethyl-7,11,18,21-tetraoxa-3,15-diazatrispiro[5.2.2.5.2.2.]heneicosane, 22.8 g (227 mmol) of 90 % aq t-butyl hydroperoxide, 2.0 g of molybdenum trioxide, and 125 ml of cyclohexane is heated in a Fischer-Porter bottle at 155-160°C (bath temperature) for 6 hours. The pressure is maintained at 40-50 psi by occasional venting. The catalyst is filtered and the filtrate is concentrated. The residue is dissolved in hexane and passed through silica gel. Crystallization from 2-propanol yields 8.0 g (35 %) of a white solid, m.p. 163-175°C

| | | | |
|---|---|---|---|
| Anal. Calcd. for C₃₅N₆₂N₂O₆ | C, 69.3; | H, 10.3; | N, 4.6. |
| Found | C, 68.7; | H, 10.3; | N, 4.7. |

### Example 31 Di-(1-methoxy-2,2,6,6-tetramethylpiperidin-4-yl) n-Butylmalonate

A mixture of diethyl n-butylmalonate (11.5 g, 53.4 mmol), 4-hydroxy-1-methoxy-2,2,6,6-tetramethylpiperidine (20.0 g, 107 mmol), lithium amide (120 mg), and xylene (100 ml) is distilled until the distillate reaches a constant temperature of 137°C. Xylene is evaporated and the residue is dissolved in heptane. Acetic acid is added and the resulting precipitate is filtered. The filtrate is concentrated to obtain 26.2 g (98 % yield) of a light yellow oil.

| | | | |
|---|---|---|---|
| Anal. Calcd. for C₂₇H₅₀N₂O₆ | C, 65.0; | H, 10.1; | N, 5.6. |
| Found | C, 65.0; | H, 10.4; | N, 5.5. |

### Example 32 Di-(1-methoxy-2,2,6,6-tetramethylpiperidin-4-yl) (3,5-Di-t-butyl-4-hydroxybenzyl)-n-butylmalonate

A mixture of 11.9 g, (45.2 mmol) of N,N-dimethyl-3,5-di-t-butyl-4-hydroxybenzylamine, 18.8 g (37.7 mmol) of di-(1-methoxy-2,2,8,8-tetramethylpiperidin-4-yl)n-butylmalonate, 173 mg of lithium amide, and 100 ml of tetrahydrofuran is refluxed for 90 minutes. The reaction mixture is diluted with ethyl acetate (350 ml). The organic solution is washed with 1N HCl (2x100 ml), water (2x250 ml), and saturated NaHCO₃ solution (250 ml), then dried over magnesium sulfate and evaporated to obtain a brown oil. Crystallization from 9:1 methanol:water affords 14.9 g (55 % yield) of a white solid (m.p. 111-13°C).

| | | | |
|---|---|---|---|
| Anal. Calcd. for C₄₂H₇₂N₂O₇ | C, 70.3; | H, 10.1; | N, 3.9. |
| Found | C, 70.2; | H, 10.2; | N, 3.9. |

### Example 33 Di-(1-cyclohexyloxy-2,2,6,6-tetramethylpiperidin-4-yl) n-Butylmalonate

The title compound, a viscous oil, is prepared in 89 % yield following the procedure given in Example 31 utilizing the 1-cyclohexyloxy starting material.

| | | | |
|---|---|---|---|
| Anal. Calcd. for C₃₇H₆₆N₂O₆ | C, 70.0; | H, 10.5; | N, 4.4. |
| Found | C, 69.8; | H, 10.7; | N, 4.4. |

### Example 34 Di-(1-cyclohexyloxy-2,2,6,6-tetramethylpiperidin-4-yl) (3,5-Di-t-butyl-4-hydroxybenzyl)-n-butylmalonate

The title compound, a white crystalline solid, is prepared in 80 % yield following the procedure given in Example 32 utilizing the 1-cyclohexyloxy material, m.p. 184-5°C (ethyl acetate).

| | | | |
|---|---|---|---|
| Anal. Calcd. for C₅₂H₈₈N₂O₇ | C, 73.2; | H, 10.4; | N, 3.3. |
| Found | C, 73.7; | H, 10.8; | N, 3.3. |

### Example 35 Poly-{(6-(1,1,3,3-tetramethylbutyl)-imino]-1,3,5-triazine-2,4-diyl] [2-(1-cyclohexyloxy-2,2,6,6-tetramethylpiperidyl)-imino]-hexamethylene-[4-(1-cyclohexyloxy-2,2,6,6-tetramethylpiperidyl)-imino]}

A mixture of 46.9 g of N-oxyl precursor, 40.2 g (402 mmol) of 90 % t-butyl hydroperoxide, 6.0 g of molybdenum oxide, and 200 ml of cyclohexane is heated for 3 hours at 155°C under pressure. The colorless reaction mixture is diluted with a mixture of ether, methylenen chloride, and toluene, and filtered. The filtrate is stirred with 5 % aqueous sodium sulfite (400 ml) for 45 minutes. The organic phase is washed with water, dried with magnesium sulfate, and filtered. After the filtrate is concentrated, methanol is added to precipitate the product. The yield is 19.2 g (36 %) of a white powder, m.p. 187-200°C.

| | | | |
|---|---|---|---|
| Anal. Calcd. for (C₄₇H₈₆N₈O₂)ₙ | C, 71.0; | H, 10.9; | N, 14.1. |
| Found | C, 68.2; | H, 10.6; | N, 14.0. |

### Example 36 Di-(1-cyclohexyloxy-2,2,6,6-tetramethylpiperidin-4-yl) Succinate

A two-phase mixture of 70 % aqueous t-butyl hydroperoxide (103.9 g, 807 mmol), cyclohexane (200 ml) and sodium chloride (15 g) is shaken in a separatory funnel. The organic phase is dried over magnesium sulfate, filtered, and added to 40.0 g of di-(2,2,6,6-tetramethylpiperidin-4-yl) succinate. Molybdenum oxide (2.0 g) is added, and the mixture is refluxed for one hour. Water is collected in a Dean-Stark trap. The entire reaction mixture is then transferred to a Fischer-Porter bottle and heated at 140 °C for 6 hours. Additional t-butyl hydroperoxide (90 %, 10.1 g, 101 mmol) is added and heating is resumed for another 4 hours. The colorless reation mixture is filtered, concentrated, and dissolved in heptane (20.0 ml). The heptane solution is passed through a short column of silica gel with heptane. Subsequent evaporation affords an oil which is crystallized from ethanol to yield 41.2 g (69 %) of a white powder, m.p. 122-6°C.

| | | | |
|---|---|---|---|
| Anal. Calcd. for C₃₄H₆₀N₂O₆ | C, 68.9; | H, 10.2; | N, 4.7. |
| Found | C, 68.4; | H, 10.5; | N, 4.5. |

### Example 37 Di-(1-alphy-methylbenzyloxy-2,2,6,6-tetramethylpiperidin-4-yl) Succinate

The title compound is prepared following the procedure given in Example 25 utilizing the succinate starting material. Crystallization from ethanol affords a 78 % yield of a white solid, m.p. 85-88°C.

| | | | |
|---|---|---|---|
| Anal. Calcd. for C₃₈H₅₆N₂O₆ | C, 71.7; | H, 8.9; | N, 4.4. |
| Found | C, 71.5; | H, 8.6; | N, 4.3. |

### Example 38 Di-(1-nonyloxy-2,2,6,6-tetramethylpiperidin-4-yl) Sebacate

The title compound is prepared following the procedure given in Example 36 using the sebacate starting material and nonane, except that the reaction mixture is refluxed for 22 hours at atmospheric pressure. The crude product is passed through a short column of silica gel with heptane as the eluent to obtain a 73 % yield of a colorless oil.

| | | | |
|---|---|---|---|
| Anal. Calcd. for C₄₆H₈₈N₂O₆ | C, 72.2; | H, 11.6; | N, 3.7. |
| Found | C, 71.6; | H, 11.5; | N, 4.7. |

### Example 39 Di-(1-octadecyloxy-2,2,6,6-tetramethylpiperidin-4-yl) Sebacate

The reaction is run in a Fischer-Porter bottle in a nitrogen atmosphere. The reaction vessel is charged with 15.0 g (31.2 mmol) of di-(2,2,6,6-tetramethylpiperidin-4-yl) sebacate, 101 g of octadecane, 25.3 g (253 mmol) of 90 % t-butyl hydroperoxide, and 1.25 g of molybdenum trioxide. The Fischer-Porter bottle is placed in an oil bath and the bath temperature is brought to 143°C over 1.3 hours. Heating is continued another 3.2 hours at 145 ± 3 C. The colorless reaction mixture is cooled to room temperature, diluted with hexane (100 ml), and filtered to remove solids. The solids are rinsed with hexane (2 x 50 ml). The organic solution is stirred for 90 minutes with 16.1 g sodium sulfite in 200 ml of water to decompose unreacted hydroperoxide. Ethyl acetate is added (200 ml), and the organic solution is washed with water (4 x 250 ml), dried over magnesium sulfate, and concentrated to obtain 121 g of a colorless oil. The crude material is purified by flash chromatography (silica gel; heptane; then 20:1 heptane:ethyl acetate) to afford 20.8 g (66 % yield) of the title compound as a colorless oil.

| | | | |
|---|---|---|---|
| Anal. Calcd. for C₆₄H₁₂₄N₂O₆ | C, 75.5; | H, 12.3; | N, 2.75. |
| Found | C, 75.1; | H, 12.6; | N, 3.2. |

### Example 40 Di-(1-cyclohexyloxy-2,2,6,6-tetramethylpiperidin-4-yl) Phthalate

A mixture of 30.0 g (67.5 mmol) of di-2,2,6,6-tetramethylpiperidin-4-yl) phthalate, 27.5 g (214 mmol) of 70 % aqueous t-butyl hydroperoxide, 2.0 g of molybdenum trioxide and 200 ml of cyclohexane is heated to reflux.

Water is collected in a Dean-Stark trap. After 75 min., the reaction mixture becomes red. Another portion of t-butyl hydroperoxide (42.5 g, 70 %, 330 mmol) is added over 30 minutes. After the additional water is collected, the reaction mixture is transferred to a Fischer-Porter bottle and heated at 140°C for 4.5 hours. The nearly colorless reaction mixture is treated with 6.9 g (90 %, 69 mmol) of t-butyl hydroperoxide and heated at 140°C for 90 minutes to remove the last traces of pink color. The reaction mixture is cooled, filtered, and stirred with a solution of 43 g of sodium sulfite in 530 ml of water for 2 hours. Dichloromethane (600 ml) is added, and the organic layer is separated, dried over magnesium sulfate, and concentrated to obtain a crude solid. Purification (Waters Prep. 500A HPLC, 25:1 heptane:ethyl acetate) affords 31.0 g (72 % yield) of a white solid, m.p. 149-51 °C.

| | | | |
|---|---|---|---|
| Anal. Calcd. for C₃₈H₆₀N₂O₆ | C, 71.2; | H, 9.2; | N, 4.4. |
| Found | C, 71.1; | H, 9.3; | N, 4.3. |

### Example 41 1-Cyclohexyloxy-2,2,6,6-tetramethylpiperidine-4-yl 1-Methoxy-2,2,6,6-tetramethylpiperidin-4-yl Phthalate

The title compound, a glass, is obtained as a by-product in Example 40.
Mass spec.: M⁺ = 572

### Example 42 1-Cyclohexyloxy-4-(n-dodecylamino)-2,2,6,6-tetramethylpiperidine

Acetic acid (22.0 g, 367 mmol) is added dropwise to a solution of 15.0 g (59.2 mmol) of 1-cyclohexyloxy-2,2,6,6-tetramethylpiperidin-4-one and 54.9 g (296 mmol) of dodecylamine in 200 ml of dry tetrahydrofurane containing 5A molecular sieves (25 g). The reaction mixture warms during the addition. The mixture is then diluted with tetrahydrofuran (150 ml) and cooled to 21°C. Sodium cyanoborhydride (4.46 g, 7.1 mmol) is added in one portion. The reaction mixture is stirred at room temperature for 4 hours, then filtered. The filtrate is concentrated and the residue is partitioned between ether (400 ml) and 5 % sodium hydroxide (250 ml). The organic layer is dried over magnesium sulfate, filtered, and concentrated. Residual dodecylamine is removed by Kugelrohr distillation (110°C, 0.3 mm). The crude product is purified by chromatography (silica gel) to afford 20.4 g (82 % yield) of the title compound, as a nearly colorless oil.

| | | | |
|---|---|---|---|
| Anal. Calcd. for C₂₇H₅₄N₂O | C, 76.7; | H, 12.9; | N, 6.6. |
| Found | C, 76.7; | H, 13.2; | N, 6.7. |

### Example 43 1-alpha-Methylbenzyloxy-4-(n-dodecylamino)-2,2,6,6-tetramethylpiperidine

The title compound, a colorless oil, is prepared from 1-alpha-methylbenzyloxy-2,2,6,6-tetramethylpiperidin-4-one and dodecylamine according to the procedure given in Example 42.

| | | | |
|---|---|---|---|
| Anal. Calcd. for C₂₉H₅₂N₂O | C, 78.3; | H, 11.8; | N, 6.3. |
| Found | C, 77.7; | H, 11.6; | N, 6.6. |

### Example 44 1-alpha-Methylbenzyloxy-4-(n-butylamino)-2,2,6,6-tetramethylpiperidine

The title compound, a colorless oil, is prepared from 1-alpha-methylbenzyloxy-2,2,6,6-tetramethylpiperidin-4-one and butylamine according to the procedure given in Example 42 except that acetonitrile is substituted for tetrahydrofuran and the molecular sieves are omitted.

| | | | |
|---|---|---|---|
| Anal. Calcd. for C₂₁H₃₆N₂O | C, 75.8; | H, 10.9; | N, 8.4. |
| Found | C, 74.7; | H, 11.0; | N, 8.6. |

### Example 45 N,N'-Di-(1-cyclohexyloxy-2,2,6,6-tetramethylpiperidin-4-yl)-1,6-hexanediamine

A mixture of 15.0 g (59.1 mmol) of 1-cyclohexyloxy-2,2,6,6-tetramethylpiperidin-4-one, 3.4 g of hexanediamine, 500 mg of platinum oxide, and 150 g of ethanol is hydrogenated in Paar apparatus for 4 hours. The calalyst is removed by filtration. The filtrate is concentrated to an oil which is purified by flash chromatography (silica gel:ethyl acetate, then ethyl acetate:methanol) to afford 9.7 g (55 % yield) of the title compound, as a yellow oil.

| | | | |
|---|---|---|---|
| Anal. Calcd. for C₃₆H₇₀N₄O₂ | C, 73.2; | H, 11.9; | N, 9.5. |
| Found | C, 72.7; | H, 12.1; | N, 9.3. |

### Example 46 1-Cyclohexyloxy-4-(n-butylamino)-2,2,6,6-tetramethylpiperidine

The title compound, a colorless oil, is prepared from 1-cyclohexyloxy-2,2,6,6-tetramethylpiperidin-4-one and butylamine according to the procedure given in Example 45.

| | | | |
|---|---|---|---|
| Anal. Calcd. for C₁₉H₃₈N₂O | C, 73.5; | H, 12.3; | N, 9.0. |
| Found | C, 73.0; | H, 12.6; | N, 8.6. |

### Example 47 Di-(1-nonyloxy-2,2,6,6-tetramethylpiperidin-4-yl) Succinate

The title compound, a light yellow oil, is prepared from di-(2,2,6,6-tetramethylpiperidin-4-yl) succinate and nonane according to the procedure given in Example 36.

| | | | |
|---|---|---|---|
| Anal. Calcd. for C₄₆H₇₆N₂O₆ | C, 70.5; | H, 11.2; | N, 4.1. |
| Found | C, 70.6; | H, 11.3; | N, 4.0. |

### Example 48 Di-(1-decyloxy-2,2,6,6-tetramethylpiperidin-4-yl) Sebacate

t-Butyl hydroperoxide (55.0 g of a 70 % aqueous solution, 427 mmol) is added dropwise over 15 minutes to a mixture of 25.0 g (52.0 mmol) of di-(2,2,6,6-tetramethylpiperidin-4-yl) sebacate, 1.5 g (10.4 mmol) of molybdenum trioxide, and 225 ml of n-decane which has been heated to 90°C. The reaction mixture is refluxed for 7.5 hours, and water is collected in a Dean-Stark trap. The reaction mixture is cooled to room temperature, and then stirred for 2 hours with a solution of 26 g of sodium sulfite in 500 ml of water. The reaction mixture is diluted with ethyl acetate (200 ml). The organic layer is dried over magnesium sulfate and concentrated to an oil. The crude product is purified by flash chromatography (silica gel; 97:3 heptane:ethyl acetate) to afford 29.2 g (71 % yield) of the title compound, as a colorless oil.

| | | | |
|---|---|---|---|
| Anal. Calcd. for C₄₈H₉₂N₂O₆ | C, 72.7; | H, 11.7; | N, 3.5. |
| Found | C, 73.1; | H, 12.2; | N, 3.5. |

### Example 49 Di-(1-dodecyloxy-2,2,6,6-tetramethylpiperidin-4-yl) Sebacate

The title compound, a colorless oil, is prepared from di-(2,2,6,6-tetramethylpiperidin-4-yl) sebacate and n-dodecane according to the procedure given in Example 48.

| | | | |
|---|---|---|---|
| Anal. Calcd. for C₅₂H₁₀₀N₂O₆ | C, 73.5; | H, 11.9; | N, 3.3. |
| Found | C, 73.2; | H, 12.2; | N, 3.2. |

### Example 50 Di-[1-(1-Methylcyclohexyloxy)-2,2,6,6-tetramethylpiperidin-4-yl) Sebacate

t-Butyl hydroperoxide (70 % 133.9 g 1.04 mol), methylcyclohexane (250 ml), and sodium chloride (20 g) are agitated in a separatory funnel. The organic layer is dried over mangesium sulfate. The t-butyl hydroperoxide-methylcyclohexane solution is mixed with 50.0 g (104 mmol) of di-(2,2,6,6-tetramethylpiperidin-4-yl) sebacate, 3.0 g of molybdenum trioxide, and 100 ml of methylcyclohexane. The reaction mixture is heated at reflux for 4.5 hours and water is collected in a Dean-Stark trap. The reaction is cooled to room temperature and filtered. The filtrate is concentrated at reduced pressure to obtain oil which is purified by flash chromatography (silica gel; 19:1 heptane:ethyl acetate) to obtain 51.6 g (72 % yield) of a colorless oil.

| | | | |
|---|---|---|---|
| Anal. Calcd. for C₄₂H₇₆N₂O₆ | C, 71.6; | H, 10.9; | N, 4.0. |
| Found | C, 71.4; | H, 11.0; | N, 3.9. |

### Example 51

### Step A 4-Benzoyloxy-1-(2-cyclohexen-1-yloxy)2,2,6,6-tetramethylpiperidine

A solution of 33.6 g (122 mmol) of 4-benzoyloxy-1-oxyl-2,2,6,6-tetramethylpiperidine, 23.0 g (157 mmol) of di-t-butyl peroxide, and 70 ml of cyclohexene is heated in a Fischer-Porter bottle at 138°C for 6.5 hours. The reaction mixture is chromatographed on silica gel (200:1 heptane:ethyl acetate) to afford 35.1 g (81 % yield) of a colorless oil.

| | | | |
|---|---|---|---|
| Anal. Calcd. for C₂₂H₃₁NO₃ | C, 73.9; | H, 8.7; | N, 3.9. |
| Found | C, 73.7; | H, 8.8; | N, 3.9. |

### Step B 1-(2-Cyclohexen-1-yloxy)-4-hydroxy-2,2,6,6-tetramethylpiperidine

The title compound, a white solid (m.p. 66-9 °C) is prepared by the hydrolysis (KOH-water-methanol) of 4-benzoyloxy-1-(2-cyclohexen-1-yloxy)-2,2,6,6-tetramethylpiperidine.

| | | | |
|---|---|---|---|
| Anal. Calcd for C₁₅H₂₇NO₂ | C, 71.1; | H, 10.7; | N, 5.5. |
| Found | C, 71.7; | H, 11.4; | N, 5.5. |

### Step C Di-[1-(2-cyclohexen-1-yloxy)-2,2,6,6-tetramethylpiperidin-4-yl] Sebacate

The title compound, a colorless oil, is prepared from the reaction of 1-(2-cyclohexen-1-yloxy)-4-hydroxy-2,2,6,6-tetramethylpiperidine and sebacoyl chloride according to the procedure given in Example 26.

| | | | |
|---|---|---|---|
| Anal. Calcd. for C₄₀H₆₈N₂O₆ | C, 71.4; | H, 10.2; | N, 4.2. |
| Found | C, 71.6; | H, 10.7; | N, 4.0. |

### Example 52

### Step A 4-Benzoyloxy-1-t-butoxy-2,2,6,6-tetramethylpiperidine

A solution of 56.0 g (203 mmol) of 4-benzoyloxy-1-oxyl-2,2,6,6-tetramethylpiperidine, 125 ml of chlorobenzene, and 58.9 g of t-butyl iodide is cooled to 5°C. Tri-n-butyltin hydride (29 g, 100 mmol) is added dropwise over 110 minutes while the temperature is maintained below 20 °C. The reaction mixture is concentrated at reduced pressure and then purified by silica gel chromatography (200:1, then 100:3 heptane:ethyl acetate) to afford 30.1 g of a white solid (m.p. 80-82.5°C).

| | | | |
|---|---|---|---|
| Anal. Calcd. for C₂₀H₃₁NO₃ | C, 72.0; | H, 9.4; | N, 4.2. |
| Found | C, 71.9; | H, 9.6; | N, 4.1. |

### Step B 1-t-Butoxy-4-hydroxy-2,2,6,6-tetramethylpiperidine

The title compound, a white solid (m.p. 115-16°C) is prepared by the hydrolysis (KOH-water-methanol) of 4-benzoyloxy-1-t-butoxy-2,2,6,6-tetramethylpiperidine.

| | | | |
|---|---|---|---|
| Anal. Calcd. for C₁₃H₂₇NO₂ | C, 68.1; | H, 11.9; | N, 6.1. |
| Found | C, 68.5; | H, 12.4; | N, 6.1. |

### Step C Di-(1-t-butoxy-2,2,6,6-tetramethylpiperidin-4-yl) Sebacate

The title compound, a white solid (m.p. 62-3°C), is prepared from the reaction of 1-t-butoxy-4-hydroxy-2,2,6,6-tetramethylpiperidine and sebacoyl chloride according to the procedure given in Example 26.

| | | | |
|---|---|---|---|
| Anal. Calcd. for C₃₆H₆₈N₂O₆ | C, 69.2; | H, 11.0; | N, 4.4. |
| Found | C, 69.5; | H, 11.3; | N, 4.4. |

### Example 53 4-Acetamido-1-cyclohexyloxy-2,2,6,6-tetramethylpiperidine

A stirred mixture of 10.0 g of 4-acetamido-2,2,6,6-tetramethylpiperidine-1-oxyl, 16 ml of 70 % aqueous tert-butylhydroperoxide and 0.67 g of molybdenum trioxide in 75 ml of cyclohexane is heated under reflux in a flask fitted with a Dean-Stark apparatus. After 6 ml of water is collected, the reaction mixture is transferred to a Fischer-Porter apparatus and heated at 140°C and 30 psi for 4 hours. The decolorized reation mixture is filtered and the filtrate is washed with water, aqueous sodium sulfite, brine, dried (MgSO₄) and concentrated to give 13.61 g of a white solid. Recrystallization from heptane affords 10.39 g of title compound as a white crystalline solid, m.p. 140-44°C.

| | | | |
|---|---|---|---|
| Anal. Calcd. for C₁₇H₃₂N₂O₂ | C, 68.9; | H, 10.9; | N, 9.5. |
| Found | C, 68.6; | H, 11.3; | N, 9.3. |

### Example 54 4-Amino-1-cyclohexyloxy-2,2,6,6-tetramethylpiperidine

A solution of 5.0 g of the acetamide from Example 53 in 7.5 ml of water and 7.5 ml of conc. hydrochloric acid is heated at reflux for 10 hours. The reaction mixture is then quenched with saturated sodium carbonate and extracted with ethyl acetate. The combined organic extracts is washed with water, brine, dried (MgSO₄) and evaporated to leave a light brown oil. Distillation (Kugelrohr, 140°C @ 1.5 mm) affords the title compound as a clear colorless oil.

### Example 55 N,N'-Bis[1-cyclohexyloxy-2,2,6,6-tetramethylpiperidin-4-yl] Succinamide

A mixture of 1-cyclohexyloxy-4-amino-2,2,6,6-tetramethylpiperidine (2.65 g), dimethyl succinate (1.46 g) and sodium methoxide (50 mg) is heated at 180-190°C for 4 hours. The crude product is recrystallized from ethanol/water to afford 1.38 g of the title compound as a white solid, m.p. 230-34°C.

| | | | |
|---|---|---|---|
| Anal. Calcd. for C₃₄H₆₂N₄O₄.H₂O | C, 67.1; | H, 10.6; | N, 9.2. |
| Found | C, 66.8; | H, 10.7; | N, 9.1. |

### Example 56 Bis(1-methoxy-2,2,6,6-tetramethylpiperidin-4-yl)succinate

A mixture of 20.00 g (107 mmol) of 4-hydroxy-1-methoxy-2,2,6,6-tetramethylpiperidine, 7.41 g (50.7 mmol) of dimethyl succinate, 0.08 g of lithium amide, and 100 ml of toluene is heated at reflux for 7 hours. Methanol is distilled from the reaction mixture along with some of the toluene. The reaction mixture is cooled and a solution of 0.21 g of glacial acetic acid in toluene is added. The precipitate is removed by filtration. The filtrate is concentrated to give an oil which is purified by HPLC (Prep. 500 A, 29:1 heptane:ethyl acetate) to afford 14.5 g (63 % yield) of the title compound.

| | | | |
|---|---|---|---|
| Anal. Calcd. for C₂₄H₄₄N₂O₆ | C, 63.1; | H, 9.7; | N, 6.1. |
| Found | C, 63.1; | H, 9.9; | N, 6.0. |

### Example 57 Bis(1-octyloxy-2,2,6,8-tetramethylpiperidin-4-yl)succinate

A solution of 143.1 g (1.11 mol) of 70 % aqueous t-butyl hydroperoxide is added over a four hour period to a refluxing mixture of 55.0 g (139 mmol) of bis(2,2,6,6-tetramethylpiperidin-4-yl)succinate, 1.0 g of molybdenum trioxide, and 350 ml of n-octane. The reaction mixture is heated at reflux for 16 hours after the addition is complete in order to discharge the red color. The mixture is filtered to remove solids. The filtrate is concentrated to obtain a residue which is purified by flash chromatography to afford 53.6 g (59 % yield) of the title compound, a yellow oil.

| | | | |
|---|---|---|---|
| Anal. Calcd. for C₃₈H₇₂N₂O₆ | C, 69.9; | H, 11.1; | N, 4.3. |
| Found | C, 70.0; | H, 11.7; | N, 4.4. |

### Example 58 Bis(1-methoxy-2,2,6,6-tetramethylpiperidin-4-yl)terephthalate

A mixture of 7.36 g (16.6 mmol) of bis(2,2,6,6-tetramethylpiperidin-4-yl)terephthalate, 0.1 g of molybdenum trioxide, and 25 ml of chlorobenzene is heated to 130°C in a nitrogen atmosphere. A solution of 40.2 g (132 mmol) of cumene hydroperoxide in chlorobenzene is added to the reaction mixture over one hour. A Dean-Stark trap is used to remove water from the reaction. The reaction is heated at reflux for 4 hours after the addition is complete, then cooled and filtered. The filtrate is concentrated and the residue purified by filtration through silica gel with 19:1 heptane:ethyl acetate as the eluent. Crystallization from methanol affords 5.0 g (60 % yield) of the title compound, a white powder, m.p. 179-81°C.

| | | | |
|---|---|---|---|
| Anal. Calcd. for C₂₈H₄₄N₂O₆ | C, 66.6; | H, 8.8; | N, 5.5. |
| Found | C, 66.7; | H, 8.9; | N, 5.4. |

### Example 59 1 -Methoxy-4-n-butylamino-2,2,6,6-tetramethylpiperidine

A mixture of 10.1 g (54.5 mmol) of 1-methoxy-2,2,6,6-tetramethylpiperidin-4-one, 27.9 g (382 mmol) of n-butyl amine, 100 ml of methanol, and 1.0 g of 5 % platinum on carbon is hydrogenated (50 psi, 25 °C) for 5 hours. The catalyst is removed by filtration. Evaporation of the filtrate gives an oil which is purified by fractional distillation to afford 10.6 g (80 % yield) of the title compound, a colorless oil, b.p. 93-100°C (0.25 mm).

| | | | |
|---|---|---|---|
| Anal. Calcd. for C₁₄H₃₀N₂O | C, 69.4; | H, 12.5; | N, 11.6 |
| Found | C, 68.4; | H, 12.2; | N, 11.2 |

### Example 60 Bis[N-(1-cyclohexyloxy-2,2,6,6-tetramethylpiperidin-4-yl)-N-butyl]sebacamide

70 % aq. t-butyl hydroperoxide (41.4 g, 321 mmol) is partitioned between 200 ml of cyclohexane and 50 ml of saturated sodium chloride. A mixture of t-butyl hydroperoxide/cyclohexane solution, 19.0 g (32 mmol) of bis[N-(2,2,6,6-tetramethylpiperidin-4-yl)-N-butyl]sebacamide, and 0,4 g of molybdenum trioxide is heated in a Fischer-Porter pressure bottle at 150-160°C for 2 hours. The reaction mixture is filtered and the filtrate evaporated. The residual oil is purified by flash chromatography on silica gel (3:1 heptane:ethyl acetate). Crystallization from methanol affords 13.1 g (52 % yield) of the title compound, a white solid, m.p. 124-28°C.

| | | | |
|---|---|---|---|
| Anal. Calcd. for C₄₈H₉₀N₄O₄ | C, 73.2; | H, 11.5; | N, 7.1. |
| Found | C, 72.7; | H, 11.7; | N, 7.0. |

### Example 61 Bis[N-(1-octyloxy-2,2,6,6-tetramethylpiperidin-4-yl)-N-butyl]sebacamide

A solution of 70 % aq. t-butyl hydroperoxide (33.0 g, 256 mmol) is saturated with sodium chloride and extracted with 200 ml of n-octane. A mixture of 19.0 g (32 mmol) of bis[N-(22,66-tetramethylpiperidin-4-yl)-N-butyl]sebacamide, 0,2 g of molybdenum trioxide, and one-half of the t-butyl hydroperoxide/octane solution is heated at reflux for 30 minutes. Water is collected in a Dean-Stark trap. The remainder of the t-butyl hydroperoxide/octane solution is then added to the refluxing reaction mixture over 2 hours. The reaction mixture is heated at reflux an additional 4.5 hours, then treated with 20.0 g (155 mmol) of 70 % aq. t-butyl hydroperoxide and heated at reflux for two more hours to discharge the red color. Solids are removed by filtration, and the filtrate is evaporated.
Purification of the crude product by flash chromatography on silica gel (4:1 heptane:ethyl acetate) affords 16.0 g (59 % yield) of the title compound, a pale yellow oil.

| | | | |
|---|---|---|---|
| Anal. Calcd. for C₅₂H₁₀₂N₄O₄ | C, 73.7; | H, 12.1; | N, 6.6. |
| Found | C, 74.0; | H, 12.0; | N, 6.5. |

### Example 62 1,6-Bis[N-(1-cyclohexyloxy-2,2,6,6-tetramethylpiperidin-4-yl)-acetylamino]hexane

A mixture of 8.0 g (16.7 mmol) of 1,6-bis[N-(2,2,6,6-tetramethylpiperidin-4-yl)-acetylamino]hexane, 21.5 g (167 mmol) of 70 % aqueous t-butyl hydroperoxide, 0.1 g of molybdenum trioxide, and 100 ml of cyclohexane is heated at reflux for two hours. Water is collected in a Dean-Stark trap. The red reaction mixture is transferred to a Fischer-Porter pressure bottle and heated at 150-60°C for one hour to discharge the red color. Solids are removed by filtration, and the filtrate is evaporated to give an oil. Trituration of the oil in methanol affords 8.4 g (74 % yield) of the title compound, a white solid, m.p. 65-72 °C.

| | | | |
|---|---|---|---|
| Anal. Calcd. for C₄₀H₇₄N₄O₄ | C, 71.2; | H, 11.1; | N, 8.3. |
| Found | C, 70.5; | H, 11.0; | N, 8.2. |

### Example 63 Bis(1-cyclooctyloxy-2,2,6,6-tetramethylpiperidin-4-yl)-sebacate

A mixture of 75 g, (156 mmol) of bis(2,2,6,6-tetramethylpiperidin-4-yl)sebacate, 1.3 g of molybdenum trioxide, and 475 ml of cyclooctane is heated to 118°C To this mixture is added 130 g (1.01 mol) of 70 % aq. t-butyl hydroperoxide during a 5 hour period. The reaction mixture is maintained at reflux during the addition, and water is collected in a Dean-Stark trap. The red reaction mixture is heated for 7 hours after the addition to discharge the red color. Solids are removed by filtration, and the filtrate is evaporated to give a yellow oil. Purification by flash chromatography (20:1 heptane:ethyl acetate) affords 104 g (91 %) of the title compound, a light yellow oil.

### Example 64 Bis(1-octyloxy-2,2,6,6-tetramethylpiperidin-4-yl)sebacate

70 % Aqeous t-butyl hydroperoxide (140 9, 1.09 mol) is added over a 6 hours period to a mixture of 75.4 g (0.157 mol) of bis(2,2,6,6-tetramethylpiperidin-4-yl)sebacte, 1.25 g (8.7 mmol) of molybdenum trioxide, and 570 ml of n-octane that has been heated to 115°C under a nitrogen atmosphere. During the addition, the reaction is maintained at reflux. Water is collected in a Dean-Stark trap. Upon completion of the addition, the red recation mixture is heated at reflux (95-97 °C) for seven hours to discharge the red color. The molybdenum trioxide is removed by filtration. The yellow filtrate is stirred ad ambient temperature for 30 minutes with 15 g of activated charcoal (DARCO) to remove some of the yellow color, and then concentrated at reduced pressure. The crude product is purified by flash chromatography on silica gel (100:3 heptane:ethyl acetate) to afford 92.9 g (80 % yield) of the title compound, a colorless oil.

| | | | |
|---|---|---|---|
| Anal. Calcd. for C₄₄H₈₄N₂O₆ | C, 71.7; | H, 11.5; | N, 3.8. |
| Found | C, 71.6; | H, 11.5; | N, 3.6. |

### Example 65 Bis(1-heptyloxy-2,2,6,6-tetramethylpiperidin-4-yl)succinate

70 % Aqeous t-butyl hydroperoxide (78.9 g, 0.613 mol) is added over a 30 minute period to a mixture of 55 g (0.139 mol) of bis(2,2,6,6-tetramethylpiperidin-4-yl)succinate, 1.0 g of molybdenum trioxide, and 400 ml of n-heptane maintained at 110°C. Water is collected in a Dean-Stark trap. After the addition is complete, the recation mixtrue is heated at reflux for 30 minutes. Another portion of 70 % aqeous t-butyl hydroperoxide (100 g, 0.777 mol) is added to the red reaction mixture over 90 minute interval. The reaction is heated at reflux for 16 hours to discharge the red color. The molybdenum trioxide is removed by filtration, and the filtrate is evaporated at reduced pressure. The residue is purified by flash chromatography (19:1, then 9:1 heptane:ethyl acetate) on silica gel to afford 63.3 g (73 % yield) of the title compound, a clear yellow liquid.

| | | | |
|---|---|---|---|
| Anal. Calcd. for C₃₆H₆₈N₂O₆ | C, 69.2; | H, 11.0; | N, 4.5. |
| Found | C, 68.8; | H, 11.1; | N, 4.5. |

### Example 66 Bis(1-decahydronaphthyloxy-2,2,6,6-tetramethylpiperidin-4-yl)-sebacate

A mixture of 25.0 g, (0.052 mol) of bis(2,2,6,6-tetramethylpiperidin-4-yl)sebacate, 55.0 g (0.427 mol) of 70 % aqueous t-butyl hydroperoxide, 1.5 g (0.010 mol) of molybdenum trioxide and 180 ml of decahydronaphthalene is heated at reflux for 4.5 hours until the red color disappears. Water is collected in a Dean-Stark trap. The molybdenum trioxide is removed by filtration and the filtrate stirred with a solution of 26 g of sodium sulfite in 500 ml of water to decompose unreacted t-butyl hydroperoxide. The two-phase mixture is diluted with ethyl acetate (200 ml) and the organic layer washed with saturated sodium chloride, dried over magnesium sulfate, and concentrated at reduced pressure to obtain an oil. Purification by flash chromatography (silica gel, 19:1 heptane:ethyl acetate) affords 28.8 g (71 % yield) of the title compound, a pale yellow oil.

| | | | |
|---|---|---|---|
| Anal. Calcd. for C₄₈H₈₄N₂O₆ | C, 73.4; | H, 10.8; | N, 3.6. |
| Found | C, 74.9; | H, 11.5; | N, 3.4. |

### Example 67 Bis(1-cyclododecyloxy-2,2,6,6-tetramethylpiperidin-4-yl)-sebacate

A mixture of 30.1 g, (62.6 mmol) of bis(2,2,6,6-tetramethylpiperidin-4-yl)sebacate, 44 g (439 mmol) of 90 % t-butyl hydroperoxide, 0.5 g of molybdenum trioxide and 207 g of cyclododecane is heated in a Fischer-Porter pressure bottle at 135-145 °C for 7.5 hours. The reaction mixture is diluted with heptane (350 ml) and purified by flash chromatography on silica gel (heptane, then 20:1 heptane:ethyl acetate) to afford 49.4 g (93 % yield) of the title compound, a colorless, viscous oil.

### Example 68 N,N',N",N"'-Tetrakis-(2,4-bis[N-(1-cyclohexyloxy-2,2,6,6-tetramethylpiperidin-4-yl)-butylamino]-1,3,5-triazin-6-yl)-1,5,8,12-tetrazadodecane

To a refluxing mixture of 30.0 g, (13.0 mmol) of N,N',N",N"'-tetrakis-(2,4-bis[N-(2,2,6,6-tetramethylpiperidin-4-yl)-butylamino]-1,3,5-triazin-6-yl)-1,5,8,12-tetrazadodecane, 1.0 g of molybdenum trioxide, and 300 ml of cyclohexane is added 88.7 g (689 mmol) of 70 % aqueous t-butyl hydroperoxide over a one hour period. The reaction mixture is heated at reflux for another hour after the addition is completed. Water and some organic distillate (100 ml) are collected in a Dean-Stark trap and removed from the reaction mixture. The reaction mixture is then transferred to a Fischer-Porter pressure bottle and heated for five hours at 130-135°C. An additional portion of 70 % t-butyl hydroperoxide (14.0 g, 107 mmol) is added to the mixture, and heating is resumed for three hours. The reaction mixture is cooled and solids are removed by filtration. The filtrate is concentrated, diluted with 19:1 heptane:ethyl acetete, and purified by flash chromatography on silica gel (19:1 heptane:ethyl acetate) to afford 24.0 g (59 %) of the title compound, a pale yellow glass (glass softening point 108-123°C).

| | | | |
|---|---|---|---|
| Anal. Calcd. for C₁₇₂H₃₁₄N₃₂O₈ | C, 69.8; | H, 10.7; | N, 15.1. |
| Found | C, 66.6; | H, 10.6; | N, 14.7. |

### Example 69 2,4,6-Tris[N-(1-cyclohexyloxy-2,2,6,6-tetramethylpiperidin-4-yl)-n-butylamino]-1,3,5-triazine

To a refluxing mixture of 13.5 g, (19.0 mmol) of 2,4,6-tris[N-(2,2,6,6-tetramethylpiperidin-4-yl)-n-butylamino]-1,3,5-triazine, 0.2 g of molybdenum trioxide and 175 ml of cyclohexane are added 36.6 g (284 mmol) of 70 % aqueous t-butyl hydroperoxide over a 10 minute interval. The red reaction mixture is heated at reflux for one hour and then transferred to a Fischer-Porter pressure bottle using 25 ml of fresh cyclohexane. The reaction mixture is then heated for 3 hours at 150°C to discharge the red color. Solids are removed by filtration and the filtrate is concentrated to a residue which is purified by flash chromatography on silica gel (19:1 heptane:ethyl acetate). The product is crystallized from isopropyl alcohol to afford 7.6 g (40 % yield) of a white powder, m.p. 189-94°C.

| | | | |
|---|---|---|---|
| Anal. Calcd. for C₆₀H₁₁₁N₉O₃₉ | C, 71.6; | H, 11.1; | N, 12.5. |
| Found | C, 71.8; | H, 11.1; | N, 12.6. |

### Example 70 2,4-Bis[N-(1-cyclohexyloxy-2,2,6,6-tetramethylpiperidin-4-yl)-n-butylamino]-6-t-octylamino-1,3,5-triazine

A mixture of 22.2 g, (35.3 mmol) of 2,4-bis[N-(2,2,6,6-tetramethylpiperidin-4-yl)-n-butylamino]-6-t-octylamino-1,3,5-triazine, 0.3 g of molybdenum trioxide, 35.3 g (353 mmol) of 90 % aqueous t-butyl hydroperoxide, and 250 ml of cyclohexane is heated for five hours at 150-55 °C in a Fischer-Porter pressure bottle. The pressure is kept below 45 psi by occasional venting. The mixture is then cooled and solids are removed by filtration. The filtrate contains two major products, which are separated by a combination of flash chromatography (39:1 heptane:ethyl acetate) and MPLC (Waters Prep. 500 A, 99:1 heptane:ethyl acetate followed by ethyl acetate). The title compound is the more polar product, a pale yellow glass, m.p. 85-103°C. The yield is 11 g (38 %).

| | | | |
|---|---|---|---|
| Anal. Calcd. for C₄₉H₉₂N₈O₂ | C, 71.3; | H, 11.2; | N, 13.6. |
| Found | C, 71.2; | H, 12.0; | N, 13.6. |

### Example 71 2,4-Bis[N-(1-cyclohexyloxy-2,2,6,6-tetramethylpiperidin-4-yl)-n-butylamino]-6-morpholino-1,3,5-triazine

A mixture of 10.0 g, (13.7 mmol) of 2-chloro-4,6-bis[N-(1-cyclohexyloxy-2,2,6,6-tetramethylpiperidin-4-yl)-n-butylamino]-1,3,5-triazine, 1.43 g (16.4 mmol) of morpholine, 0.8 g of sodium hydroxide, and 30 g of toluene is heated at reflux for four hours. Water is collected in a Dean-Stark trap. The liquid phase is decanted, and residual solids are washed with toluene. The combined organic solutions are dried over magnesium sulfate and concentrated to give a viscous oil. Purification by MPLC (Waters Prep. 500 A, 29:1 heptane:ethyl acetate) affords 5.9 g (55 % yield) of the title compound, a white powder, m.p. 159-63 °C.

| | | | |
|---|---|---|---|
| Anal. Calcd. for C₄₅H₈₂N₈O₃ | C, 69.0; | H, 10.6; | N, 14.3. |
| Found | C, 69.4; | H, 10.6; | N, 14.3. |

### Example 72 24-Bis[N-(1-cyclohexyloxy-2,2,6,6-tetramethylpiperidin-4-yl)-n-butylamino]-6-diethylamino-1,3,5-triazine

A mixture of 50.0 g, (271 mmol) of cyanuric chloride, 22.0 g of 50 % aqueous sodium hydroxide, 22 ml of water, and 150 ml of toluene, cooled to 0°, is admixed with a solution of 19.8 g (271 mmol) of diethylamine in 25 mo of toluene over a 45 minute interval. The reaction temperature is maintained at 0-5°C throughout the addition. After the addition is complete, the reaction mixture is stirred for two hrs. at ambient temperature. Water is added to the mixture to dissolve the sodium chloride, and the phases are separated. The organic phase is dried over magnesium sulfate and concentrated to give an oil, which is crystallized from heptane to give 42.0 g (70 % yield) of 2,4-dichloro-6-diethylamino-1,3,5-triazine, a white crystalline material, m.p. 77-9°C.

4-n-Butylamino-2,2,6,6-tetramethylpiperidine (31.7 g, 149 mmol) is added over 15 min to a suspension of 15.0 g (67.8 mmol) of the 2,4-dichloro-6-diethylamino-1,3,5-triazine, 150 ml of xylene, and 7.0 g of powdered sodium hydroxide that had been heated to 70 °C The reaction mixture is heated at reflux for 23 hrs. Sodium chloride is removed by filtration, and the filtrate is concentrated to a viscous oil. Further concentration of the oil by Kugelrohr distillation (140-50°C 0.05 mm) yields a residue which is crystallized from methanol-water to afford 25.8 g (66 % yield) of 2,4-bis[N-(2,2,6,6-tetramethylpiperidin-4-yl)-n-butylamino]-6-diethylamino-1,3,5-triazine, m.p. 74-76°C.

A mixture of 14.0 g, (24.4 mol) of 2,4-bis[N-(2,2,6,6-tetramethylpiperidin-4-yl)-n-butylamino]-6-diethylamino-1,3,5-triazine, 31.4 g (244 mmol) of 70 % aq. t-butyl hydroperoxide, 0.15 g of molybdenum trioxide, and 140 ml of cyclohexane is heated at reflux for 3 hrs. The red mixture is then transferred to a Fischer-Porter bottle and heated at 145-55°C for 3 hours to discharge the red color. Solids are removed by filtration and the filtrate is concentrated to give a residue which is purified by flash chromatography (39:1 heptane:ethyl acetate) to give 4.9 g (26 %) of the title compound, a white solid, m.p. 91-9 °C.

| | | | |
|---|---|---|---|
| Anal. Calcd. for C₄₅H₈₄N₈O₂ | C, 70.3; | H, 11.0; | N, 14.6. |
| Found | C, 70.2; | H, 10.9; | N, 14.4. |

### Example 73 2,4,6-Tris[N-(1-octyloxy-2,2,6,6-tetramethylpiperidin-4-yl)-n-butylamino]-1,3,5-triazine

2,4,6-Tris[(2,2,6,6-tetramethylpiperidin-4-yl)-n-butylamino]-1,3,5-triazine (50.0 g, 70.2 mmol) is added to a mixture of 108.4 g (842 mmol) of 70 % aqueous t-butylhydroperoxide, 1.0 g of molybednum trioxide, and 350 ml of n-octane that has been heated to 50 ° C. The reaction mixture is carefully brought to reflux. A Dean-Stark trap is used to remove water from the reaction. The reaction mixture is heated at reflux for 16 hours. Solids are removed by filtration, and the filtrate is concentrated to give a viscous oily residue. Purification by flash chromatography (39:1 heptane:ethyl acetate) affords 15.0 g (19 % yield) of the title compound.

| | | | |
|---|---|---|---|
| Anal. Calcd. for C₆₆H₁₂₉N₉O₃ | C, 72.3; | H, 11.9; | N, 11.5. |
| Found | C, 72.2; | H, 12.1; | N, 11.4. |

### Example 74 N,N',N",N'"-Tetrakis-(2,4-bis[N-(1-octyloxy-2,2,6,6-tetramethylpiperidin-4-yl)-n-butylamino]-1,3,5-triazin-6-yl)-1,5,8,12-tetrazadodecane

94.6 g (736 mmol) of 70 % aqueous t-butyl hydroperoxide is extracted with 300 ml of n-octane. A mixture of 100 ml of the t-butyl hydroperoxide/octane solution, 50.0 g (23.0 mmol) of N,N',N",N"'-tetrakis-(2,4-bis[N-(2,2,6,6-tetramethylpiperidin-4-yl)-butylamino]-1,3,5-triazin-6-yl)-1,5,8,12-tetrazadodecane, 0.5 g of molybdenum trioxide, and 100 ml of n-octane is heated to reflux. Water is collected in a Dean-Stark trap. The remaining t-butyl hydroperoxide-octane solution is then added over 2.5 hrs to the red reaction mixture. The reaction is heated at reflux for four hrs. after the addition, then treated with 60 g (470 mmol) of 70 % t-butyl hydroperoxide and heated at reflux for another four hours to discharge the red color. The reaction mixture is cooled and solids are removed by filtrtion. The filtrate is stirred with 300 ml of 5 % aq. sodium sulfite, dried over magnesium sulfate, and concentrated. The residue is purified by flash chromatography (19:1 heptane:ethyl acetate) on silica gel to afford 20.2 g (27 % yield) of the title compound, a yellow glass, m.p. 81-91 °C.

| | | | |
|---|---|---|---|
| Anal. Calcd. for C₁₀₈H₃₆₂N₃₂O₈ | C, 70.6; | H, 11.4; | N, 14.0. |
| Found | C, 68.7; | H, 11.9; | N, 14.0. |

### Example 75 N,N'-Bis(1-cyclohexyloxy-2,2,6,6-tetramethylpiperidin-4-yl)-N,N'-bis{2,4-bis[N-(1-cyclohexyloxy-2,2,6,6-tetramethylpiperidin-4-yl)-n-butylamino]-1,3,5-triazin-6-yl}hexamethylene diamine

A solution of 19.0 g, (32.1 mmol) of 1,6-bis[N,N'-1-cyclohexyloxy-2,2,6,6-tetramethylpiperidin-4-yl]-aminohexane in 50 ml of toluene is added over 20 minutes to a mixture of 11.9 g (64.3 mmol) of cyanuric chloride, 100 ml of toluene, 6.8 g of sodium carbonate, and 30 ml of water that has been cooled to 0°. The reaction temperature is maintained at 2-5°C during the addition. The reaction is then stirred at ambient temperature for two hours. The precipitate is filtered and washed successively with water and toluene, then dissolved in dichloromethane.The toluene solution is dried over magnesium sulfate and evaporated to obtain a residue which is added to the dichloromethane solution. Evaporation of this solution affords 12.1 g (42 % yield) of N,N'-bis(1-cyclohexyloxy-2,2,6,6-tetramethylpiperidin-4-yl)-N,N'-bis(2,4-dichloro-1,3,5-triazin-6-yl)-hexamethylene diamine.

A mixture of 7.0 g (7.9 mmol) of N,N'-bis(1-cyclohexyloxy-2,2,6,6-tetramethylpiperidin-4-yl)-N,N'-bis(2,4-dichloro-1,3,5-triazin-6-yl)-hexamethylene diamine, 13.0 g (41.9 mmol) of 1-cyclohexyloxy-4-n-butylamino-2,2,6,6-tetramethylpiperidine, 2.5 g of sodium hydroxide, and 100 ml of xylene is heated at reflux under nitrogene for 30 hrs. Solids are removed by filtration. The filtrate is concentrated to a residue which is partially dissolved in boiling dichioromethane. The hot solution is filtered to remove an insoluble impurity then partially evaporated and diluted with ethanol to obtain 12.2 g (78 %) of the title compound, a white powder, m.p. 254-7°C (dec).

| | | | |
|---|---|---|---|
| Anal. Calcd. for C₁₈H₂₁₆N₁₈O₆ | C, 71.5; | H, 11.0; | N, 12.7. |
| Found | C, 70.4; | H, 10.7; | N, 12.4. |

### Example 76 2,4-Bis[N-(1-methoxy-2,2,6,6-tetramethylpiperidin-4-yl)-n-butylamino]-6-diethylamino-1,3,5-triazine

A mixture of 5.0 g (22.6 mmol) of 2,4-dichloro-6-diethylamino-1,3,5-triazine, 14.0 g (57.8 mmol) of 1-methoxy-4-n-butylamino-2,2,6,6-tetramethylpiperidine, 50 ml of xylene, and 1.8 g of sodium hydroxide is heated at reflux for 18 hrs. Salts are removed by filtration and the filtrate is concentrated to an oil. Purification by column chromatography (heptane) affords 9.7 g (68 % yield) of the title compound, a white solid, m.p. 109-112°C

| | | | |
|---|---|---|---|
| Anal. Calcd. for C₃₅H₆₈N₈O₂ | C, 66.4; | H, 10.8; | N, 17.7. |
| Found | C, 66.2; | H, 10.9; | N, 17.4. |

### Example 77 24-Bis[N-(1-cyclohexyloxy-2,2,6,6-tetramethylpiperidin-4-yl)-n-butylamino]-6-n-butylamino-1,3,5-triazine

A mixture of 5.0 g (22.5 mmol) of 2,4-dichloro-6-n-butylamino-1,3,5-triazine, 21.1 g (67.8 mmol) of 1-cyclohexyloxy-4-n-butylamino-2,2,6,6-tetramethylpiperidine, 100 ml of xylene, and 5.4 g of 50 % aq. sodium hydroxide is heated at reflux for 16 hrs. The reaction mixture is partitioned between ether and water. The ether layer is washed with 1 N HCI (2x100 ml) saturated sodium bicarbonate (100 ml) and saturated sodium chloride (100 ml), then dried over magnesium sulfate and concentrated. The crude product is purified by flash chromatography (heptane) and crystallized form ethanol to afford 7.6 g (44 % yield) of the title compound, a glass, m.p. 80-86°C.

| | | | |
|---|---|---|---|
| Anal. Calcd. for C₄₅H₈₄N₈O₂ | C, 70.3; | H, 11.0; | N, 14.6. |
| Found | C, 70.2; | H, 11.1; | N, 14.6. |

Summarizing, this invention is seen to provide a series of new OR₁-substituted N-hydroxy hindered amine stabilizers. Variations may be made in proportions, procedures and materials without departing form the scope of the invention as defined by the following claims.

## Patentansprüche (Patentansprüche für folgende(n) Vertragsstaat(en): BE, DE, FR, GB, IT, NL, SE)

1. Stabilisierte, bei Raumtemperatur härtende oder säurekatalysierte, wärmehärtende Überzugszusammensetzung, enthaltend eine wirksame, stabilisierende Menge einer gehinderten Aminverbindung, die die Gruppe 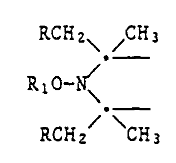 aufweist, worin R für Wasserstoff oder Methyl steht und R₁ C₁₋₁₈-Alkyl, C₂₋₁₈-Alkenyl, C₂₋₁₈-Alkinyl, C₅₋₁₂-Cycloalkyl, C₆₋₁₀-Bicycloalkyl, C₅₋₈-Cycloalkenyl, C₆₋₁₀-Aryl, C₇₋₉-Aralkyl oder durch C₁₋₄-Alkyl oder Phenyl substituiertes C₇₋₉-Aralkyl bedeutet.

2. Zusammensetzung gemäß Anspruch 1, die eine gehinderte Aminverbindung entsprechend einer der Formeln A-N enthält: 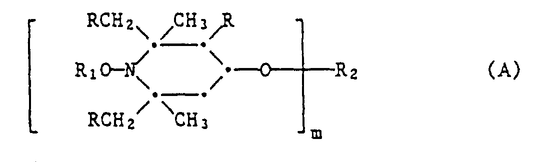 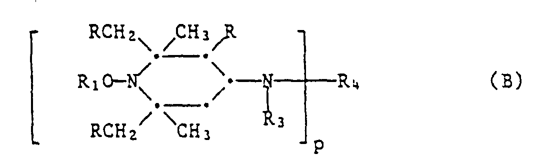 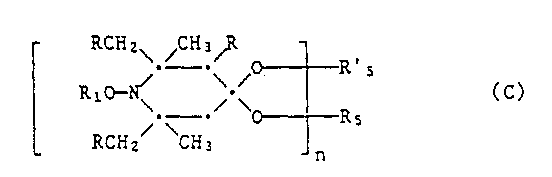 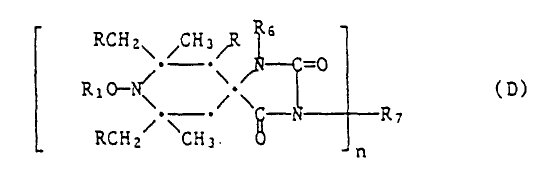 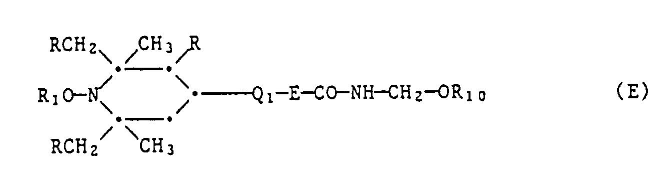 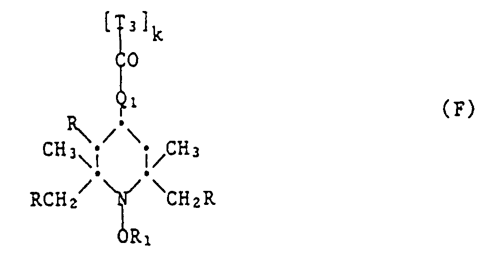 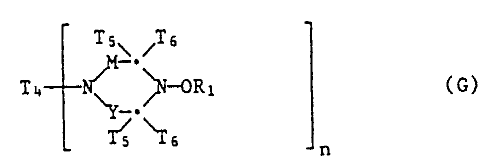 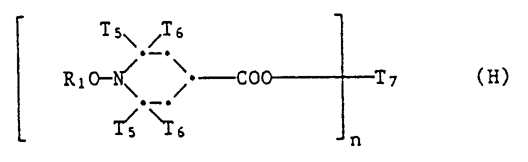 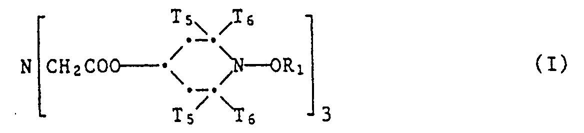 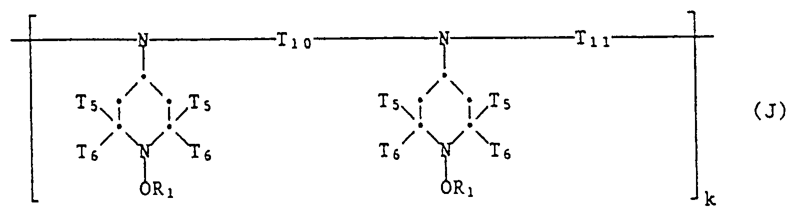 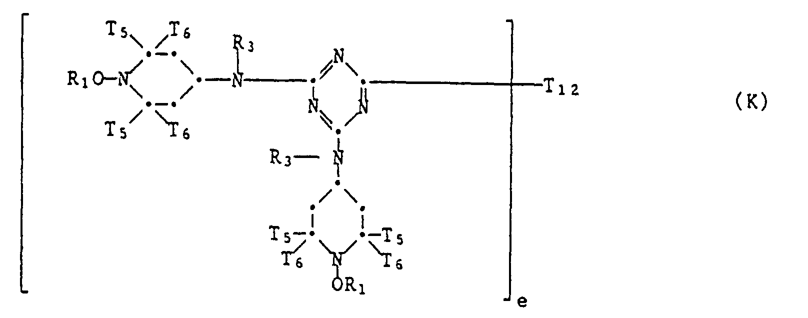 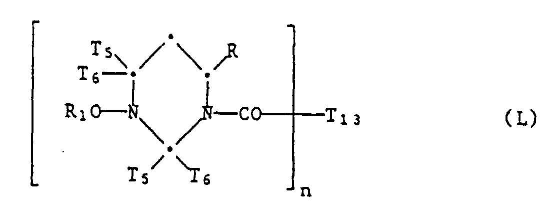 oder 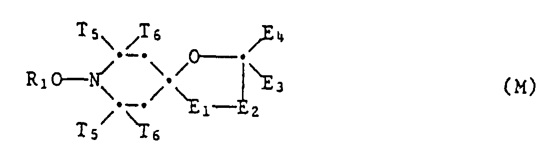 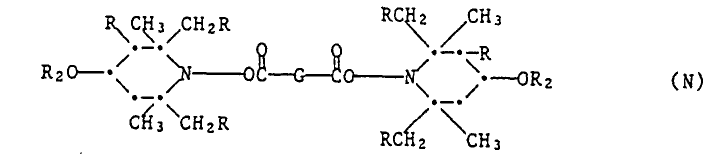 worin
R für Wasserstoff oder Methyl steht,
R₁ C₁₋₁₈-Alkyl, C₂₋₁₈-Alkenyl, C₂₋₁₈-Alkinyl, C₅₋₁₂-Cycloalkyl, C₆₋₁₀-Bicycloalkyl, C₅₋₈-Cycloalkenyl, C₆₋₁₀-Aryl, C₇₋₉-Aralkyl oder durch C₁₋₄-Alkyl oder Phenyl substituiertes C₇₋₉-Aralkyl bedeutet;
m für 1 bis 4 steht,
wenn m 1 ist,
R₂ Wasserstoff, C₁₋₁₈-Alkyl, das gegebenenfalls durch ein oder mehrere Sauerstoffatome unterbrochen ist, C₂₋₁₂-Alkenyl, C₆₋₁₀-Aryl, C₇₋₁₈-Aralkyl, Glycidyl, einen einwertigen Acylrest einer aliphatischen, cycloaliphatischen, araliphatischen oder aromatischen Carbonsäure oder einer Carbamidsäure bedeutet oder R₂ eine Gruppe 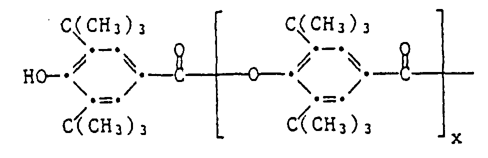 ist, worin x für 0 oder 1 steht, oder eine Gruppe 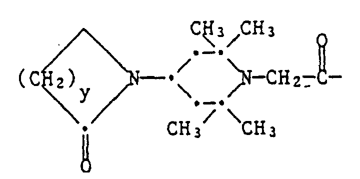 ist, worin y für 2-4 steht;
wenn m für 2 steht,
R₂ C₁₋₁₂-Alkylen, C₄₋₁₂-Alkenylen, Xylylen, einen zweiwertigen Acylrest einer aliphatischen, cycloaliphatischen, araliphatischen oder aromatischen Dicarbonsäure oder einer Dicarbamidsäure bedeutet oder eine Gruppe 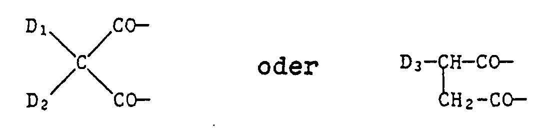 ist, worin D₁ und D₂ unabhängig Wasserstoff, Alkyl mit bis zu8 Kohlenstoffatomen, Phenyl, Benzyl oder 3,5-Di-tert.-butyl-4-hydroxybenzyl bedeuten und D₃ einen Alkyl- oder Alkenylrest mit bis zu 18 Kohlenstoffatomen darstellt;
wenn m für 3 steht, R₂ einen dreiwertigen Acylrest einer aliphatischen, cycloaliphatischen oder aromatischen Tricarbonsäure bedeutet;
wenn m für 4 steht, R₂ einen vierwertigen Acylrest einer aliphatischen oder aromatischen Tetracarbonsäure bedeutet; p für 1, 2 oder 3 steht,
R₃ Wasserstoff, C₁₋₁₂-Alkyl, C₅₋₇-Cycloalkyl, C₇₋₉-Aralkyl, C₂₋₁₈-Alkanoyl, C₃₋₅-Alkenoyl oder Benzoyl bedeutet; wenn p für 1 steht,
R₄ Wasserstoff, C₁₋₁₈-Alkyl, C₅₋₇-Cycloalkyl, C₂₋₈-Alkenyl, welches unsubstituiert oder durch eine Cyano-, Carbonyl- oder Carbamidgruppe substituiert ist, bedeutet oder Aryl, Aralkyl, Glycidyl, eine Gruppe der Formel -CH₂-CH(OH)-Z oder -CONH-Z ist, worin Z für Wasserstoff, Methyl oder Phenyl steht; oder R₄ eine Gruppe der Formel I 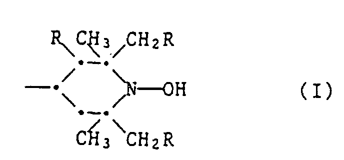 oder eine Gruppe der Formel 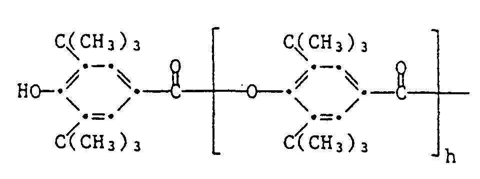 wobei h = 0 oder 1,
ist; oder R₃ und R₄ gemeinsam Alkylen mit 4 bis 6 Kohlenstoffatomen oder 1-Oxo-alkylen oder den zweiwertigen Acylrest einer aliphatischen oder aromatischen 1,2- oder 1,3-Dicarbonsäure darstellen;
wenn p für 2 steht,
R₄ C₂₋₁₂-Alkylen, C₆₋₁₂-Arylen, Xylylen, eine Gruppe -CH₂CH(OH)-CH₂- oder eine Gruppe -CH₂-CH(OH)-CH₂-O-X-O-CH₂-CH(OH)-CH₂- darstellt, worin X C₂₋₁₀-Alkylen, C₆₋₁₅-Arylen oder C₆₋₁₂-Cycloalkylen bedeutet; oder, mit der Maßgabe, daß R₃ nicht Alkanoyl, Alkenoyl oder Benzoyl bedeutet, R₄ auch ein zweiwertiger Acylrest einer aliphatischen, cycloaliphatischen oder aromatischen Dicarbonsäure oder Dicarbamidsäure sein kann oder die Gruppe -CO- sein kann; oder R₄ eine Gruppe der Formel II 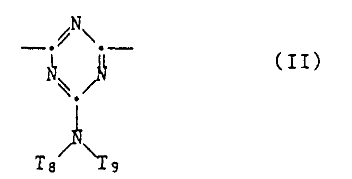 ist, worin T₈ und T₉ unabhängig Wasserstoff, Alkyl mit 1 bis 18 Kohlenstoffatomen oder eine Gruppe der Formel I bedeuten oder T₈ und T₉ gemeinsam Alkylen mit 4 bis 6 Kohlenstoffatomen oder 3-Oxapentamethylen bedeuten;
wenn p für 3 steht,
R₄ 2,4,6-Triazintriyl ist,
n für 1 oder 2 steht,und
wenn n für 1 steht,
R₅ und R'₅ unabhängig C₁₋₁₂-Alkyl, C₂₋₁₂-Alkenyl, C₇₋₁₂-Aralkyl bedeuten oder R₅ auch Wasserstoff ist oder R₅ und R'₅ gemeinsam C₂₋₈-Alkylen oder Hydroxyalkylen oder C₄₋₂₂-Acyloxyalkylen bedeuten;
wenn n für 2 steht,
R₅ und R'₅ gemeinsam für (-CH₂)₂C(CH₂-)₂ stehen;
R₆ Wasserstoff, C₁₋₁₂-Alkyl, Allyl, Benzyl, Glycidyl oder C₂₋₆-Alkoxyalkyl bedeutet;
wenn n für 1 steht,
R₇ Wasserstoff, C₁₋₁₂-Alkyl, C₃₋₅-Alkenyl, C₇₋₉-Aralkyl, C₅₋₇-Cycloalkyl, C₂₋₄-Hydroxyalkyl, C₂₋₆-Alkoxyalkyl, C₆₋₁₀-Aryl, Glycidyl, eine Gruppe der Formel -(CH₂)ₜ-COO-Q oder der Formel -(CH₂)ₜ-O-CO-Q darstellt, worin t für 1 oder 2 steht und Q C₁₋₄-Alkyl oder Phenyl bedeutet;
oder wenn n für 2 steht,
R₇ C₂₋₁₂-Alkylen, C₆₋₁₂-Arylen, eine Gruppe -CH₂CH(OH)-CH₂-O-X-O-CH₂-CH(OH)-CH₂- worin X C₂₋₁₀-Alkylen, C₆₋₁₅-Arylen oder C₆₋₁₂-Cycloalkylen bedeutet, oder eine Gruppe -CH₂CH(OZ')CH₂-(OCH₂-CH(OZ')CH₂)₂-, worin Z' Wasserstoff, C₁₋₁₈-Alkyl, Allyl, Benzyl, C₂₋₁₂-Alkanoyl oder Benzoyl bedeutet, darstellt;
Q₁ für -N(R₈)- oder -O- steht;
E C₁₋₃-Alkylen, die Gruppe -CH₂-CH(R₉)-O- bedeutet, worin R₉ Wasserstoff, Methyl oder Phenyl ist, oder E die Gruppe -(CH₂)₃-NH- oder eine direkte Bindung darstellt;
R₁₀ für Wasserstoff oder C₁₋₁₈-Alkyl steht;
R₈ Wasserstoff, C₁₋₁₈-Alkyl, C₅₋₇-Cycloalkyl, C₇₋₁₂-Aralkyl, Cyanoethyl, C₆₋₁₀-Aryl, die Gruppe -CH₂-CH(R₉)-OH, in der R₉ die vorstehend angegebene Bedeutung besitzt, eine Gruppe der Formel I oder eine Gruppe der Formel 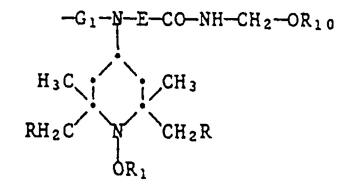 darstellt,
worin G₁ C₂₋₆-Alkylen oder C₆₋₁₂-Arylen sein kann, oder R₈ eine Gruppe -E-CO-NH-CH₂-OR₁₀ ist;
T₃ Ethylen oder 1,2-Propylen bedeutet oder die sich wiederholende Struktureinheit, abgeleitet von einem α-Olefincopolymeren mit einem Alkylacrylat oder -methacrylat, darstellt;
k für 2 bis 100 steht;
T₄ die für R₄ angegebene Bedeutung besitzt, wenn p für 1 oder 2 steht,
T₅ Methyl bedeutet,
T₆ Methyl oder Ethyl bedeutet oder T₅ und T₆ gemeinsam Tetramethylen oder Pentamethylen bedeuten;
M und Y unabhängig Methylen oder Carbonyl sind;
T₇ die gleiche Bedeutung wie R₇ besitzt;
T₁₀ und T₁₁ unabhängig Alkylen mit 2 bis 12 Kohlenstoffatomen darstellen oder T₁₁ eine Gruppe der Formel II ist;
e für 2, 3 oder 4 steht und
T₁₂ eine Gruppe der Formel -N(R₅)-(CH₂)_{d}-N(R₅)- oder 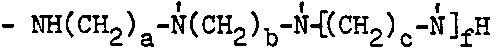 darstellt, worin a, b und c unabhängig für 2 oder 3 stehen, d für 2 bis 10 steht und f für 0 oder 1 steht,
T₁₃ die Bedeutung von R₄ besitzt mit der Maßgabe, daß T₁₃ nicht Wasserstoff sein kann, wenn n für 1 steht;
E₁ und E₂, die verschieden sind, jeweils -CO- oder -N(E₅)-bedeuten, worin E₅ Wasserstoff, C₁₋₁₂-Alkyl oder C₄₋₂₂-Alkoxycarbonylalkyl ist;
E₃ Wasserstoff, Alkyl mit 1 bis 30 Kohlenstoffatomen, Phenyl, Naphthyl, dieses Phenyl oder dieses Naphthyl,substituiert durch Chlor oder durch Alkyl mit 1 bis 4 Kohlenstoffatomen, oder Phenylalkyl mit 7 bis 12 Kohlenstoffatomen oder dieses Phenylalkyl,substituiert durch Alkyl mit 1 bis 4 Kohlenstoffatomen, bedeutet;
E₄ Wasserstoff, Alkyl mit 1 bis 30 Kohlenstoffatomen, Phenyl, Naphthyl oder Phenylalkyl mit 7 bis 12 Kohlenstoffatomen bedeutet, oder
E₃ und E₄ gemeinsam Polymethylen mit 4 bis 17 Kohlenstoffatomen oder dieses Polymethylen, substituiert durch bis zu vier Alkylgruppen mit 1 bis 4 Kohlenstoffatomen, bedeuten; R₂ der Formel (N) wie vorstehend definiert ist, wenn m für 1 steht; und
G eine direkte Bindung, C₁₋₁₂-Alkylen, Phenylen oder -NH-G'-NH, worin G' C₁₋₁₂-Alkylen bedeutet, darstellt.

3. Zusammensetzung gemäß Anspruch 2, die eine Verbindung der Formel A, B, C, D, J, K oder M enthält, worin R für Wasserstoff steht und T₅ und T₆ Methyl sind.

4. Zusammensetzung gemäß Anspruch 2, die eine Verbindung der Formel A, B, C, J oder K enthält, worin R für Wasserstoff steht und R₁ C₁₋₁₈-Alkyl, C₆₋₁₂-Cycloalkyl, Cyclohexenyl oder C₇₋₉-Phenylalkyl bedeutet.

5. Zusammensetzung gemäß Anspruch 2, die eine Verbindung der Formel A enthält,
worin R Wasserstoff bedeutet und R₁ C₁₋₁₈-Alkyl, C₆₋₁₂-Cycloalkyl, Cyclohexenyl oder C₇₋₉-Phenylalkyl bedeutet, m für 1, 2 oder 4 steht und wenn m für 1 steht, R₂ C₁₋₁₂-Alkyl, Allyl, Benzyl oder einen Acylrest einer aliphatischen C₂₋₁₈-Carbonsäure, einer cycloaliphatischen C₆₋₁₂-Carbonsäure oder einer aromatischen C₇₋₁₅-Carbonsäure bedeutet, und wenn m für 2 steht, R₂ C₁₋₈-Alkylen, Butylen, Xylylen ist oder einen zweiwertigen Acylrest einer aliphatischen C₂₋₁₈-Dicarbonsäure, einer cycloaliphatischen oder aromatischen C₈₋₁₄-Dicarbonsäure oder einer aliphatischen, cycloaliphatischen oder aromatischen C₈₋₁₄-Dicarbamidsäure bedeutet oder R₂ eine Gruppe 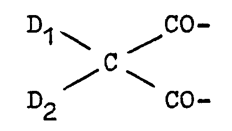 darstellt, worin D₁ C₁₋₈-Alkyl oder 3,5-'Di-tert.-butyl-4-hydroxybenzyl bedeutet und D₂ die Bedeutung von D₁ besitzt oder Wasserstoff darstellt und wenn m für 4 steht, R₂ einen vierwertigen Acylrest einer Butan- oder Pentantetracarbonsäure darstellt.

6. Zusammensetzung gemäß Anspruch 5, die eine Verbindung der Formel A enthält,
worin R Wasserstoff bedeutet, R₁ C₁₋₁₀-Alkyl, Cycloalkyl, Cyclohexyl oder C₇₋₉-Phenylalkyl ist, m für 1 oder 2 steht und wenn m für 1 steht, R₂ Benzyl, C₂₋₁₈-Alkanoyl, Benzoyl oder 3,5-Di-tert.-butyl-4-hydroxybenzoyl bedeutet, und wenn m für 2 steht, R₂ Xylylen oder einen zweiwertigen Acylrest einer aliphatischen C₄₋₁₀-Dicarbonsäure oder einer Benzoldicarbonsäure bedeutet.

7. Zusammensetzung gemäß Anspruch 2, die eine Verbindung der Formel B enthält,
worin R Wasserstoff bedeutet und R₁ C₁₋₁₈-Alkyl, C₆₋₁₂-Cycloalkyl, Cyclohexenyl oder C₇₋₉-Phenylalkyl bedeutet, p für 1 oder 2 steht, R₃ Wasserstoff, C₁₋₁₂-Alkyl oder C₂₋₁₂-Alkanoyl, Allyl bedeutet und wenn p für 1 steht, R₄ Wasserstoff, C₁₋₁₂-Alkyl oder eine Gruppe der Formel I darstellt, und wenn p für 2 steht, R₄ C₂₋₈-Alkylen oder Xylylen bedeutet, und wenn R₃ nicht Alkanoyl ist, R₄ auch ein zweiwertiger Acylrest einer aliphatischen C₄₋₁₀-Dicarbonsäure oder einer Benzoldicarbonsäure sein kann, oder eine Gruppe der Formel II darstellt, worin T₈ Wasserstoff oder C₁₋₄-Alkyl bedeutet und T₉ C₁₋₁₂-Alkyl oder eine Gruppe der Formel I darstellt.

8. Zusammensetzung gemäß Anspruch 1, worin die gehinderte Aminverbindung in einer Menge von 0,1 bis 10 Gew.%, basierend auf Harzfeststoffen, enthalten ist.

9. Zusammensetzung gemäß Anspruch 1, die zusätzlich einen UV-Absorber, ausgewählt unter Benzophenonen, Benzotriazolen, Acrylsäurederivaten, Aryl-s-triazinen, organischen Nickelverbindungen und Oxaniliden, enthält.

10. Zusammensetzung gemäß Anspruch 9, die einen Benzotriazol-UV-Absorber enthält.

11. Zusammensetzung gemäß Anspruch 9, die einen Benzotriazol-UV-Absorber, ausgewählt unter 2-[2-Hydroxy-3,5-di-(α,α-dimethylbenzyl)-phenyl]-benzotriazol, 2-(2-Hydroxy-3,5-di-tert.-octylphenyl)-benzotriazol, 2-(2-Hydroxy-3-α,α-dimethylbenzyl-5-tert.-octylphenyl)-benzotriazol, 2-(2-Hydroxy-3-tert.-octyl-5-α,α-dimethylbenzylphenyl)-benzotriazol, 2-(2-Hydroxy-3,5-di-tert.-amylphenyl)-benzotriazol, 2-[2-Hydroxy-3-tert.-butyl-5-(2-(omega-hydroxy-octa-(ethylenoxy)-carbonyl)-ethylphenyl]-benzotriazol, 5-Chlor-2-[2-hydroxy-3,5-di-(α,α-dimethylbenzyl)-phenyl]-benzotriazol, 5-Chlor-2-(2-hydroxy-3,5-di-tert.-butylphenyl)-benzotriazol, 2-[2-Hydroxy-3-tert.-butyl-5-(2-octyloxycarbonylethyl)-phenyl]-5-chlor-benzotriazol, 2-(2-Hydroxy-3-sek.-dodecyl-5-methylphenyl)-benzotriazol und Hexamethylen-di-[β-(3-tert.-butyl-4-hydroxy-5-[2-benzotriazolyl]-phenyl)-propionat], enthält.

12. Zusammensetzung gemäß Anspruch 9, worin das Benzotriazol 2-[2-Hydroxy-3,5-di-(α,α-dimethylbenzyl)-phenyl]-benzotriazol und 2-[2-Hydroxy-3-tert.-butyl-5-(2-(omega-hydroxy-octa-(ethylenoxy)-carbonyl)-ethylphenyl]-benzotriazol ist.

13. Zusammensetzung gemäß Anspruch 9, die zusätzlich ein Phosphit- oder Phosphonit-Antioxidans enthält.

14. Zusammensetzung gemäß Anspruch 1, die zusätzlich ein gehindertes Phenol-Antioxidans enthält.

15. Überzugszusammensetzung gemäß Anspruch 1, die ein bei Raumtemperatur härtbares System, basierend auf einem Alkydharz, thermoplastischen Acrylharz, Acrylalkydharz, Polyurethanharz oder Polyesterharz oder diesen Harzen, modifiziert mit Siliconen, Isocyanaten, Epoxiden, Isocyanuraten, Ketiminen oder Oxazolidinen, ist oder das System auf einem Celluloseester oder einem Epoxidharz basiert.

16. Überzugszusammensetzung gemäß Anspruch 1, die ein säurekatalysiertes, wärmehärtendes System, basierend auf einem heiß vernetzbaren Acryl-, Polyester-, Polyurethan-, Polyamid- oder Alkydharz, ist.

17. Zusammensetzung gemäß Anspruch 1, die ein Email für industrielle Anstriche ist.

18. Zusammensetzung gemäß Anspruch 1, die ein Refinishemail für Kraftfahrzeuge ist.

19. Verbindung gemäß einer der Formeln A' bis M' 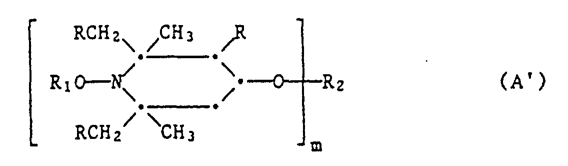 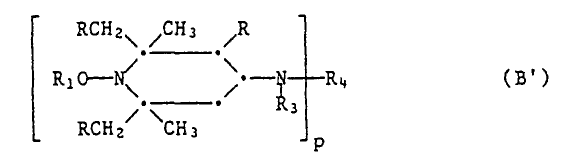 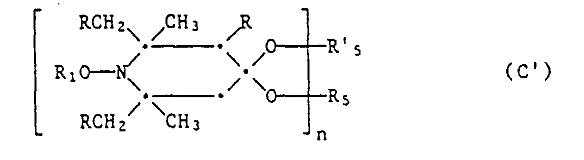 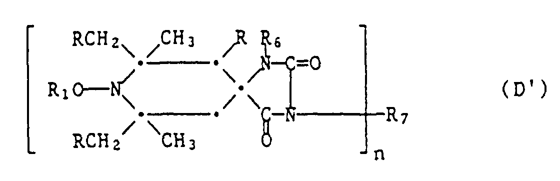 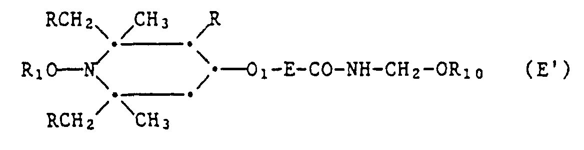 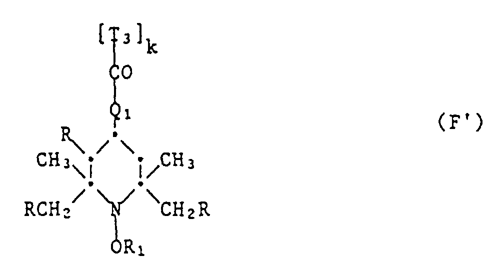 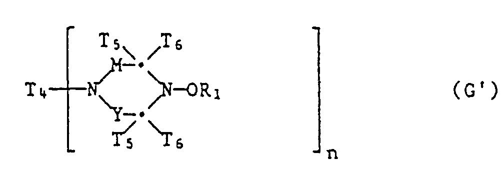 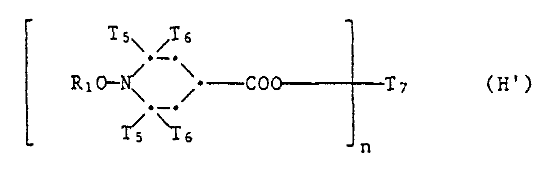 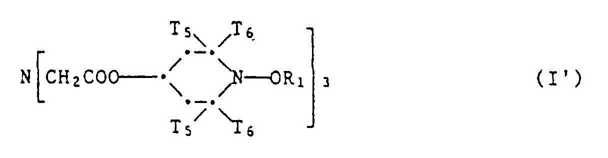 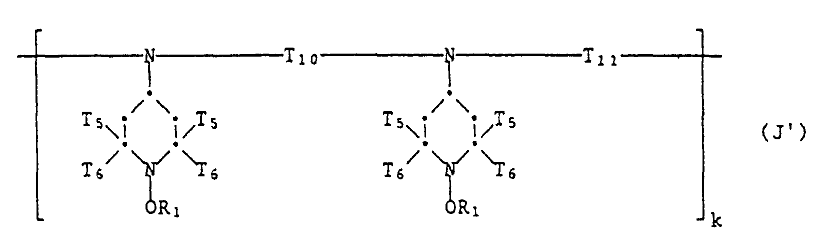 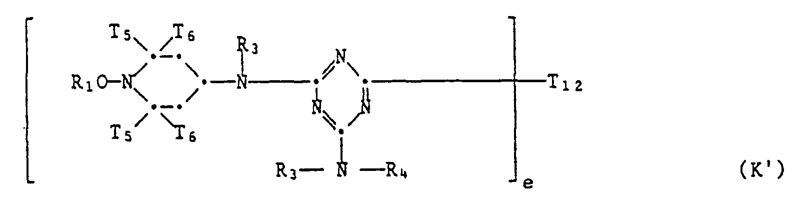 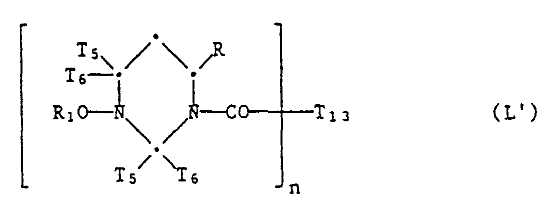 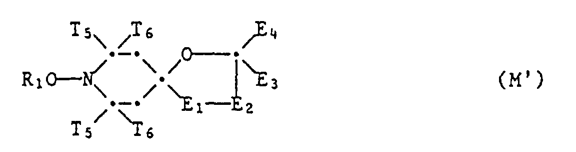 worin
R Wasserstoff oder Methyl bedeutet,
R₁ C₁₋₁₈-Alkyl, C₂₋₁₈-Alkenyl, C₂₋₁₈-Alkinyl, C₅₋₈-Cycloalkenyl, C₅₋₁₂-Cycloalkyl, C₆₋₁₀-Bicycloalkyl, C₆₋₁₀-Aryl, C₇₋₉-Aralkyl oder C₇₋₉-Aralkyl, substituiert durch Alkyl oder Aryl, bedeutet;
m für 2 bis 4 steht,
wenn m für 2 steht,
R₂ C₁₋₁₂-Alkylen, C₄₋₁₂-Alkenylen, Xylylen, einen zweiwertigen Acylrest einer aliphatischen, cycloaliphatischen, araliphatischen oder aromatischen Dicarbonsäure oder Dicarbamidsäure mit bis zu 20 Kohlenstoffatomen bedeutet oder eine Gruppe der Formel 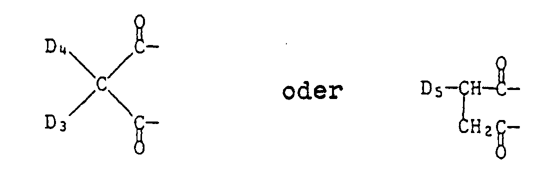 ist, wobei D₃ und D₄ unabhängig Wasserstoff, C₁₋₆-Alkyl, Phenyl, Benzyl oder 3,5-Di-tert.-butyl-4-hydroxybenzyl bedeuten und D₅ Alkyl oder Alkenyl mit bis zu 18 Kohlenstoffatomen ist;
wenn m für 3 steht, R₂ ein dreiwertiger Acylrest einer aliphatischen, cycloaliphatischen oder aromatischen Tricarbonsäure mit bis zu 12 Kohlenstoffatomen ist;
wenn m für 4 steht, R₂ ein vierwertiger Acylrest einer aliphatischen oder aromatischen Tetracarbonsäure mit bis zu
18 Kohlenstoffatomen ist;
p für 1, 2 oder 3 steht,
R₃ Wasserstoff, C₁₋₁₂-Alkyl, C₅₋₈-Cycloalkyl, C₇₋₉-Aralkyl, C₂₋₁₈-Alkanoyl, C₃₋₅-Alkenoyl oder Benzoyl bedeutet; wenn p für 1 steht,
R₄ Wasserstoff, C₁₋₁₈-Alkyl, C₅₋₈-Cycloalkyl, C₂₋₈-Alkenyl, das unsubstituiert oder durch eine Cyano-, Carbonyl- oder Carbamidgruppe substituiert ist, bedeutet oder R₄ C₆₋₁₀-Aryl, C₇₋₉-Aralkyl, Glycidyl, eine Gruppe der Formel -CH₂-CH(OH)-Z oder -CONH-Z bedeutet, worin Z für Wasserstoff, Methyl oder Phenyl steht; oder R₄ eine Gruppe der Formel 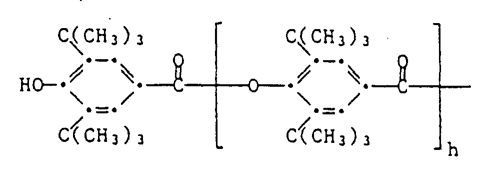 wobei h = 0 oder 1
oder eine Gruppe der Formel I 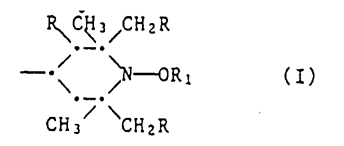 ist oder R₃ und R₄ gemeinsam Alkylen mit 4 bis 6 Kohlenstoffatomen oder 1-Oxoalkylen oder den zweiwertigen Acylrest einer aliphatischen oder aromatischen 1,2- oder 1,3-Dicarbonsäure bedeuten;
wenn p für 2 steht,
R₄ C₁₋₁₂-Alkylen, C₆₋₁₂-Arylen, Xylylen, eine Gruppe -CH₂CH(OH)-CH₂ oder eine Gruppe -CH₂-CH(OH)-CH₂-O-X-O-CH₂-CH(OH)-CH₂- bedeutet, worin X für C₂₋₁₀-Alkylen,
C₆₋₁₅-Arylen oder C₆₋₁₂-Cycloalkylen steht; oder mit der Maßgabe, daß R₃ nicht Alkanoyl, Alkenoyl oder Benzoyl ist, R₄ auch ein zweiwertiger Acylrest einer aliphatischen, cycloaliphatischen oder aromatischen Dicarbonsäure oder einer Dicarbamidsäure sein kann oder die Gruppe -CO- sein kann;
oder R₄ eine Gruppe der Formel II 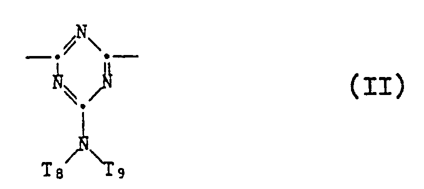 ist, worin T₈ und T₉ unabhängig Wasserstoff, Alkyl mit 1 bis 18 Kohlenstoffatomen bedeuten oder T₈ und T₉ gemeinsam Alkylen mit 4 bis 6 Kohlenstoffatomen oder 3-Oxapentamethylen sind,
wenn p für 3 steht,
R₄ 2,4,6-Triazintriyl bedeutet;
n für 1 oder 2 steht und
wenn n für 1 steht,
R₅ und R'₅ unabhängig C₁₋₁₂-Alkyl, C₂₋₁₂-Alkenyl, C₇₋₁₂-Aralkyl bedeuten oder R₅ auch Wasserstoff bedeutet oder R₅ und R'₅ gemeinsam C₂₋₈-Alkylen oder Hydroxyalkylen oder C₄₋₂₂-Acyloxyalkylen bedeuten;
und wenn n für 2 steht,
R₅ und R'₅ gemeinsam für (-CH₂)₂C(CH₂-)₂ stehen;
R₆ Wasserstoff, C₁₋₁₂-Alkyl, Allyl, Benzyl, Glycidyl oder C₂₋₆-Alkoxyalkyl bedeutet;
wenn n für 1 steht,
R₇ Wasserstoff, C₁₋₁₂-Alkyl, C₃₋₅-Alkenyl, C₇₋₉-Aralkyl, C₅₋₇-Cycloalkyl, C₂₋₄-Hydroxyalkyl, C₂₋₆-Alkoxyalkyl, C₆₋₁₀-Aryl, Glycidyl, eine Gruppe der Formel -(CH₂)ₜ-COO-Q oder der Formel -(CH₂)ₜ-O-CO-Q darstellt, worin t für 1 oder 2 steht und Q für C₁₋₄-Alkyl oder Phenyl steht;
und wenn n für 2 steht,
R₇ C₂₋₁₂-Alkylen, C₆₋₁₂-Arylen, eine Gruppe -CH₂CH(OH)-CH₂-O-X-O-CH₂-CH(OH)-CH₂-, worin X C₂₋₁₀-Alkylen, C₆₋₁₅-Arylen oder C₆₋₁₂-Cycloalkylen bedeutet oder eine Gruppe -CH₂CH(OZ')CH₂-(OCH₂-CH(OZ')CH₂)₂-, worin Z' Wasserstoff, C₁₋₁₈-Alkyl, Allyl, Benzyl, C₂₋₁₂-Alkanoyl oder Benzoyl ist, darstellt;
Q₁ für -N(R₈)- oder -O- steht;
E C₁₋₃-Alkylen, die Gruppe -CH₂-CH(R₉)-O-, worin R₉ Wasserstoff, Methyl oder Phenyl ist, darstellt oder E die Gruppe -CH₂)₃-NH- oder eine direkte Bindung ist;
R₁₀ für Wasserstoff oder C₁₋₁₈-Alkyl steht,
R₈ Wasserstoff, C₁₋₁₈-Alkyl, C₅₋₇-Cycloalkyl, C₇₋₁₂-Aralkyl, Cyanoethyl, C₆₋₁₀-Aryl, die Gruppe -CH₂-CH(R₉)-OH, worin R₉ die vorstehend angegebene Bedeutung besitzt, darstellt; oder R₈ eine Gruppe der Formel I oder eine Gruppe der Formel 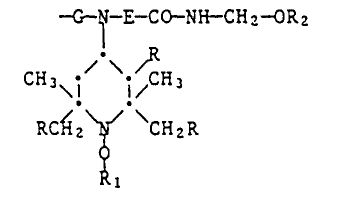 ist, worin G C₂₋₆-Alkylen oder C₆₋₁₂-Arylen bedeutet; oder R₈ eine Gruppe -E-CO-NH-CH₂-OR₁₀ ist;
T₃ Ethylen oder 1,2-Propylen ist oder die sich wiederholende Struktureinheit, abgeleitet von einem α-Olefin-Copolymeren mit einem Alkylacrylat oder -methacrylat, darstellt; k für 2 bis 100 steht;
T₄ die gleiche Bedeutung wie R₄ besitzt, wenn p für 1 oder 2 steht,
T₅ Methyl ist,
T₆ Methyl oder Ethyl bedeutet oder T₅ und T₆ gemeinsam für Tetramethylen oder Pentamethylen stehen,
M und Y unabhängig Methylen oder Carbonyl bedeuten;
T₇ die gleiche Bedeutung wie R₇ besitzt,
T₁₀ und T₁₁ unabhängig Alkylen mit 2 bis 12 Kohlenstoffatomen bedeuten oder T₁₁ eine Gruppe der Formel II ist,
e für 2, 3 oder 4 steht,
T₁₂ eine Gruppe -N(R⁴)-(CH₂)_{d}-N(R⁴)- oder 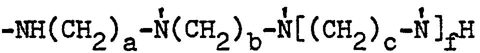 darstellt, worin a, b und
c unabhängig für 2 oder 3 stehen, d für 2-10 steht und f für 0 oder 1 steht, wenn n für 1 steht, T₁₃ C₂₋₁₈-Alkyl, C₂₋₁₈-Alkenyl, C₆₋₈-Cycloalkyl, C₆₋₁₂-Aryl oder Phenyl, substituiert durch C₁₋₄-Alkyl, Hydroxy oder Halogen, bedeutet und,wenn n für 2 steht, T₁₃ die gleiche Bedeutung wie R₂ besitzt;
E₁ und E₂ unabhängig jeweils für -CO- oder -N(E₅)- stehen, worin E₅ Wasserstoff, C₁₋₁₂-Alkyl oder Alkoxycarbonylalkyl mit 4 bis 22 Kohlenstoffatomen bedeutet;
E₃ Wasserstoff, Alkyl mit 1 bis 30 Kohlenstoffatomen, Phenyl, Naphthyl, dieses Phenyl oder dieses Naphthyl, substituiert durch Chlor oder durch Alkyl mit 1 bis 4 Kohlenstoffatomen, oder Phenylalkyl mit 7 bis 12 Kohlenstoffatomen oder dieses Phenylalkyl, substituiert durch Alkyl mit 1 bis 4 Kohlenstoffatomen, bedeutet, und
E₄ Wasserstoff, Alkyl mit 1 bis 30 Kohlenstoffatomen, Phenyl, Naphthyl oder Phenylalkyl mit 7 bis 12 Kohlenstoffatomen ist, oder
E₃ und E₄ gemeinsam Polymethylen mit 4 bis 17 Kohlenstoffatomen oder dieses Polymethylen, substituiert mit bis zu vier Alkylgruppen mit 1 bis 4 Kohlenstoffatomen, bedeuten, vorausgesetzt, daß in Formel B' R₃ oder R₄ nicht Wasserstoff bedeuten, wenn R₁ Alkyl ist.

20. Verbindungen gemäß Anspruch 19 der Formeln(A') bis (F'), worin R für Wasserstoff steht.

21. Verbindungen gemäß Anspruch 19 der Formeln (G') bis (M'), worin T₅ und T₆ Methyl bedeuten.

22. Verbindungen gemäß Anspruch 19 der Formeln (A') bis (M'), worin R Wasserstoff bedeutet, R₁ C₁₋₁₈-Alkyl, C₂₋₆-Alkenyl, C₅₋₈-Cycloalkyl, Cyclohexyl, Phenyl oder C₇₋₉-Aralkyl bedeutet; m für 2-4 steht und, wenn m für 2 steht, R₂ C₂₋₈-Alkylen, C₄₋₈-Alkenylen, Xylylen, einen zweiwertigen Acylrest einer aliphatischen, cycloaliphatischen oder aromatischen Dicarbonsäure mit bis zu 12 Kohlenstoffatomen oder einer aliphatischen oder aromatischen Dicarbamidsäure mit bis zu 12 Kohlenstoffatomen bedeutet oder eine Gruppe der Formel 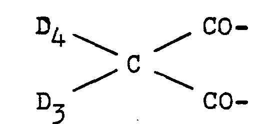 ist, worin D₃ und D₄ unabhängig Wasserstoff, C₁₋₆-Alkyl, Benzyl oder 3,5-Di-tert.-butyl-4-hydroxybenzyl bedeuten, und wenn m für 3 steht, R₂ einen dreiwertigen Acylrest einer aliphatischen oder aromatischen Tricarbonsäure mit bis zu 12 Kohlenstoffatomen darstellt, und wenn m für 4 steht, R₂ einen vierwertigen Acylrest einer aliphatischen oder aromatischen Tetracarbonsäure mit bis zu 12 Kohlenstoffatomen darstellt;
p für 1, 2 oder 3 steht, R₃ Wasserstoff, C₁₋₁₂-Alkyl, C₅₋₈-Cycloalkyl, C₇₋₉-Aralkyl, C₂₋₁₈-Alkanoyl oder Benzoyl bedeutet, und wenn p für 1 steht, R₄ Wasserstoff, C₁₋₁₈-Alkyl, C₅₋₈-Cycloalkyl, Phenyl, Benzyl oder eine Gruppe der Formel 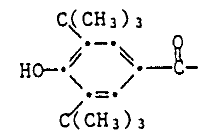 oder der Formel 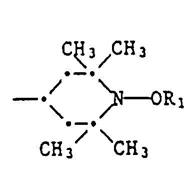 darstellt, und wenn p für 2 steht, R₄ C₂₋₁₂-Alkylen, C₆₋₁₂-Arylen, Xylylen bedeutet oder, mit der Maßgabe, daß R₃ nicht Alkanoyl oder Benzoyl ist, R₄ auch ein zweiwertiger Acylrest einer aliphatischen oder aromatischen Dicarbonsäure mit bis zu 12 Kohlenstoffatomen, einer aliphatischen oder aromatischen Dicarbamidsäure mit bis zu 12 Kohlenstoffatomen sein kann oder eine Gruppe der Formel II sein kann, worin T₈ und T₉ unabhängig Wasserstoff oder C₁₋₁₂-Alkyl bedeuten oder T₈ und T₉ gemeinsam C₄₋₆-Alkylen oder 3-Oxapentamethylen sind, und wenn p für 3 steht, R₄ 2,4,6-Triazintriyl bedeutet; n für 1 oder 2 steht, und wenn n für 1 steht, R₅ und R'₅ C₁₋₁₂-Alkyl oder Benzyl bedeuten oder R₅ und R'₅ gemeinsam C₂₋₈-Alkylen oder Hydroxyalkylen bedeuten, und wenn n für 2 steht, R₅ und R'₅ gemeinsam für (-CH₂)₂C(CH₂-)₂ stehen;
R₆ Wasserstoff, C₁₋₁₂-Alkyl, Allyl oder Benzyl bedeutet, und wenn n für 1 steht, R₇ Wasserstoff, C₁₋₁₂-Alkyl, Allyl, Benzyl, Cyclohexyl, 2-Hydroxyethyl oder eine Gruppe der Formel -CH₂CH₂-COOQ bedeutet, worin Q für C₁₋₄-Alkyl steht, und wenn n für 2 steht, R₇ C₂₋₁₂-Alkylen oder C₆₋₁₂-Arylen ist;
Q₁ für -N(R₈)- oder -O- steht; E C₁₋₃-Alkylen oder eine direkte Bindung darstellt; R₁₀ Wasserstoff oder C₁₋₁₄-Alkyl bedeutet, R₈ Wasserstoff, C₁₋₁₂-Alkyl, Cyclohexyl, Benzyl, Cyanoethyl oder eine Gruppe 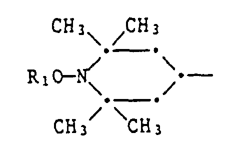 darstellt, T₃ für Ethylen oder 1,2-Propylen steht, k für 2 bis 100 steht;
T₄ die gleiche Bedeutung wie R₄ besitzt, wenn p für 1 oder 2 steht,
T₅ und T₆ Methyl sind, M und Y unabhängig für -CH₂- oder -CO- stehen;
T₇ die gleiche Bedeutung wie R₇ besitzt;
T₁₀ und T₁₁ unabhängig C₂₋₈-Alkylen bedeuten oder T₁₁ eine Gruppe der Formel I ist,
e für 3 oder 4 steht,
T₁₂ eine Gruppe 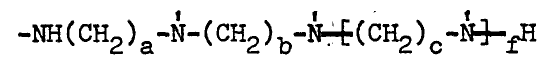 ist, worin a, b und c unabhängig für 2 oder 3 stehen und f für 0 oder 1 steht;
wenn n für 1 steht, T₁₃ C₂₋₁₈-Alkyl, Cyclohexyl oder Phenyl bedeutet, und wenn n für 2 steht, T₁₃ die gleiche Bedeutung wie R₂ besitzt;
E₁ für -CO- steht und E₂ für -N(E5)- steht, worin E₅ Wasserstoff, C₁₋₁₂-Alkyl oder C₄₋₁₈-Alkoxycarbonylalkyl bedeutet, E₃ und E₄ unabhängig C₁₋₁₂-Alkyl oder Phenyl bedeuten oder E₃ und E₄ gemeinsam C₄₋₁₂-Polymethylen darstellen.

23. Verbindungen gemäß Anspruch 19 der Formel (A'), (B'), (C'), (J'), (K') oder (M'), worin R Wasserstoff bedeutet, R₁ C₁₋₁₈-Alkyl, Cyclohexyl, Cyclohexenyl, Methylcyclohexyl oder C₇₋₉-Phenylalkyl ist;
m für 2 steht, R₂ C₂₋₈-Alkylen, Xylylen oder eine Gruppe -CO-R₁₁-CO- darstellt, worin R₁₁ C₂₋₈-Alkylen, Cyclohexylen oder Phenylen oder eine Gruppe 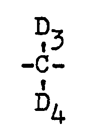 bedeutet, worin D₃ und D₄ unabhängig Wasserstoff, C₁₋₄-Alkyl, Benzyl oder 3,5-Di-tert.-butyl-4-hydroxybenzyl darstellen;
p für 1 oder 2 steht, R₃ Wasserstoff, C₁₋₁₂-Alkyl oder C₂₋₈-Alkanoyl bedeutet, und wenn p für 1 steht, R₄ Wasserstoff, C₁₋₁₂-Alkyl darstellt, und wenn p für 2 steht, R₄ C₂₋₈-Alkylen bedeutet oder -CO-R₁₁-CO- ist;
n für 1 oder 2 steht, und wenn n für 1 steht, R₅ und R'₅ gemeinsam C₂₋₈-Alkylen bedeuten, und wenn n für 2 steht,
R₅ und R'₅ gemeinsam (-CH₂)₂C(CH₂-)₂ bedeuten;
k für 5-20 steht, T₁₀ C₂₋₈-Alkylen bedeutet und T₁₁ eine Gruppe der Formel II ist, worin T₈ und T₉ unabhängig Wasserstoff oder C₁₋₁₂-Alkyl bedeuten oder T₈ und T₉ gemeinsam Pentamethylen oder 3-Oxapentamethylen sind, T₅ und T₆ Methyl sind;
e für 4 steht, T₁₂ eine Gruppe 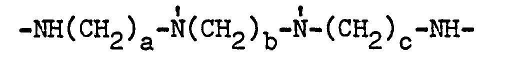 darstellt, worin a, b und c unabhängig für 2 oder 3 stehen;
E₁ für -CO- steht und E₂ für -N(E₅)- steht, worin E₅ Wasserstoff, C₁₋₁₂-Alkyl oder C₄₋₁₅-Alkoxycarbonylalkyl bedeutet und E₃ und E₄ unabhängig C₁₋₁₂-Alkyl darstellen oder E₃ und E₄ gemeinsam C₅₋₁₂-Polymethylen sind.

24. Di-(1-methoxy-2,2,6,6-tetramethylpiperidin-4-yl)-sebacat gemäß Anspruch 19.

25. Di-(1-cyclohexyloxy-2,2,6,6-tetramethylpiperidin-4-yl)-sebacat gemäß Anspruch 19.

26. Di-(1-cyclohexyloxy-2,2,6,6-tetramethylpiperidin-4-yl)-isophthalat gemäß Anspruch 19.

27. Di-(1-octyloxy-2,2,6,6-tetramethylpiperidin-4-yl)-sebacat gemäß Anspruch 19.

28. 3,15-Dicyclohexyloxy-2,2,4,4,14,14,16,16-octamethyl-7,11,18,21-tetraoxa-3,15-diazatrispiro[5.2.2.5.2.2.]-heneicosan gemäß Anspruch 19.

29. Di-(1-cyclohexyloxy-2,2,6,6-tetramethylpiperidin-4-yl)-succinat gemäß Anspruch 19.

30. Verwendung einer Verbindung gemäß Anspruch 19 als Stabilisator für organische Materialien.

31. Verwendung gemäß Anspruch 30 als Stabilisator für Polymere.

32. Verwendung gemäß Anspruch 30 als Stabilisator für photographische Schichten.

33. Organisches Material, enthaltend eine wirksame Menge einer Verbindung gemäß Anspruch 19.

## Patentansprüche (Patentansprüche für folgende(n) Vertragsstaat(en): ES)

1. Stabilisierte, bei Raumtemperatur härtende oder säurekatalysierte, wärmehärtende Überzugszusammensetzung, enthaltend eine wirksame, stabilisierende Menge einer gehinderten Aminverbindung, die die Gruppe 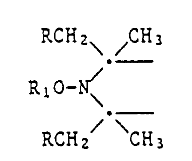 aufweist, worin R für Wasserstoff oder Methyl steht und R₁ C₁₋₁₈-Alkyl, C₂₋₁₈-Alkenyl, C₂₋₁₈-Alkinyl, C₅₋₁₂-Cycloalkyl, C₆₋₁₀-Bicycloalkyl, C₅₋₈-Cycloalkenyl, C₆₋₁₀-Aryl, C₇₋₉-Aralkyl oder durch C₁₋₄-Alkyl oder Phenyl substituiertes C₇₋₉-Aralkyl bedeutet.

2. Zusammensetzung gemäß Anspruch 1, die eine gehinderte Aminverbindung entsprechend einer der Formeln A-N enthält: 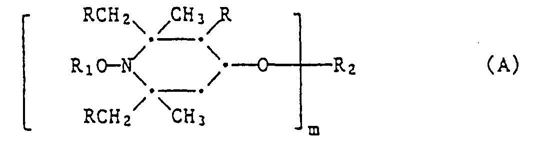 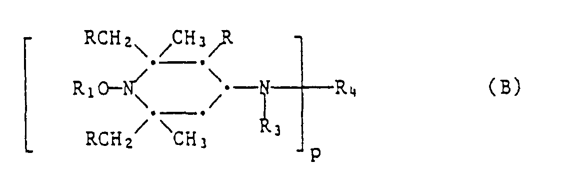 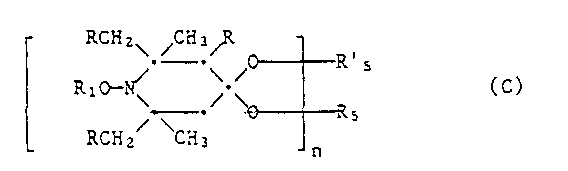 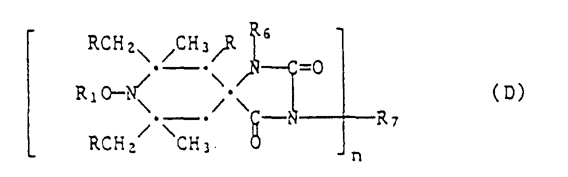 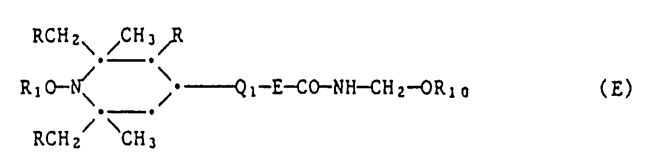 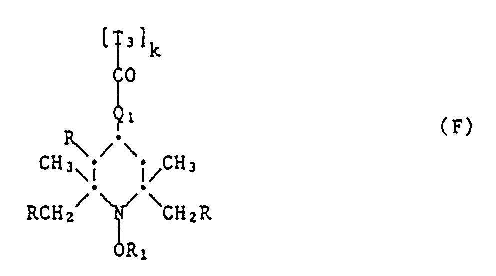 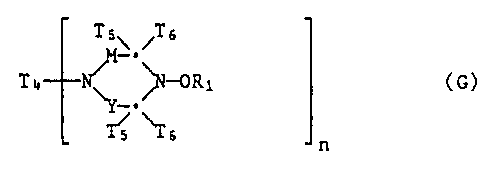 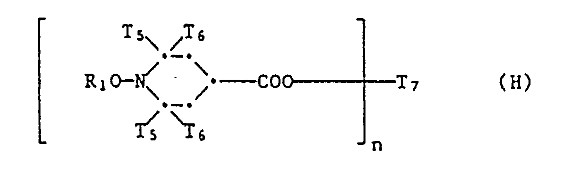 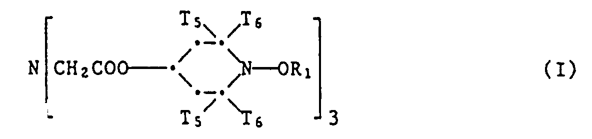 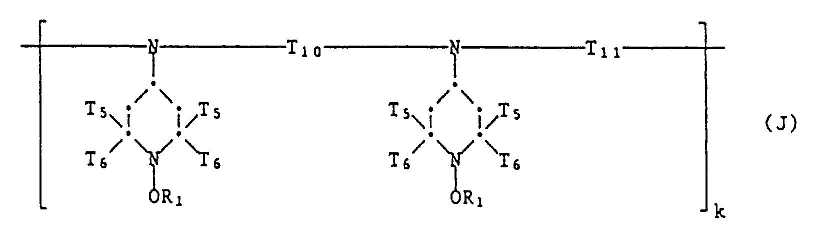 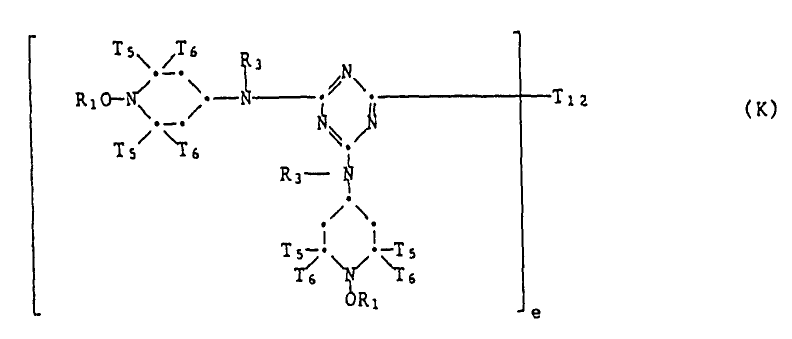 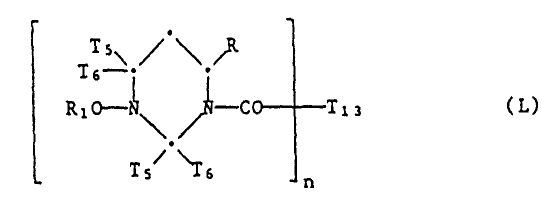 oder 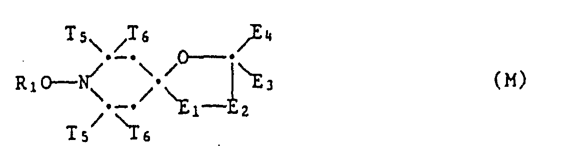 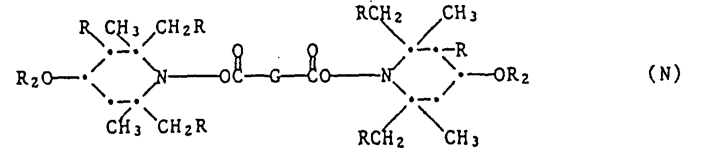 worin
R für Wasserstoff oder Methyl steht,
R₁ C₁₋₁₈-Alkyl, C₂₋₁₈-Alkenyl, C₂₋₁₈-Alkinyl, C₅₋₁₂-Cycloalkyl, C₆₋₁₀-Bicycloalkyl, C₅₋₈-Cycloalkenyl, C₆₋₁₀-Aryl, C₇₋₉-Aralkyl oder durch C₁₋₄-Alkyl oder Phenyl substituiertes C₇₋₉-Aralkyl bedeutet;
m für 1 bis 4 steht,
wenn m 1 ist,
R₂ Wasserstoff, C₁₋₁₈-Alkyl, das gegebenenfalls durch ein oder mehrere Sauerstoffatome unterbrochen ist, C₂₋₁₂-Alkenyl, C₆₋₁₀-Aryl, C₇₋₁₈-Aralkyl, Glycidyl, einen einwertigen Acylrest einer aliphatischen, cycloaliphatischen, araliphatischen oder aromatischen Carbonsäure oder einer Carbamidsäure bedeutet oder R₂ eine Gruppe 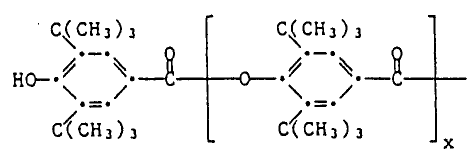 ist, worin x für 0 oder 1 steht, oder eine Gruppe 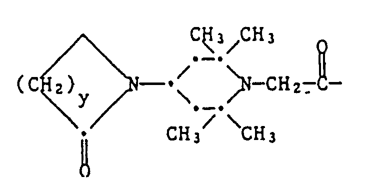 ist, worin y für 2-4 steht;
wenn m für 2 steht,
R₂ C₁₋₁₂-Alkylen, C₄₋₁₂-Alkenylen, Xylylen, einen zweiwertigen Acylrest einer aliphatischen, cycloaliphatischen, araliphatischen oder aromatischen Dicarbonsäure oder einer Dicarbamidsäure bedeutet oder eine Gruppe 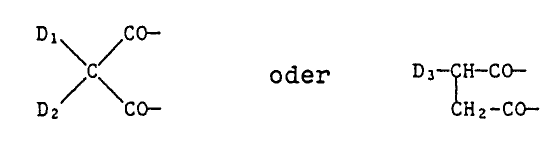 ist, worin D₁ und D₂ unabhängig Wasserstoff, Alkyl mit bis zu8 Kohlenstoffatomen, Phenyl, Benzyl oder 3,5-Di-tert.-butyl-4-hydroxybenzyl bedeuten und D₃ einen Alkyl- oder Alkenylrest mit bis zu 18 Kohlenstoffatomen darstellt;
wenn m für 3 steht, R₂ einen dreiwertigen Acylrest einer aliphatischen, cycloaliphatischen oder aromatischen Tricarbonsäure bedeutet;
wenn m für 4 steht, R₂ einen vierwertigen Acylrest einer aliphatischen oder aromatischen Tetracarbonsäure bedeutet; p für 1, 2 oder 3 steht,
R₃ Wasserstoff, C₁₋₁₂-Alkyl, C₅₋₇-Cycloalkyl, C₇₋₉-Aralkyl, C₂₋₁₈-Alkanoyl, C₃₋₅-Alkenoyl oder Benzoyl bedeutet; wenn p für 1 steht,
R₄ Wasserstoff, C₁₋₁₈-Alkyl, C₅₋₇-Cycloalkyl, C₂₋₈-Alkenyl, welches unsubstituiert oder durch eine Cyano-, Carbonyloder Carbamidgruppe substituiert ist, bedeutet oder Aryl, Aralkyl, Glycidyl, eine Gruppe der Formel -CH₂-CH(OH)-Z oder -CONH-Z ist, worin Z für Wasserstoff, Methyl oder Phenyl steht; oder R₄ eine Gruppe der Formel I 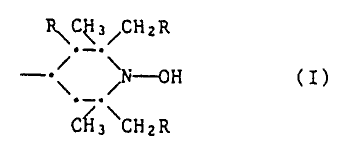 oder eine Gruppe der Formel 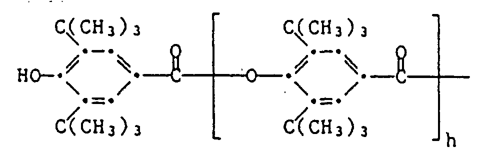 wobei h = 0 oder 1,
ist; oder R₃und R₄ gemeinsam Alkylen mit 4 bis 6 Kohlenstoffatomen oder 1-Oxo-alkylen oder den zweiwertigen Acylrest einer aliphatischen oder aromatischen 1,2- oder 1,3-Dicarbonsäure darstellen;
wenn p für 2 steht,
R₄ C₂₋₁₂-Alkylen, C₆₋₁₂-Arylen, Xylylen, eine Gruppe -CH₂CH(OH)-CH₂- oder eine Gruppe -CH₂-CH(OH)-CH₂-O-X-O-CH₂-CH(OH)-CH₂- darstellt, worin X C₂₋₁₀-Alkylen, C₆₋₁₅-Arylen oder C₆₋₁₂-Cycloalkylen bedeutet; oder, mit der Maßgabe, daß R₃ nicht Alkanoyl, Alkenoyl oder Benzoyl bedeutet, R₄ auch ein zweiwertiger Acylrest einer aliphatischen, cycloaliphatischen oder aromatischen Dicarbonsäure oder Dicarbamidsäure sein kann oder die Gruppe -CO- sein kann; oder R₄ eine Gruppe der Formel II 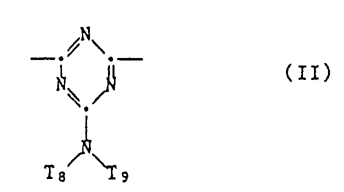 ist, worin T₈ und T₉ unabhängig Wasserstoff, Alkyl mit 1 bis 18 Kohlenstoffatomen oder eine Gruppe der Formel I bedeuten oder T₈ und T₉ gemeinsam Alkylen mit 4 bis 6 Kohlenstoffatomen oder 3-Oxapentamethylen bedeuten;
wenn p für 3 steht,
R₄ 2,4,6-Triazintriyl ist,
n für 1 oder 2 steht,und
wenn n für 1 steht,
R₅ und R'₅ unabhängig C₁₋₁₂-Alkyl, C₂₋₁₂-Alkenyl, C₇₋₁₂-Aralkyl bedeuten oder R₅ auch Wasserstoff ist oder R₅ und R'₅ gemeinsam C₂₋₈-Alkylen oder Hydroxyalkylen oder C₄₋₂₂-Acyloxyalkylen bedeuten;
wenn n für 2 steht,
R₅ und R'₅ gemeinsam für (-CH₂)₂C(CH₂-)₂ stehen;
R₆ Wasserstoff, C₁₋₁₂-Alkyl, Allyl, Benzyl, Glycidyl oder C₂₋₆-Alkoxyalkyl bedeutet;
wenn n für 1 steht,
R₇ Wasserstoff, C₁₋₁₂-Alkyl, C₃₋₅-Alkenyl, C₇₋₉-Aralkyl, C₅₋₇-Cycloalkyl, C₂₋₄-Hydroxyalkyl, C₂₋₆-Alkoxyalkyl, C₆₋₁₀-Aryl, Glycidyl, eine Gruppe der Formel -(CH₂)ₜ-COO-Q oder der Formel -(CH₂)ₜ-O-CO-Q darstellt, worin t für 1 oder 2 steht und Q C₁₋₄-Alkyl oder Phenyl bedeutet; oder wenn n für 2 steht,
R₇ C₂₋₁₂-Alkylen, C₆₋₁₂-Arylen, eine Gruppe -CH₂CH(OH)-CH₂-O-X-O-CH₂-CH(OH)-CH₂- worin X C₂₋₁₀-Alkylen, C₆₋₁₅-Arylen oder C₆₋₁₂-Cycloalkylen bedeutet, oder eine Gruppe -CH₂CH(OZ')CH₂-(OCH₂-CH(OZ')CH₂)₂-, worin Z' Wasserstoff, C₁₋₁₈-Alkyl, Allyl, Benzyl, C₂₋₁₂-Alkanoyl oder Benzoyl bedeutet, darstellt;
Q₁ für -N(R₈)- oder -O- steht;
E C₁₋₃-Alkylen, die Gruppe -CH₂-CH(R₉)-O- bedeutet, worin R₉ Wasserstoff, Methyl oder Phenyl ist, oder E die Gruppe -(CH₂)₃-NH- oder eine direkte Bindung darstellt;
R₁₀ für Wasserstoff oder C₁₋₁₈-Alkyl steht;
R₈ Wasserstoff, C₁₋₁₈-Alkyl, C₅₋₇-Cycloalkyl, C₇₋₁₂-Aralkyl, Cyanoethyl, C₆₋₁₀-Aryl, die Gruppe -CH₂-CH(R₉)-OH, in der R₉ die vorstehend angegebene Bedeutung besitzt, eine Gruppe der Formel I oder eine Gruppe der Formel 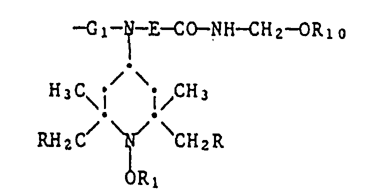 darstellt,
worin G₁ C₂₋₆-Alkylen oder C₆₋₁₂-Arylen sein kann, oder R₈ eine Gruppe -E-CO-NH-CH₂-OR₁₀ ist;
T₃ Ethylen oder 1,2-Propylen bedeutet oder die sich wiederholende Struktureinheit, abgeleitet von einem α-Olefincopolymeren mit einem Alkylacrylat oder -methacrylat, darstellt;
k für 2 bis 100 steht;
T₄ die für R₄ angegebene Bedeutung besitzt, wenn p für 1 oder 2 steht,
T₅ Methyl bedeutet,
T₆ Methyl oder Ethyl bedeutet oder T₅ und T₆ gemeinsam Tetramethylen oder Pentamethylen bedeuten;
M und Y unabhängig Methylen oder Carbonyl sind;
T₇ die gleiche Bedeutung wie R₇ besitzt;
T₁₀ und T₁₁ unabhängig Alkylen mit 2 bis 12 Kohlenstoffatomen darstellen oder T₁₁ eine Gruppe der Formel II ist;
e für 2, 3 oder 4 steht und
T₁₂ eine Gruppe der Formel -N(R₅)-(CH₂)_{d}-N(R₅)- oder 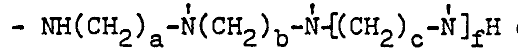 darstellt, worin a, b und c unabhängig für 2 oder 3 stehen, d für 2 bis 10 steht und f für 0 oder 1 steht,
T₁₃ die Bedeutung von R₄ besitzt mit der Maßgabe, daß T₁₃ nicht Wasserstoff sein kann, wenn n für 1 steht;
E₁ und E₂, die verschieden sind, jeweils -CO- oder -N(E₅)-bedeuten, worin E₅ Wasserstoff, C₁₋₁₂-Alkyl oder C₄₋₂₂-Alkoxycarbonylalkyl ist;
E₃ Wasserstoff, Alkyl mit 1 bis 30 Kohlenstoffatomen, Phenyl, Naphthyl, dieses Phenyl oder dieses Naphthyl,substituiert durch Chlor oder durch Alkyl mit 1 bis 4 Kohlenstoffatomen, oder Phenylalkyl mit 7 bis 12 Kohlenstoffatomen oder dieses Phenylalkyl,substituiert durch Alkyl mit 1 bis 4 Kohlenstoffatomen, bedeutet;
E₄ Wasserstoff, Alkyl mit 1 bis 30 Kohlenstoffatomen, Phenyl, Naphthyl oder Phenylalkyl mit 7 bis 12 Kohlenstoffatomen bedeutet, oder
E₃ und E₄ gemeinsam Polymethylen mit 4 bis 17 Kohlenstoffatomen oder dieses Polymethylen, substituiert durch bis zu vier Alkylgruppen mit 1 bis 4 Kohlenstoffatomen, bedeuten; R₂ der Formel (N) wie vorstehend definiert ist, wenn m für 1 steht; und
G eine direkte Bindung, C₁₋₁₂-Alkylen, Phenylen oder -NH-G'-NH, worin G' C₁₋₁₂-Alkylen bedeutet, darstellt.

3. Zusammensetzung gemäß Anspruch 2, die eine Verbindung der Formel A, B, C, D, J, K oder M enthält, worin R für Wasserstoff steht und T₅ und T₆ Methyl sind.

4. Zusammensetzung gemäß Anspruch 2, die eine Verbindung der Formel A, B, C, J oder K enthält, worin R für Wasserstoff steht und R₁ C₁₋₁₈-Alkyl, C₆₋₁₂-Cycloalkyl, Cyclohexenyl oder C₇₋₉-Phenylalkyl bedeutet.

5. Zusammensetzung gemäß Anspruch 2, die eine Verbindung der Formel A enthält,
worin R Wasserstoff bedeutet und R₁ C₁₋₁₈-Alkyl, C₆₋₁₂-Cycloalkyl, Cyclohexenyl oder C₇₋₉-Phenylalkyl bedeutet, m für 1, 2 oder 4 steht und wenn m für 1 steht, R₂ C₁₋₁₂-Alkyl, Allyl, Benzyl oder einen Acylrest einer aliphatischen C₂₋₁₈-Carbonsäure, einer cycloaliphatischen C₆₋₁₂-Carbonsäure oder einer aromatischen C₇₋₁₅-Carbonsäure bedeutet, und wenn m für 2 steht, R₂ C₁₋₈-Alkylen, Butylen, Xylylen ist oder einen zweiwertigen Acylrest einer aliphatischen C₂₋₁₈-Dicarbonsäure, einer cycloaliphatischen oder aromatischen C₈₋₁₄-Dicarbonsäure oder einer aliphatischen, cycloaliphatischen oder aromatischen C₈₋₁₄-Dicarbamidsäure bedeutet oder R₂ eine Gruppe 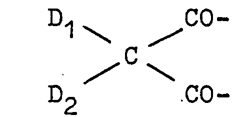 darstellt, worin D₁ C₁₋₈-Alkyl oder 3,5-'Di-tert.-butyl-4-hydroxybenzyl bedeutet und D₂ die Bedeutung von D₁ besitzt oder Wasserstoff darstellt und wenn m für 4 steht, R₂ einen vierwertigen Acylrest einer Butan- oder Pentantetracarbonsäure darstellt.

6. Zusammensetzung gemäß Anspruch 5, die eine Verbindung der Formel A enthält,
worin R Wasserstoff bedeutet, R₁ C₁₋₁₀-Alkyl, Cycloalkyl, Cyclohexyl oder C₇₋₉-Phenylalkyl ist, m für 1 oder 2 steht und wenn m für 1 steht, R₂ Benzyl, C₂₋₁₈-Alkanoyl, Benzoyl oder 3,5-Di-tert.-butyl-4-hydroxybenzoyl bedeutet, und wenn m für 2 steht, R₂ Xylylen oder einen zweiwertigen Acylrest einer aliphatischen C₄₋₁₀-Dicarbonsäure oder einer Benzoldicarbonsäure bedeutet.

7. Zusammensetzung gemäß Anspruch 2, die eine Verbindung der Formel B enthält,
worin R Wasserstoff bedeutet und R₁ C₁₋₁₈-Alkyl, C₆₋₁₂-Cycloalkyl, Cyclohexenyl oder C₇₋₉-Phenylalkyl bedeutet, p für 1 oder 2 steht, R₃ Wasserstoff, C₁₋₁₂-Alkyl oder C₂₋₁₂-Alkanoyl, Allyl bedeutet und wenn p für 1 steht, R₄ Wasserstoff, C₁₋₁₂-Alkyl oder eine Gruppe der Formel I darstellt, und wenn p für 2 steht, R₄ C₂₋₈-Alkylen oder Xylylen bedeutet, und wenn R₃ nicht Alkanoyl ist, R₄ auch ein zweiwertiger Acylrest einer aliphatischen C₄₋₁₀-Dicarbonsäure oder einer Benzoldicarbonsäure sein kann, oder eine Gruppe der Formel II darstellt, worin T₈ Wasserstoff oder C₁₋₄-Alkyl bedeutet und T₉ C₁₋₁₂-Alkyl oder eine Gruppe der Formel I darstellt.

8. Zusammensetzung gemäß Anspruch 1, worin die gehinderte Aminverbindung in einer Menge von 0,1 bis 10 Gew.%, basierend auf Harzfeststoffen, enthalten ist.

9. Zusammensetzung gemäß Anspruch 1, die zusätzlich einen UV-Absorber, ausgewählt unter Benzophenonen, Benzotriazolen, Acrylsäurederivaten, Aryl-s-triazinen, organischen Nickelverbindungen und Oxaniliden, enthält.

10. Zusammensetzung gemäß Anspruch 9, die einen Benzotriazol-UV-Absorber enthält.

11. Zusammensetzung gemäß Anspruch 9, die einen Benzotriazol-UV-Absorber, ausgewählt unter 2-[2-Hydroxy-3,5-di-(α,α-dimethylbenzyl)-phenyl]-benzotriazol, 2-(2-Hydroxy-3,5-di-tert.-octylphenyl)-benzotriazol, 2-(2-Hydroxy-3-α,α-dimethylbenzyl-5-tert.-octylphenyl)-benzotriazol, 2-(2-Hydroxy-3-tert.-octyl-5-α,α-dimethylbenzylphenyl)-benzotriazol, 2-(2-Hydroxy-3,5-di-tert.-amylphenyl)-benzotriazol, 2-[2-Hydroxy-3-tert.-butyl-5-(2-(omega-hydroxy-octa-(ethylenoxy)-carbonyl)-ethylphenyl]-benzotriazol, 5-Chlor-2-[2-hydroxy-3,5-di-(α,α-dimethylbenzyl)-phenyl]-benzotriazol, 5-Chlor-2-(2-hydroxy-3,5-di-tert.-butylphenyl)-benzotriazol, 2-[2-Hydroxy-3-tert.-butyl-5-(2-octyloxycarbonylethyl)-phenyl]-5-chlor-benzotriazol, 2-(2-Hydroxy-3-sek.dodecyl-5-methylphenyl)-benzotriazol und Hexamethylen-di-[β-(3-tert.-butyl-4-hydroxy-5-[2-benzotriazolyl]-phenyl)-propionat], enthält.

12. Zusammensetzung gemäß Anspruch 9, worin das Benzotriazol 2-[2-Hydroxy-3,5-di-(α,α-dimethylbenzyl)-phenyl]-benzotriazol und 2-[2-Hydroxy-3-tert.-butyl-5-(2-(omegahydroxy-octa-(ethylenoxy)-carbonyl)-ethylphenyl]-benzotriazol ist.

13. Zusammensetzung gemäß Anspruch 9, die zusätzlich ein Phosphit- oder Phosphonit-Antioxidans enthält.

14. Zusammensetzung gemäß Anspruch 1, die zusätzlich ein gehindertes Phenol-Antioxidans enthält.

15. Überzugszusammensetzung gemäß Anspruch 1, die ein bei Raumtemperatur härtbares System, basierend auf einem Alkydharz, thermoplastischen Acrylharz, Acrylalkydharz, Polyurethanharz oder Polyesterharz oder diesen Harzen, modifiziert mit Siliconen, Isocyanaten, Epoxiden, Isocyanuraten, Ketiminen oder Oxazolidinen, ist oder das System auf einem Celluloseester oder einem Epoxidharz basiert.

16. Überzugszusammensetzung gemäß Anspruch 1, die ein säurekatalysiertes, wärmehärtendes System, basierend auf einem heiß vernetzbaren Acryl-, Polyester-, Polyurethan-, Polyamid- oder Alkydharz, ist.

17. Zusammensetzung gemäß Anspruch 1, die ein Email für industrielle Anstriche ist.

18. Zusammensetzung gemäß Anspruch 1, die ein Refinishemail für Kraftfahrzeuge ist.

## Claims (Claims for the following Contracting State(s): BE, DE, FR, GB, IT, NL, SE)

1. A stabilized ambient curing or acid catalyzed thermosetting coating composition containing an effective stabilizing amount of a hindered amine compound containing the group 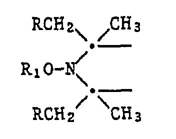 wherein R is hydrogen or methyl and R₁ is C₁-C₁₈ alkyl, C₂-C₁₈ alkenyl, C₂-C₁₈ alkinyl, C₅-C₁₂ cycloalkyl, C₆-C₁₀ bicycloalkyl, C₅-C₈ cycloalkenyl, C₆-C₁₀ aryl, C₇-C₉ aralkyl or C₇-C₉ aralkyl substituted by C₁-C₄ alkyl or phenyl.

2. The composition according to claim 1 which contains a hindered amine compound corresponding to one of formulae A-N 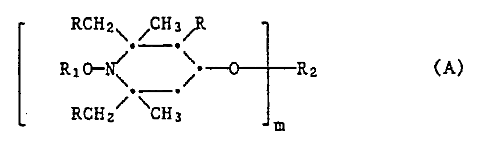 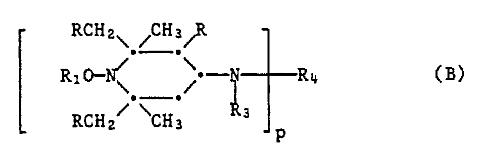 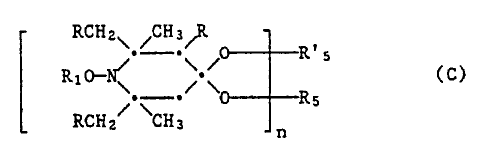 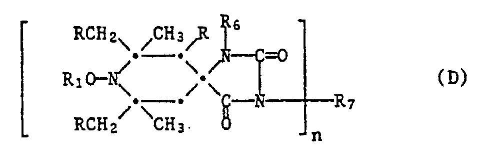 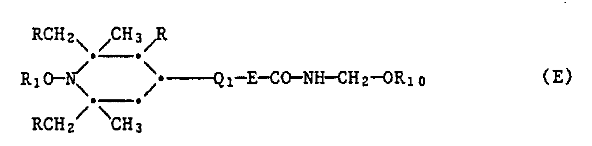 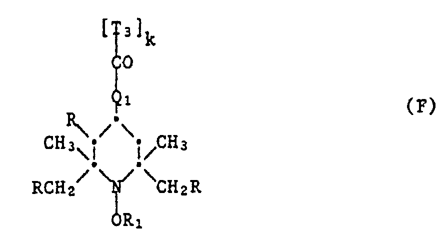 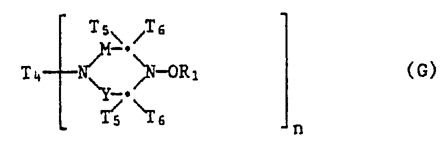 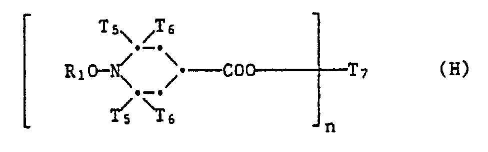 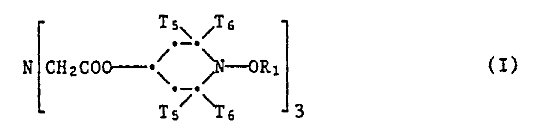 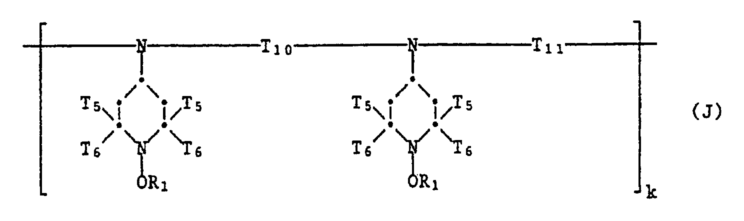 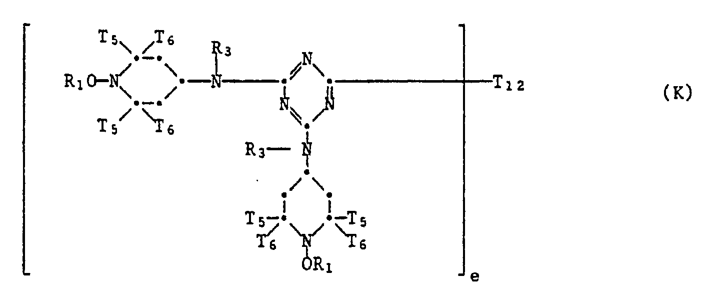 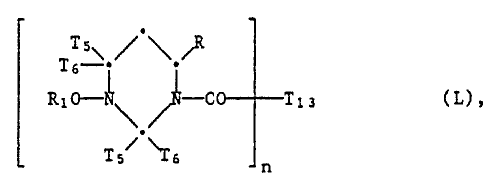 or 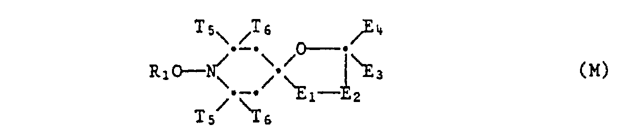 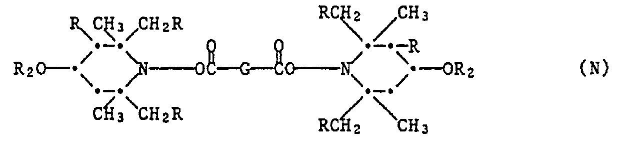 wherein
R is hydrogen or methyl,
R₁ is C₁-C₁₈ alkyl, C₂-C₁₈ alkenyl, C₂-C₁₈ alkinyl, C₅-C₁₂ cycloalkyl, C₆-C₁₀ bicycloalkyl, C₅-C₈ cycloalkenyl, C₆-C₁₀ aryl, C₇-C₉ aralkyl or C₇-C₉ aralkyl substituted by C₁-C₄ alkyl or phenyl;
m is 1-4,
when m is 1,
R₂ is hydrogen, C₁-C₁₈ alkyl optionally interrrupted by one or more oxygen atoms, C₂-C₁₂ alkenyl, C₆-C₁₀ aryl, C₇-C₁₈ aralkyl, glycidyl, a monovalent acyl radical of an aliphatic, cycloaliphatic, araliphatic or aromatic carboxylic acid, or of a carbamic acid or R₂ is a group 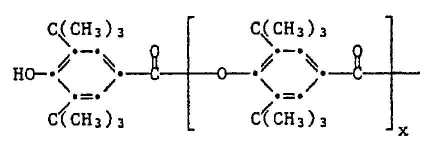 wherein x is 0 or 1, or is a group 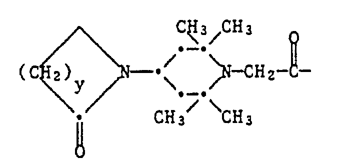 wherein y is 2-4;
when m is 2,
R₂ is C₁-C₁₂ alkylene, C₄-C₁₂ alkenylene, xylylene, a divalent acyl radical of an aliphatic, cycloaliphatic, araliphatic or aromatic dicarboxylic acid or of a dicarbamic acid, or is a group 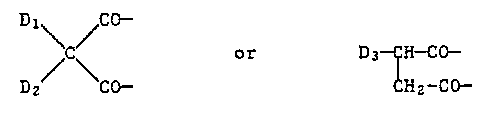 wherein D₁ and D₂ independently are hydrogen, alkyl containing up to 8 carbon atoms, phenyl, benzyl or 3,5-di-t-butyl-4-hydroxybenzyl and D₃ is an alkyl or alkenyl radical containing up to 18 carbon atoms; when m is 3, R₂ is a triavalent acyl radical of an aliphatic, cycloaliphatic, or aromatic tricarboxylic acid;
when m is 4, R₂ is a tetravalent acyl radical of an aliphatic or aromatic tetracarboxylic acid;
p is 1, 2 oder 3,
R₃ is hydrogen, C₁-C₁₂ alkyl, C₅-C₇ cycloalkyl, C₇-C₉ aralkyl, C₂-C₁₈ alkanoyl, C₃-C₅ alkenoyl or benzoyl;
when p is 1,
R₄ is hydrogen, C₁-C₁₈ alkyl, C₅-C₇ cycloalkyl, C₂-C₈ alkenyl unsubstituted or substituted by a cyano, carbonyl or carbamide group, or it is aryl, aralkyl, glycidyl, a group of the formula -CH₂-CH(OH)-Z or -CONH-Z wherein Z is hydrogen, methyl or phenyl; or R₄ is a group of formula I 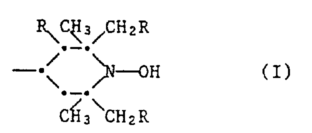 or a group of formula 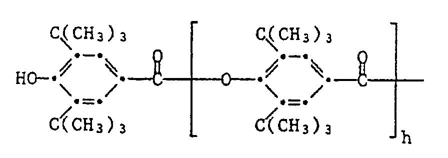 with h as 0 or 1; or R₃ and R₄ together are alkylene of 4 to 6 carbon atoms or 1-oxo-alkylene or the divalent acyl radical of an aliphatic or aromatic 1,2- or 1,3-dicarboxylic acid;
when p is 2,
R₄ is C₂-C₁₂ alkylene, C₆-C₁₂ arylene, xylylene, a -CH₂CH(OH)-CH₂- group, or a group -CH₂-CH(OH)-CH₂-O-X-O-CH₂-CH(OH)-CH₂- wherein X is C₂-C₁₀ alkylene, C₆-C₁₅ arylene or C₆-C₁₂ cycloalkylene; or, provided that R₃ is not alkanoyl, alkenoyl or benzoyl, R₄ can also be a divalent acyl radical of an aliphatic, cycloaliphatic or aromatic dicarboxylic acid or dicarbamic acid, or can be the group -CO-; or R₄ is a group of formula II 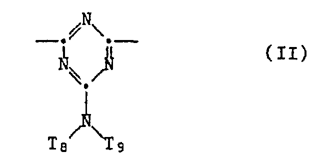 where T₈ and T₉ are independently hydrogen, alkyl of 1 to 18 carbon atoms or a group of formula I, or T₈ and T₉ together are alkylene of 4 to 6 carbon atoms or 3-oxapentamethylene;
when p is 3,
R₄ is 2,4,6-triazinetriyl,
n is 1 or 2 and
when n is 1,
R₅ and R'₅ are independently C₁-C₁₂ alkyl, C₂-C₁₂ alkenyl, C₇-C₁₂ aralkyl, or R₅ is also hydrogen, or R₅ and R'₅ together are C₂-C₈ alkylene or hydroxyalkylene or C₄-C₂₂ acyloxyalkylene;
when n is 2,
R₅ and R'₅ together are (-CH₂)₂C(CH₂-)₂;
R₆ is hydrogen, C₁-C₁₂ alkyl, allyl, benzyl, glycidyl or C₂-C₆ alkoxyalkyl;
when n is 1,
R₇ is hydrogen, C₁-C₁₂ alkyl, C₃-C₅ alkenyl, C₇-C₉ aralkyl, C₅-C₇ cycloalkyl, C₂-C₄ hydroxyalkyl, C₂-C₆ alkoxyalkyl, C₆-C₁₀ aryl, glycidyl, a group of the formula -(CH₂)ₜ-COO-Q or of the formula -(CH₂)ₜ-O-CO-Q wherein t is 1 or 2, and Q is C₁-C₄ alkyl or phenyl; or
when n is 2,
R₇ is C₂-C₁₂ alkylene, C₆-C₁₂ arylene, a group -CH₂CH(OH)--CH₂-O-X-O-CH₂-CH(OH)-CH₂- wherein X is C₂-C₁₀ alkylene, C₆-C₁₅ arylene or C₆-C₁₂ cycloalkylene, or a group -CH₂CH(OZ')CH₂-(OCH₂-CH-(OZ')CH₂)₂- wherein Z' is hydrogen, C₁-C₁₈ alkyl, allyl, benzyl, C₂-C₁₂ alkanoyl or benzoyl;
Q₁ is -N(R₈)- or -O-;
E is C₁-C₃ alkylene, the group -CH₂-CH(R₉)-O- wherein R₉ is hydrogen, methyl or phenyl, or E is the group -(CH₂)₃-NH- or a direct bond;
R₁₀ is hydrogen or C₁-C₁₈ alkyl;
R₈ is hydrogen, C₁-C₁₈ alkyl, C₅-C₇ cycloalkyl, C₇-C₁₂ aralkyl, cyanoethyl, C₆-C₁₀ aryl, the group -CH₂-CH(R₉)-OH wherein R₉ has the meaning defined above, a group of the formula I or a group of the formula 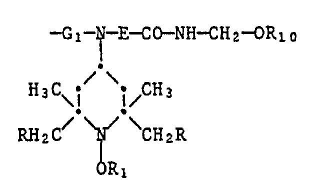 wherein G₁ can be C₂-C₆ alkylene or C₆-C₁₂ arylene, or R₈ is a group -E-CO-NH-CH₂-OR₁₀;
T₃ is ethylene or 1,2-propylene, or is the repeating structural unit derived from an alpha-olefin copolymer with an alkyl acrylate or methacrylate;
k is 2 to 100;
T₄ has the same meaning as R₄ when p is 1 or 2,
T₅ is methyl,
T₆ is methyl or ethyl, or T₅ and T₆ together are tetramethylene or pentamethylene;
M and Y are independently methylene or carbonyl;
T₇ is the same as R₇;
T₁₀ and T₁₁ are independently alkylene of 2 to 12 carbon atoms, or T₁₁ is a group of formula II;
e is 2, 3 or 4 and
T₁₂ is a group of formula -N(R₅)-(CH₂)_{d}-N(R₅)-
or 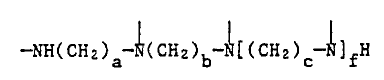 where a, b and c are independently 2 or 3, d is 2 to 10 and f is 0 or 1,
T₁₃ is the same as R₄ with the proviso that T₁₃ cannot be hydrogen when n is 1;
E₁ and E₂, being different, each are -CO- or -N(E₅)- where E₅ is hydrogen, C₁-C₁₂ alkyl or C₄-C₂₂-alkoxycarbonylalkyl;
E₃ is hydrogen, alkyl of 1 to 30 carbon atoms, phenyl, naphthyl, said phenyl or said naphthyl substituted by chlorine or by alkyl of 1 to 4 carbon atoms, or phenylalkyl of 7 to 12 carbon atoms, or said phenylalkyl substituted by alkyl of 1 to 4 carbon atoms;
E₄ is hydrogen, alkyl of 1 to 30 carbon atoms, phenyl, naphthyl or phenylalkyl of 7 to 12 carbon atoms, or
E₃ and E₄ together are polymethylene of 4 to 17 carbon atoms, or said polymethylene substituted by up to four alkyl groups of 1 to 4 carbon atoms;
R₂ of formula (N) is as previously defined when m is 1; and
G is a direct bond, C₁-C₁₂ alkylene, phenylene or -NH-G'-NH wherein G' is C₁-C₁₂ alkylene.

3. A composition according to claim 2 which contains a compound of formula A, B, C, D, J, K or M wherein R is hydrogen and T₅ and T₆ are methyl.

4. A composition according to claim 2 which contains a compound of formula A, B, C, J or K wherein R is hydrogen and R₁ is C₁-C₁₈ alkyl, C₆-C₁₂ cycloalkyl, cyclohexenyl or C₇-C₉ phenylalkyl.

5. A composition according to claim 2 which contains a compound of formula A wherein R is hydrogen and R₁ is C₁-C₁₈ alkyl, C₆-C₁₂ cycloalkyl, cyclohexenyl or C₇-C₉ phenylalkyl, m is 1, 2 or 4 and when m is 1, R₂ is C₁-C₁₂ alkyl, allyl, benzyl or an acyl radical of an aliphatic C₂-C₁₈ carboxylic acid, of a cycloaliphatic C₆-C₁₂ carboxylic acid or of an aromatic C₇-C₁₅ carboxylic acid, and when m is 2, R₂ is C₁-C₈ alkylene, butylene, xylylene or is a divalent acyl radical of an aliphatic C₂-C₁₈ dicarboxylic acid cycloaliphaticor of a cycloaliphatic or aromatic C₈-C₁₄ dicarboxylic acid, or of an aliphatic, cycloaliphatic or aromatic C₈-C₁₄ dicarbamic acid, or R₂ is a group 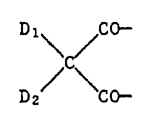 wherein D₁ C₁-C₈ alkyl or 3,5-di-tert.butyl-4-hydroxybenzyl and D₂ is D₁ or hydrogen and when m is 4, R₂ is a tetravalent acyl radical of a butane- or pentanetetracarboxylic acid.

6. A composition according to claim 5 which contains a compound of formula A wherein R is hydrogen, R₁ is C₁-C₁₀ alkyl, cycloalkyl, cyclohexyl or C₇-C₉ phenylalkyl, m is 1 or 2, and when m is 1, R₂ is benzyl, C₂-C₁₈ alkanoyl, benzoyl, or 3,5-di-tert.butyl-4-hydroxybenzoyl, and when m is 2, R₂ is xylylene or a divalent acyl radical of an aliphatic C₄-C₁₀ dicarboxylic acid or of a benzene dicarboxylic acid.

7. A composition according to claim 2 which contains a compound of formula B wherein R is hydrogen and R₁ is C₁-G₁₈ alkyl, C₆-C₁₂ cycloalkyl, cyclohexenyl or C₇-C₉ phenylalkyl, p is 1 or 2, R₃ is hydrogen, C₁-C₁₂ alkyl or C₂-C₁₂ alkanoyl, allyl, and when p is 1, R₄ is hydrogen, C₁-C₁₂ alkyl or a group of formula I, and when p is 2, R₄ is C₂-C₈ alkylene or xylylene and if R₃ is not alkanoyl, R₄ may also be a divalent acyl radical of an aliphatic C₄-C₁₀ dicarboxylic acid or of a benzene dicarboxylic acid or is a group of formula II wherein T₈ is hydrogen or C₁-C₄ alkyl and T₉ is C₁-C₁₂ alkyl or a group of formula I.

8. A composition according to claim 1 wherein the hindered amine compound is contained in an amount of 0.1 to 10 % by weight, based on resin solids.

9. A composition according to claim 1 which additionally contains a UV absorber selected from the group consisting of benzophenones, benzotriazoles, acrylic acid derivatives, aryl-s-triazines, organic nickel compounds and oxanilides.

10. A composition according to claim 9 which contains a benzotriazole UV absorber.

11. A composition according to claim 9 which contains a benzotriazole UV absorber selected from the group consisting of 2-[2-hydroxy-3,5-di(alpha,alpha-dimethylbenzyl)-phenyl]-benzotriazole, 2-(2-hydroxy-3,5-di-tert-octylphenyl]-benzotriazole, 2-(2-hydroxy-3-alpha,alpha-dimethylbenzyl-5-tert-octylphenyl)-benzotriazole, 2-(2-hydroxy-3-tert-octyl-5-alpha,alpha-dimethylbenzylphenyl)-benzotriazole, 2-(2-hydroxy-3,5-di-tert-amylphenyl)-benzotriazole, 2-[2-hydroxy-3-tert.butyl-5-(2-(omega-hydroxy-octa-(ethyleneoxy)-carbonyl)-ethylphenyl]-benzotriazole, 5-chloro-2-[2-hydroxy-3,5-di(alpha,alpha-dimethylbenzyl)-phenyl]-benzotriazole, 5-chloro-2-(2-hydroxy-3,5-di-tert-butylphenyl)-benzotriazole, 2-[2-hydroxy-3-tert-butyl-5-(2-octyloxycarbonylethyl)-phenyl]-5-chloro-benzotriazole, 2-(2-hydroxy-3-sec.dodecyl-5-methylphenyl)-benzotriazole and hexamethylene di[β-(3-tert-butyl-4-hydroxy-5-[2-benzotriazolyl]-phenyl)-propionate].

12. A composition according to claim 9 wherein the benzotriazole is 2-[2-hydroxy-3,5-di(alpha,alphadimethylbenzyl)-phenyl]-benzotriazoleand 2-[2-hydroxy-3-tert-butyl-5-(2-(omega-hydroxy-octa-(ethyleneoxy)-carbonyl)-ethylphenyl]-benzotriazole,

13. A composition according to claim 9 which additionally contains a phosphite or phosphonite antioxidant.

14. A composition according to claim 1 which additionally contains a hindered phenol antioxidant.

15. A coating composition according to claim 1, which is an ambient curable system based on an alkyd resin, thermoplastic acrylic resin, acrylic alkyd resin, polyurethane resin or polyester resin, or said resins modified with silicones, isocyanates, epoxides, isocyanurates, ketiurines or oxazolidines, or the system is based on a cellulose ester or on an epoxide resin.

16. A coating composition according to claim 1, which is an acid catalyzed thermosetting system based on a hot crosslinkable acrylic, polyester, polyurethane, polyamide or alkyd resin.

17. A composition according to claim 1 which is an enamel for industrial finishes.

18. A composition according to claim 1 which is a refinishing enamel for automobiles.

19. A compound corresponding to one of the formulae A' to M' 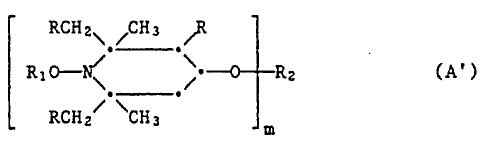 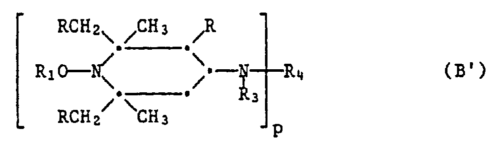 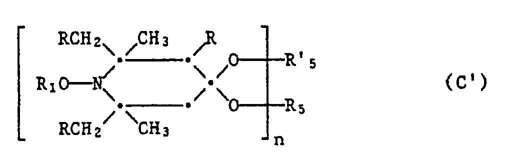 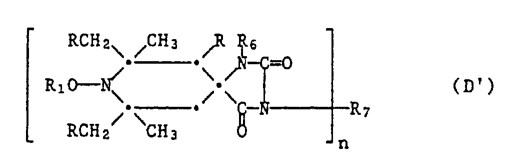 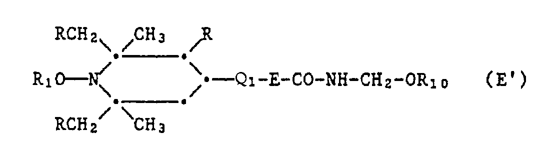 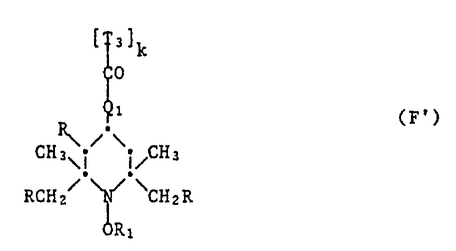 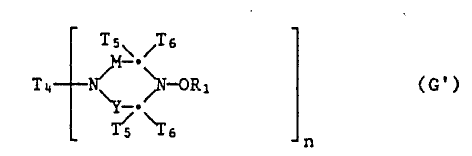 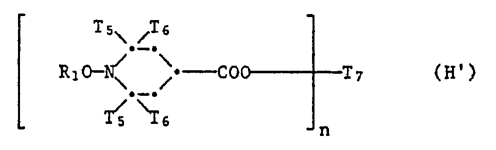 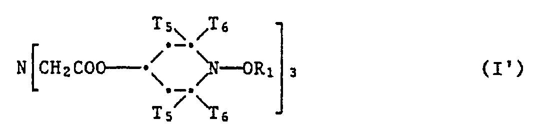 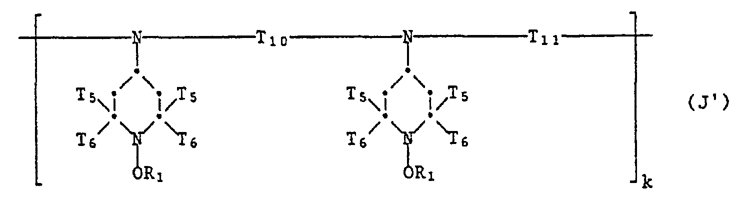 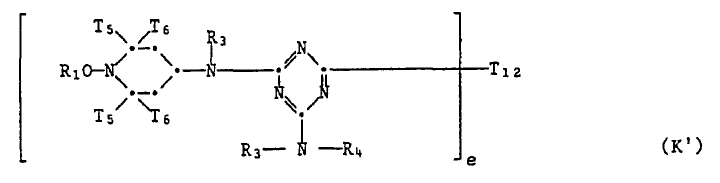 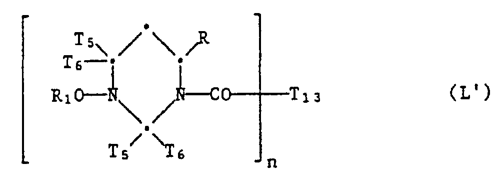 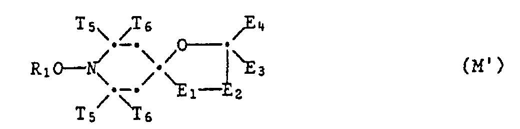 wherein
R is hydrogen or methyl,
R₁ is C₁-C₁₈ alkyl, C₂-C₁₈ alkenyl, C₂-C₁₈ alkynyl, C₅-C₈ cycloalkenyl, C₅-C₁₂ cycloalkyl, C₆-C₁₀ bicycloalkyl, C₆-C₁₀ aryl, C₇-C₉ aralkyl, or C₇-C₉ aralkyl substituted by alkyl or aryl;
m is 2-4,
when m is 2,
R₂ is C₁-C₁₂ alkylene, C₄-C₁₂ alkenylene, xylylene, a divalent acyl radical of an aliphatic, cycloaliphatic, araliphatic or aromatic dicarboxylic or dicarbamic acid having up to 20 C atoms, or is a group of formula 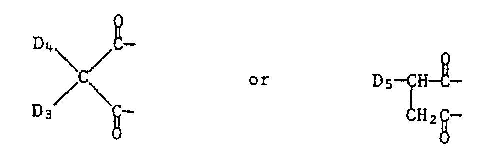 wherein D₃ and D₄ are independently hydrogen, C₁-C₆ alkyl, phenyl, benzyl or 3,5-di-t-butyl-4-hydroxybenzyl and D₅ is alkyl or alkenyl containing up to 18 carbon atoms;
when m is 3, R₂ is a trivalent acyl radical of an aliphatic, cycloaliphatic, or aromatic tricarboxylic acid having up to 12 C atomes;
when m is 4, R₂ is a tetravalent acyl radical of an aliphatic or aromatic tetracarboxylic acid having up to 18 C atomes;
p is 1, 2 or 3,
R₃ is hydrogen, C₁-C₁₂ alkyl, C₅-C₈ cycloalkyl, C₇-C₉ aralkyl, C₂-C₁₈ alkanoyl, C₃-C₅ alkenoyl or benzoyl;
when p is 1,
R₄ is hydrogen, C₁-C₁₈ alkyl, C₅-C₈ cycloalkyl, C₂-C₈ alkenyl unsubstituted or substituted by a cyano, carbonyl or carbamide group, or R₄ is C₆-C₁₀ aryl, C₇-C₉ aralkyl, glycidyl, a group of the formula -CH₂-CH(OH)-Z or -CONH-Z wherein Z is hydrogen, methyl or phenyl; or R₄ is a group of the formula 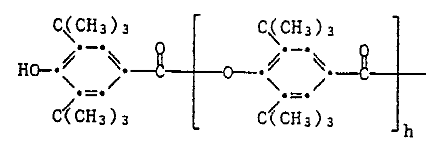 with h as 0 or 1;
or a group of the formula I 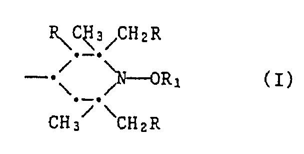 or R₃ and R₄ together are alkylene of 4 to 6 carbon atoms or 1-oxo-alkylene or the divalent acyl radical of an aliphatic or aromatic 1,2-or 1,3-dicarboxylic acid;
when p is 2,
R₄ is C₁-C₁₂ alkylene, C₆-C₁₂ arylene, xylylene, a -CH₂CH(OH)-CH₂ group, or a group -CH₂-CH(OH)-CH₂-O-X-O-CH₂-CH(OH)-CH₂- wherein X is C₂-C₁₀ alkylene, C₆-C₁₅ arylene or C₆-C₁₂ cycloalkylene; or, provided that R₃ is not alkanoyl, alkenoyl or benzoyl, R₄ can also be a divalent acyl radical of an aliphatic, cycloaliphatic or aromatic dicarboxylic acid or dicarbamic acid, or can be the group -CO-;
or R₄ is a group of formula II 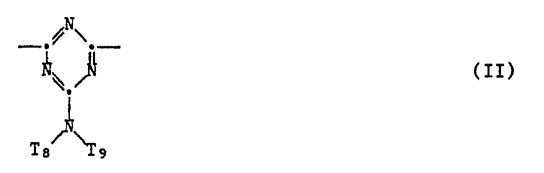 where T₈ and T₉ are independently hydrogen, alkyl of 1 to 18 carbon atoms, or T₈ and T₉ together are alkylene of 4 to 6 carbon atoms or 3-oxapentamethylene,
when p is 3,
R₄ is 2,4,6-triazinetriyl;
n is 1 or 2 and
when n is 1,
R₅ and R'₅ are independently C₁-C₁₂ alkyl, C₂-C₁₂ alkenyl, C₇-C₁₂ aralkyl, or R₅ is also hydrogen, or R₅ and R'₅ together are C₂-C₈ alkylene or hydroxyalkylene or C₄-C₂₂ acyloxyalkylene;
and when n is 2,
R₅ and R'₅ together are (-CH₂)₂C(CH₂-)₂;
R₆ is hydrogen, C₁-C₁₂ alkyl, allyl, benzyl, glycidyl or C₂-C₆ alkoxyalkyl;
when n is 1,
R₇ is hydrogen, C₁-C₁₂ alkyl, C₃-C₅ alkenyl, C₇-C₉ aralkyl, C₅-C₇ cycloalkyl, C₂-C₄ hydroxyalkyl, C₂-C₆ alkoxyalkyl, C₆-C₁₀ aryl, glycidyl, a group of the formula -(CH₂)ₜ-COO-Q or of the formula -(CH₂)ₜ-O-CO-Q wherein t is 1 or 2, and Q is C₁-C₄ alkyl or phenyl;
and when n is 2,
R₇ is C₂-C₁₂ alkylene, C₆-C₁₂ arylene, a group -CH₂CH(OH)-CH₂-O-X-O-CH₂--CH(OH)-CH₂- wherein X is C₂-C₁₀ alkylene, C₆-C₁₅ arylene or C₆-C₁₂ cycloalkylene, or a group -CH₂CH(OZ')CH₂-(OCH₂-CH-(OZ')CH₂)₂- wherein Z' is hydrogen, C₁-C₁₈ alkyl, allyl, benzyl, C₂-C₁₂ alkanoyl or benzoyl;
Q₁ is -N(R₈)- or -O-;
E is C₁-C₃ alkylene, the group -CH₂-CH(R₉)-O- wherein R₉ is hydrogen, methyl or phenyl, or E is the group -(CH₂)₃-NH- or a direct bond;
R₁₀ is hydrogen or C₁-C₁₈ alkyl,
R₈ is hydrogen, C₁-C₁₈ alkyl, C₅-C₇ cycloalkyl, C₇-C₁₂ aralkyl, cyanoethyl, C₆-C₁₀ aryl, the group -CH₂-CH(R₉)-OH wherein R₉ has the meaning defined above; or R₈ is a group of the formula I
or a group of the formula 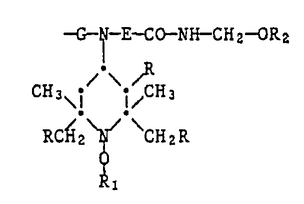 wherein G is C₂-C₆ alkylene or C₆-C₁₂ arylene; or R₈ is a group -E-CO-NH-CH₂-OR₁₀;
T₃ is ethylene or 1,2-propylene, or is the repeating structural unit derived from an alpha-olefin copolymer with an alkyl acrylate or methacrylate;
k is 2 to 100;
T₄ has the same meaning as R₄ when p is 1 or 2,
T₅ is methyl,
T₆ is methyl or ethyl, or T₅ and T₆ together are tetramethylene or pentamethylene,
M and Y are independently methylene or carbonyl;
T₇ is the same as R₇,
T₁₀ and T₁₁ are independently alkylene of 2 to 12 carbon atoms, or T₁₁ is a group of formula II,
e is 2, 3 or 4,
T₁₂ is a group -N(R⁴)-CH₂)_{d}-N(R⁴)-
or 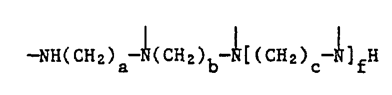 where a, b and c are independently 2 or 3, d is 2-10 and f is 0 or 1,
when n is 1, T₁₃ is C₂-C₁₈ alkyl, C₂-C₁₈ alkenyl, C₆-C₈ cycloalkyl, C₆-C₁₂ aryl or phenyl substituted by C₁-C₄ alkyl, hydroxy or halogen, and when n is 2, T₁₃ has the same meaning as R₂;
E₁ and E₂, being different, each are -CO- or -N(E₅)-, where E₅ is hydrogen, C₁-C₁₂ alkyl or alkoxycarbonylalkyl of 4 to 22 carbon atoms;
E₃ is hydrogen, alkyl of 1 to 30 carbon atoms, phenyl, naphthyl, said phenyl or said naphthyl substituted by chlorine or by alkyl of 1 to 4 carbon atoms, or phenylalkyl of 7 to 12 carbon atoms, or said phenylalkyl substituted by alkyl of 1 to 4 carbon atoms, and
E₄ is hydrogen, alkyl of 1 to 30 carbon atoms, phenyl, naphthyl or phenylalkyl of 7 to 12 carbon atoms, or
E₃ and E₄ together are polymethylene of 4 to 17 carbon atoms, or said polymethylene substituted by up to four alkyl groups of 1 to 4 carbon atoms, provided that in formula B' R₃ or R₄ are not hydrogen if R₁ is alkyl.

20. A compound of claim 19 of formula (A') to (F') wherein R is hydrogen.

21. A compound of claim 19 of formula (G') to (M') wherein T₅ and T₆ are methyl.

22. A compound of claim 19 of formula (A') to (M') wherein R is hydrogen, R₁ is C₁-C₁₈ alkyl, C₂-C₆ alkenyl, C₅-C₈ cycloalkyl, cyclohexyl, phenyl or C₇-C₉ aralkyl; m is 2-4 and when m is 2, R₂ is C₂-C₈ alkylene, C₄-C₈ alkenylene, xylylene, a divalent acyl radical of an aliphatic, cycloaliphatic or aromatic dicarboxylic acid having up to 12 carbon atoms or of an aliphatic or aromatic dicarbamic acid having up to 12 carbon atoms, or is a group of formula 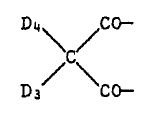 wherein D₃ and D₄ are independently hydrogen, C₁-C₆ alkyl, benzyl or 3,5-di-t-butyl-4-hydroxybenzyl, and when m is 3, R₂ is a trivalent acyl radical of an aliphatic or aromatic tricarboxylic acid having up to 12 carbon atoms, and when m is 4, R₂ is a tetravalent acyl radical of an aliphatic or aromatic tetracarboxylic acid having up to 12 carbon atoms;
p is 1, 2 or 3, R₃ is hydrogen, C₁-C₁₂ alkyl, C₅-C₈ cycloalkyl, C₇-C₉ aralkyl, C₂-C₁₈ alkanoyl or benzoyl, and when p is 1, R₄ is hydrogen, C₁-C₁₈ alkyl, C₅-C₈ cycloalkyl, phenyl, benzyl or a group of the formula 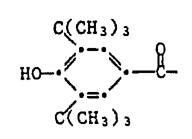 or of the formula 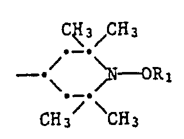 and when p is 2, R₄ is C₂-C₁₂ alkylene, C₆-C₁₂ arylene, xylylene, or provided that R₃ is not alkanoyl or benzoyl, R₄ can also be a divalent acyl radical of an aliphatic or aromatic dicarboxylic acid having up to 12 carbon atoms, of an aliphatic or aromatic dicarbamic acid having up to 12 carbon atoms, or can be a group of formula II wherein T₈ and T₉ are independently hydrogen, or C₁-C₁₂ alkyl or T₈ and T₉ together are C₄-C₆ alkylene or 3-oxapentamethylene, and when p is 3, R₄ is 2,4,6-triazinetriyl; n is 1 or 2, and when n is 1, R₅ and R'₅ are C₁-C₁₂ alkyl or benzyl, or R₅ and R'₅ together are C₂-C₈ alkylene or hydroxyalkylene and when n is 2, R₅ and R'₅ together are (-CH₂)₂C(CH₂-)₂;
R₆ is hydrogen, C₁-C₁₂ alkyl, allyl or benzyl, and when n is 1, R₇ is hydrogen, C₁-C₁₂ alkyl, allyl, benzyl, cyclohexyl, 2-hydroxyethyl or a group of the formula -CH₂CH₂-COOQ wherein Q is C₁-C₄ alkyl, and when n is 2, R₇ is C₂-C₁₂ alkylene or C₆-C₁₂ arylene;
Q₁ is -N(R₈)- or -O-; E is C₁-C₃ alkylene or a direct bond; R₁₀ is hydrogen or C₁-C₁₄ alkyl, R₈ is hydrogen, C₁-C₁₂ alkyl, cyclohexyl, benzyl, cyanoethyl or a group 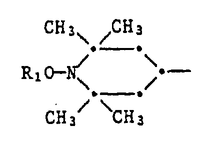 T₃ is ethylene or 1,2-propylene, k is 2 to 100;
T₄ has the same meaning as R₄ when p is 1 or 2,
T₅ and T₆ are methyl, M and Y are independently -CH₂- or -CO-;
T₇ is the same as R₇;
T₁₀ and T₁₁ are independently C₂-C₈ alkylene or T₁₁ is a group of formula I,
e is 3 or 4,
T₁₂ is a group 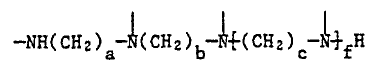 wherein a, b and c are independently 2 or 3, and f is 0 or 1;
when n is 1, T₁₃ is C₂-C₁₈ alkyl, cyclohexyl or phenyl and when n is 2,
T₁₃ has the same meaning as R₂;
E₁ is -CO- and E₂ is -N(E₅)-, wherein E₅ is hydrogen, C₁-C₁₂ alkyl or C₄-C₁₈ alkoxycarbonylalkyl, E₃ and E₄ are independently C₁-C₁₂ alkyl or phenyl or E₃ and E₄ together are C₄-C₁₂ polymethylene.

23. A compound of claim 19 of formula (A'), (B'), (C'), (J'), (K') or (M') wherein R is hydrogen, R₁ is C₁-C₁₈ alkyl, cyclohexyl, cyclohexenyl, methylcyclohexyl or C₇-C₉ phenylalkyl;
m is 2, R₂ is C₂-C₈ alkylene, xylylene or a group -CO-R₁₁-CO-, wherein R₁₁ is C₂-C₈ alkylene, cyclohexylene or phenylene or a group 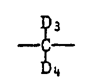 wherein D₃ and D₄ are independently hydrogen, C₁-C₄ alkyl, benzyl or 3,5-di-t-butyl-4-hydroxybenzyl;
p is 1 or 2, R₃ is hydrogen, C₁-C₁₂ alkyl or C₂-C₈ alkanoyl, and when p is 1, R₄ is hydrogen, C₁-C₁₂ alkyl, and when p is 2, R₄ is C₂-C₈ alkylene or is -CO-R₁₁-CO-;
n is 1 or 2, and when n is 1, R₅ and R'₅ together are C₂-C₈ alkylene, and when n is 2, R₅ and R'₅ together are (-CH₂)₂C(CH₂-)₂;
k is 5-20, T₁₀ is C₂-C₈ alkylene and T₁₁ is a group of formula II,
wherein T₈ and T₉ are independently hydrogen or C₁-C₁₂ alkyl or T₈ and T₉ together are pentamethylene or 3-oxapentamethylene, T₅ and T₆ are methyl;
e is 4, T₁₂ is a group 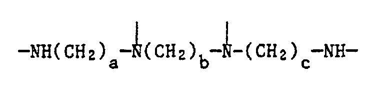 wherein a, b and c independently are 2 or 3;
E₁ is -CO- and E₂, is -N(E₅)-, wherein E₅ is hydrogen, C₁-C₁₂ alkyl or C₄-C₁₅ alkoxycarbonylalkyl, and E₃ and E₄ are independently C₁-C₁₂ alkyl or E₃ and E₄ together are C₅-C₁₂ polymethylene.

24. Di-(1-methoxy-2,2,6,6-tetramethylpiperidin-4-yl) sebacate according to claim 19.

25. Di-(1-cyclohexyloxy-2,2,6,6-tetramethylpiperidin-4-yl) sebacate according to claim 19.

26. Di-(1-cyclohexyloxy-2,2,6,6-tetramethylpiperidin-4-yl) isophthalate according to claim 19.

27. Di-(1-octyloxy-2,2,6,6-tetramethylpiperidin-4-yl) sebacate according to claim 19.

28. 3,15-Dicyclohexyloxy-2,2,4,4,14,14,16,16-octamethyl-7,11,18,21-tetraoxa-3,15-diazatrispiro-[5.2.2.5.2.2.]-heneicosane according to claim 19.

29. Di-(1-cyclohexyloxy-2,2,6,6-tetramethylpiperidin-4-yl) succinate according to claim 19.

30. The use of a compound of claim 19 as stabilizer for organic materials.

31. The use according to claim 30 as stabilizer for polymers.

32. The use according to claim 30 as stabilizer for photographic layers.

33. Organic material containing an effective amount of a compound of claim 19.

## Claims (Claims for the following Contracting State(s): ES)

1. A stabilized ambient curing or acid catalyzed thermosetting coating composition containing an effective stabilizing amount of a hindered amine compound containing the group 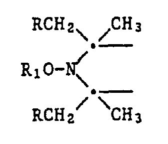 wherein R is hydrogen or methyl and R₁ is C₁-C₁₈ alkyl, C₂-C₁₈ alkenyl, C₂-C₁₈ alkinyl, C₅-C₁₂ cycloalkyl, C₆-C₁₀ bicycloalkyl, C₅-C₈ cycloalkenyl, C₆-C₁₀ aryl, C₇-C₉ aralkyl or C₇-C₉ aralkyl substituted by C₁-C₄ alkyl or phenyl.

2. The composition according to claim 1 which contains a hindered amine compound corresponding to one of formulae A-N 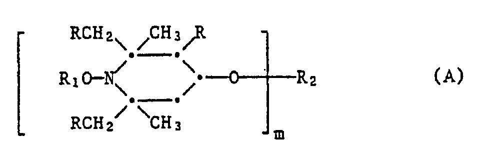 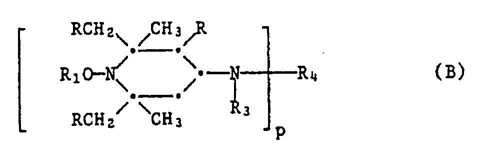 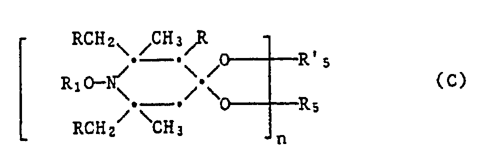 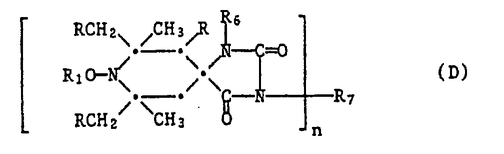 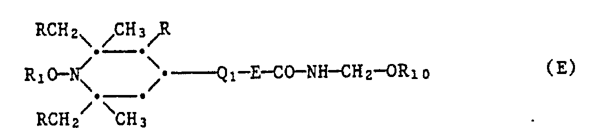 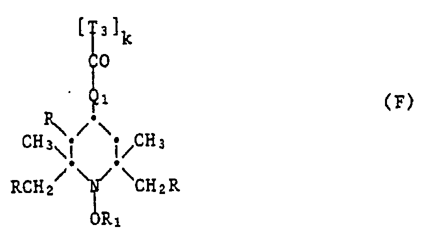 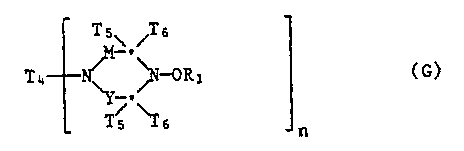 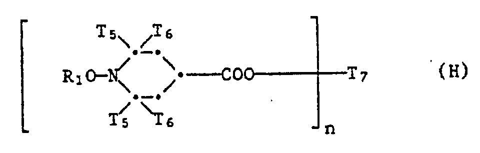 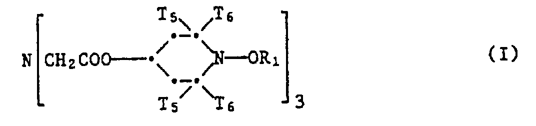 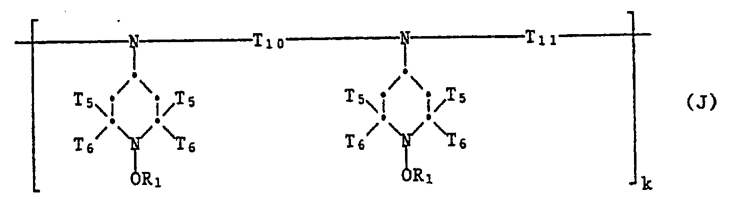 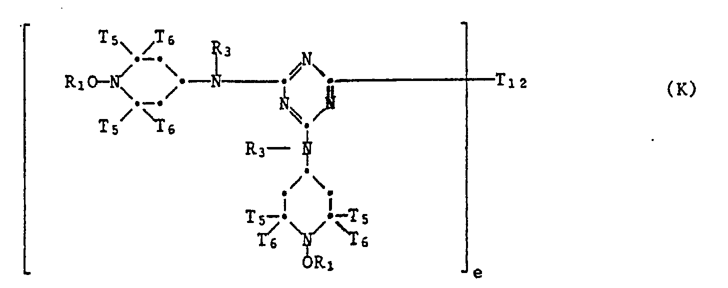 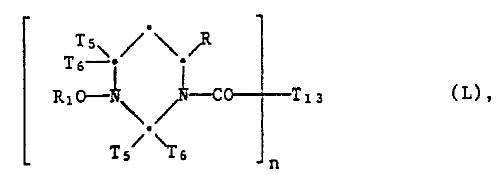 or 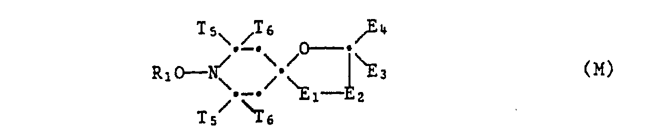 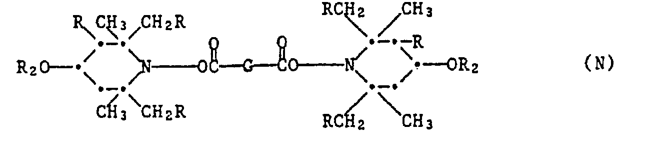 wherein
R is hydrogen or methyl,
R₁ is C₁-C₁₈ alkyl, C₂-C₁₈ alkenyl, C₂-C₁₈ alkinyl, C₅-C₁₂ cycloalkyl, C₆-C₁₀ bicycloalkyl, C₅-C₈ cycloalkenyl, C₆-C₁₀ aryl, C₇-C₉ aralkyl or C₇-C₉ aralkyl substituted by C₁-C₄ alkyl or phenyl;
m is 1-4,
when m is 1,
R₂ is hydrogen, C₁-C₁₈ alkyl optionally interrrupted by one or more oxygen atoms, C₂-C₁₂ alkenyl, C₆-C₁₀ aryl, C₇-C₁₈ aralkyl, glycidyl, a monovalent acyl radical of an aliphatic, cycloaliphatic, araliphatic or aromatic carboxylic acid, or of a carbamic acid or R₂ is a group 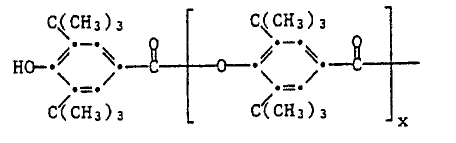 wherein x is 0 or 1, or is a group 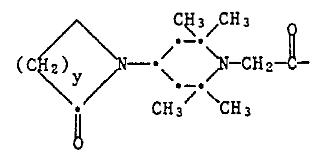 wherein y is 2-4;
when m is 2,
R₂ is C₁-C₁₂ alkylene, C₄-C₁₂ alkenylene, xylylene, a divalent acyl radical of an aliphatic, cycloaliphatic, araliphatic or aromatic dicarboxylic acid or of a dicarbamic acid, or is a group 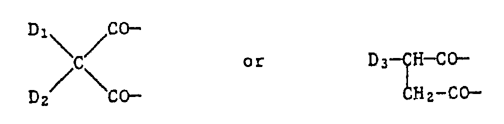 wherein D₁ and D₂ independently are hydrogen; alkyl containing up to 8 carbon atoms, phenyl, benzyl or 3,5-di-t-butyl-4-hydroxybenzyl and D₃ is an alkyl or alkenyl radical containing up to 18 carbon atoms; when m is 3, R₂ is a triavalent acyl radical of an aliphatic, cycloaliphatic, or aromatic tricarboxylic acid;
when m is 4, R₂ is a tetravalent acyl radical of an aliphatic or aromatic tetracarboxylic acid;
p is 1, 2 oder 3,
R₃ is hydrogen, C₁-C₁₂ alkyl, C₅-C₇ cycloalkyl, C₇-C₉ aralkyl, C₂-C₁₈ alkanoyl, C₃-C₅ alkenoyl or benzoyl;
when p is 1,
R₄ is hydrogen, C₁-C₁₈ alkyl, C₅-C₇ cycloalkyl, C₂-C₈ alkenyl unsubstituted or substituted by a cyano, carbonyl or carbamide group, or it is aryl, aralkyl, glycidyl, a group of the formula -CH₂-CH(OH)-Z or -CONH-Z wherein Z is hydrogen, methyl or phenyl; or R₄ is a group of formula I 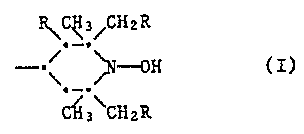 or a group of formula 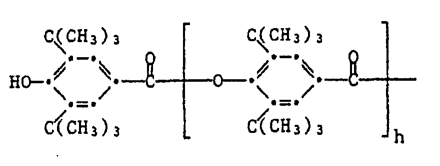 with h as 0 or 1;
or R₃ and R₄ together are alkylene of 4 to 6 carbon atoms or 1-oxo-alkylene or the divalent acyl radical of an aliphatic or aromatic 1,2- or 1,3-dicarboxylic acid;
when p is 2,
R₄ is C₂-C₁₂ alkylene, C₆-C₁₂ arylene, xylylene, a -CH₂CH(OH)-CH₂- group, or a group -CH₂-CH(OH)-CH₂-O-X-O-CH₂-CH(OH)-CH₂- wherein X is C₂-C₁₀ alkylene, C₆-C₁₅ arylene or C₆-C₁₂ cycloalkylene; or, provided that R₃ is not alkanoyl, alkenoyl or benzoyl, R₄ can also be a divalent acyl radical of an aliphatic, cycloaliphatic or aromatic dicarboxylic acid or dicarbamic acid, or can be the group -CO-; or R₄ is a group of formula II 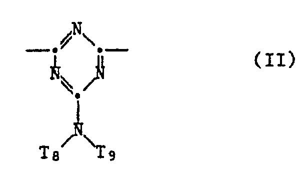 where T₈ and T₉ are independently hydrogen, alkyl of 1 to 18 carbon atoms or a group of formula I, or T₈ and T₉ together are alkylene of 4 to 6 carbon atoms or 3-oxapentamethylene;
when p is 3,
R₄ is 2,4,6-triazinetriyl,
n is 1 or 2 and
when n is 1,
R₅ and R'₅ are independently C₁-C₁₂ alkyl, C₂-C₁₂ alkenyl, C₇-C₁₂ aralkyl, or R₅ is also hydrogen, or R₅ and R'₅ together are C₂-C₈ alkylene or hydroxyalkylene or C₄-C₂₂ acyloxyalkylene;
when n is 2,
R₅ and R'₅ together are (-CH₂)₂C(CH₂-)₂;
R₆ is hydrogen, C₁-C₁₂ alkyl, allyl, benzyl, glycidyl or C₂-C₆ alkoxyalkyl;
when n is 1,
R₇ is hydrogen, C₁-C₁₂ alkyl, C₃-C₅ alkenyl, C₇-C₉ aralkyl, C₅-C₇ cycloalkyl, C₂-C₄ hydroxyalkyl, C₂-C₆ alkoxyalkyl, C₆-C₁₀ aryl, glycidyl, a group of the formula -(CH₂)ₜ-COO-Q or of the formula -(CH₂)ₜ-O-CO-Q wherein t is 1 or 2, and Q is C₁-C₄ alkyl or phenyl; or
when n is 2,
R₇ is C₂-C₁₂ alkylene, C₆-C₁₂ arylene, a group -CH₂CH(OH)-CH₂-O-X-O-CH₂-CH(OH)-CH₂- wherein X is C₂-C₁₀ alkylene, C₆-C₁₅ arylene or C₆-C₁₂ cycloalkylene, or a group -CH₂CH(OZ')CH₂-(OCH₂-CH-(OZ')CH₂)₂- wherein Z' is hydrogen, C₁-C₁₈ alkyl, allyl, benzyl, C₂-C₁₂ alkanoyl or benzoyl;
Q₁ is -N(R₈)- or -O-;
E is C₁-C₃ alkylene, the group -CH₂-CH(R₉)-O- wherein R₉ is hydrogen, methyl or phenyl, or E is the group -(CH₂)₃-NH- or a direct bond;
R₁₀ is hydrogen or C₁-C₁₈ alkyl;
R₈ is hydrogen, C₁-C₁₈ alkyl, C₅-C₇ cycloalkyl, C₇-C₁₂ aralkyl, cyanoethyl, C₆-C₁₀ aryl, the group -CH₂-CH(R₉)-OH wherein R₉ has the meaning defined above, a group of the formula I or a group of the formula 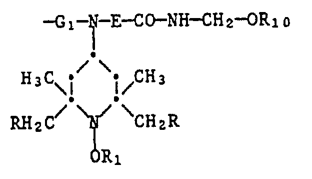 wherein G₁ can be C₂-C₆ alkylene or C₆-C₁₂ arylene, or R₈ is a group -E-CO-NH-CH₂-OR₁₀;
T₃ is ethylene or 1,2-propylene, or is the repeating structural unit derived from an alpha-olefin copolymer with an alkyl acrylate or methacrylate;
k is 2 to 100;
T₄ has the same meaning as R₄ when p is 1 or 2,
T₅ is methyl,
T₆ is methyl or ethyl, or T₅ and T₆ together are tetramethylene or pentamethylene;
M and Y are independently methylene or carbonyl;
T₇ is the same as R₇;
T₁₀ and T₁₁ are independently alkylene of 2 to 12 carbon atoms, or T₁₁ is a group of formula II;
e is 2, 3 or 4 and
T₁₂ is a group of formula -N(R₅)-(CH₂)_{d}-N(R₅)-
or 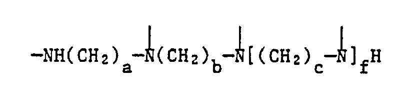 where a, b and c are independently 2 or 3, d is 2 to 10 and f is 0 or 1,
T₁₃ is the same as R₄ with the proviso that T₁₃ cannot be hydrogen when n is 1;
E₁ and E₂, being different, each are -CO- or -N(E₅)- where E₅ is hydrogen, C₁-C₁₂ alkyl or C₄-C₂₂-alkoxycarbonylalkyl;
E₃ is hydrogen, alkyl of 1 to 30 carbon atoms, phenyl, naphthyl, said phenyl or said naphthyl substituted by chlorine or by alkyl of 1 to 4 carbon atoms, or phenylalkyl of 7 to 12 carbon atoms, or said phenylalkyl substituted by alkyl of 1 to 4 carbon atoms;
E₄ is hydrogen, alkyl of 1 to 30 carbon atoms, phenyl, naphthyl or phenylalkyl of 7 to 12 carbon atoms, or
E₃ and E₄ together are polymethylene of 4 to 17 carbon atoms, or said polymethylene substituted by up to four alkyl groups of 1 to 4 carbon atoms;
R₂ of formula (N) is as previously defined when m is 1; and
G is a direct bond, C₁-C₁₂ alkylene, phenylene or -NH-G'-NH wherein G' is C₁-C₁₂ alkylene.

3. A composition according to claim 2 which contains a compound of formula A, B, C, D, J, K or M wherein R is hydrogen and T₅ and T₆ are methyl.

4. A composition according to claim 2 which contains a compound of formula A, B, C, J or K wherein R is hydrogen and R₁ is C₁-C₁₈ alkyl, C₆-C₁₂ cycloalkyl, cyclohexenyl or C₇-C₉ phenylalkyl.

5. A composition according to claim 2 which contains a compound of formula A wherein R is hydrogen and R₁ is C₁-C₁₈ alkyl, C₆-C₁₂ cycloalkyl, cyclohexenyl or C₇-C₉ phenylalkyl, m is 1, 2 or 4 and when m is 1, R₂ is C₁-C₁₂ alkyl, allyl, benzyl or an acyl radical of an aliphatic C₂-C₁₈ carboxylic acid, of a cycloaliphatic C₆-C₁₂ carboxylic acid or of an aromatic C₇-C₁₅ carboxylic acid, and when m is 2, R₂ is C₁-C₈ alkylene, butylene, xylylene or is a divalent acyl radical of an aliphatic C₂-C₁₈ dicarboxylic acid cycloaliphaticor of a cycloaliphatic or aromatic C₈-C₁₄ dicarboxylic acid, or of an aliphatic, cycloaliphatic or aromatic C₈-C₁₄ dicarbamic acid, or R₂ is a group 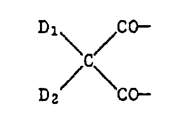 wherein D₁ C₁-C₈ alkyl or 3,5-di-tert.butyl-4-hydroxybenzyl and D₂ is D₁ or hydrogen and when m is 4, R₂ is a tetravalent acyl radical of a butane- or pentanetetracarboxylic acid.

6. A composition according to claim 5 which contains a compound of formula A wherein R is hydrogen, R₁ is C₁-C₁₀ alkyl, cycloalkyl, cyclohexyl or C₇-C₉ phenylalkyl, m is 1 or 2, and when m is 1, R₂ is benzyl, C₂-C₁₈ alkanoyl, benzoyl, or 3,5-di-tert.butyl-4-hydroxybenzoyl, and when m is 2, R₂ is xylylene or a divalent acyl radical of an aliphatic C₄-C₁₀ dicarboxylic acid or of a benzene dicarboxylic acid.

7. A composition according to claim 2 which contains a compound of formula B wherein R is hydrogen and R₁ is C₁-C₁₈ alkyl, C₆-C₁₂ cycloalkyl, cyclohexenyl or C₇-C₉ phenylalkyl, p is 1 or 2, R₃ is hydrogen, C₁-C₁₂ alkyl or C₂-C₁₂ alkanoyl, allyl, and when p is 1, R₄ is hydrogen, C₁-C₁₂ alkyl or a group of formula I, and when p is 2, R₄ is C₂-C₈ alkylene or xylylene and if R₃ is not alkanoyl, R₄ may also be a divalent acyl radical of an aliphatic C₄-C₁₀ dicarboxylic acid or of a benzene dicarboxylic acid or is a group of formula II wherein T₈ is hydrogen or C₁-C₄ alkyl and T₉ is C₁-C₁₂ alkyl or a group of formula I.

8. A composition according to claim 1 wherein the hindered amine compound is contained in an amount of 0.1 to 10 % by weight, based on resin solids.

9. A composition according to claim 1 which additionally contains a UV absorber selected from the group consisting of benzophenones, benzotriazoles, acrylic acid derivatives, aryl-s-triazines, organic nickel compounds and oxanilides.

10. A composition according to claim 9 which contains a benzotriazole UV absorber.

11. A composition according to claim 9 which contains a benzotriazole UV absorber selected from the group consisting of 2-[2-hydroxy-3,5-di(alpha,alpha-dimethylbenzyl)-phenyl]-benzotriazole, 2-(2-hydroxy-3,5-di-tert-octylphenyl)-benzotriazole, 2-(2-hydroxy-3-alpha,alphadimethylbenzyl-5-tert-octylphenyl)-benzotriazole, 2-(2-hydroxy-3-tart-octyl-5-alpha,alpha-dimethylbenzylphenyl)-benzotriazole, 2-(2-hydroxy-3,5-di-tert-amylphenyl)-benzotriazole, 2-[2-hydroxy-3-tert.butyl-5-(2-(omega-hydroxy-octa-(ethyleneoxy)-carbonyl)-ethylphenyl]-benzotriazole, 5-chloro-2-[2-hydroxy-3,5-di(alpha,alpha-dimethylbenzyl)-phenyl]-benzotriazole, 5-chloro-2-(2-hydroxy-3,5-di-tert-butylphenyl)-benzotriazole, 2-[2-hydroxy-3-tert-butyl-5-(2-octyloxycarbonylethyl)-phenyl]-5-chloro-benzotriazole, 2-(2-hydroxy-3-sec.dodecyl-5-methylphenyl)-benzotriazole and hexamethylene di[β-(3-tert-butyl-4-hydroxy-5-[2-benzotriazolyl]-phenyl)-propionate].

12. A composition according to claim 9 wherein the benzotriazole is 2-[2-hydroxy-3,5-di(alpha,alpha-dimethylbenzyl)-phenyl]-benzotriazoleand 2-[2-hydroxy-3-tert-butyl-5-(2-(omega-hydroxy-octa-(ethyleneoxy)-carbonyl)-ethylphenyl]-benzotriazole,

13. A composition according to claim 9 which additionally contains a phosphite or phosphonite antioxidant.

14. A composition according to claim 1 which additionally contains a hindered phenol antioxidant.

15. A coating composition according to claim 1, which is an ambient curable system based on an alkyd resin, thermoplastic acrylic resin, acrylic alkyd resin, polyurethane resin or polyester resin, or said resins modified with silicones, isocyanates, epoxides, isocyanurates, ketiurines or oxazolidines, or the system is based on a cellulose ester or on an epoxide resin.

16. A coating composition according to claim 1, which is an acid catalyzed thermosettingh system based on a hot crosslinkable acrylic, polyester, polyurethane, polyamide or alkyd resin.

17. A composition according to claim 1 which is an enamel for industrial finishes.

18. A composition according to claim 1 which is a refinishing enamel for automobiles.

## Revendications (Revendications pour l'(les) Etat(s) contractant(s) suivant(s): BE ,DE,FR,GB,IT,NL,SE)

1. Composition stabilisée de revêtement durcissante à la température ambiante ou catalysée par acide, contenant une quantité stabilisante efficace d'un composé d'amine encombrée contenant le groupe 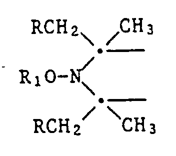 dans lequel R représente l'hydrogène ou le méthyle et R₁ représente les alkyle en C₁-C₁₈, alcényle en C₂-C₁₈, alcynyle en C₂-C₁₈, cycloalkyle en C₅-C₁₂, bicycloalkyle en C₆-C₁₀, cycloalcényle en C₅-C₈, aryle en C₆-C₁₀, aralkyle en C₇-C₉ ou aralkyle en C₇-C₉ substitué par un alkyle en C₁-C₄ ou le phényle.

2. Composition selon la revendication 1, qui contient un composé d'amine encombrée correspondant à l'une des formules A à N 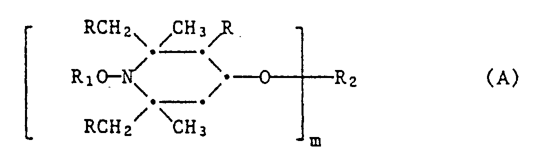 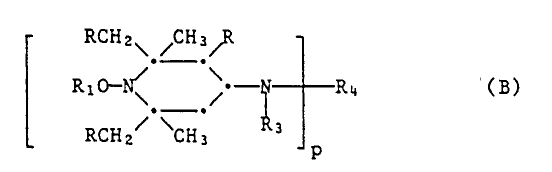 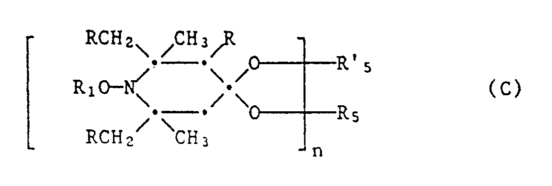 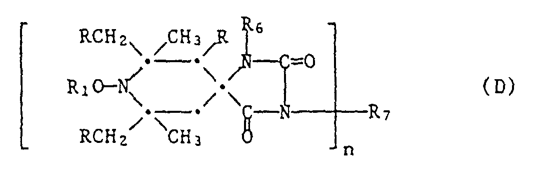 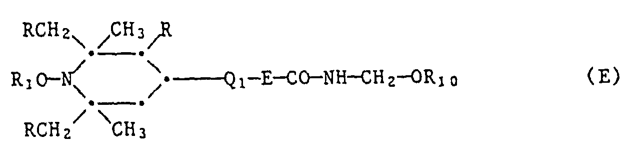 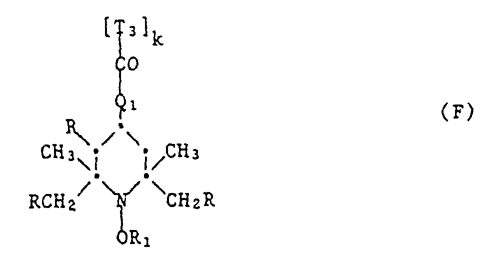 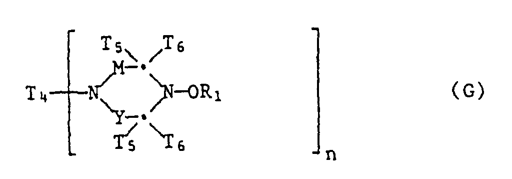 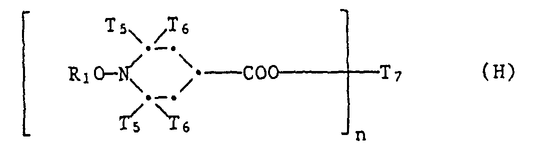 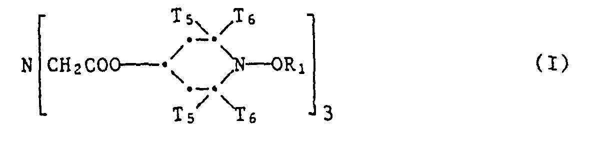 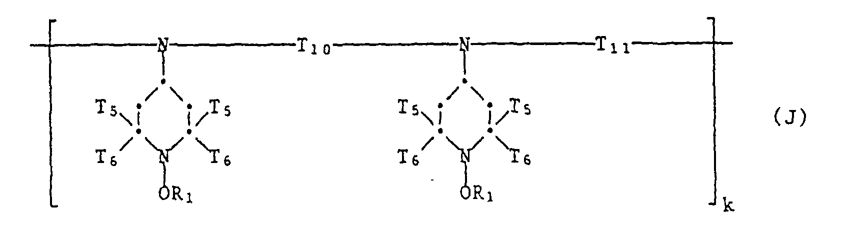 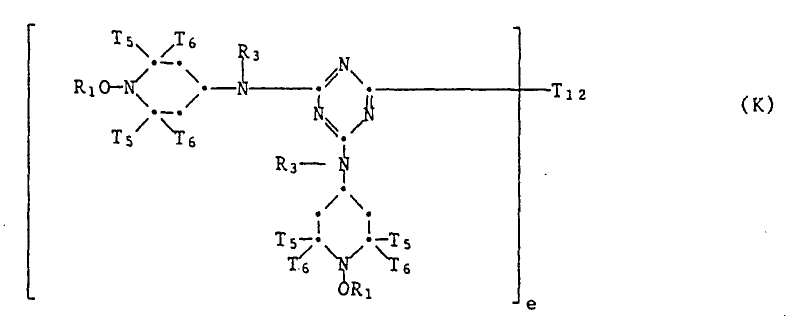 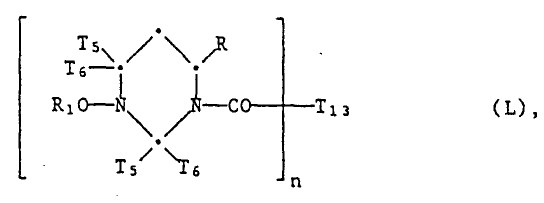 ou 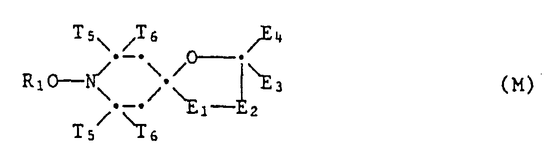 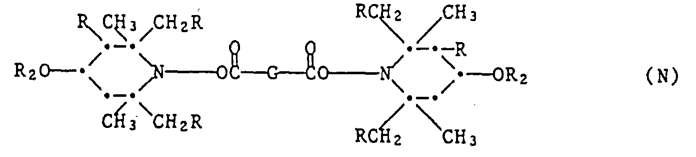 dans lesquelles
R représente l'hydrogène ou le méthyle,
R₁ représente les alkyle en C₁-C₁₈, alcényle en C₂-C₁₈, alcynyle en C₂-C₁₈, cycloalkyle en C₅-C₁₂, bicycloalkyle en C₆-C₁₀, cycloalcényle en C₅-C₈, aryle en C₆-C₁₀, aralkyle en C₇-C₉ ou aralkyle en C₇-C₉ substitué par un alkyle en C₁-C₄ ou le phényle ;
m est 1 à 4,
lorsque m est 1,
R₂ représente l'hydrogène, les alkyles en C₁-C₁₈ pouvant être éventuellement interrompus par un ou plusieurs atomes d'oxygène, les alcényle en C₂-C₁₂, aryle en C₆-C₁₀, aralkyle en C₇-C₁₈, glycidyle, un radical acyle monovalent d'un acide carboxylique aliphatique, cycloaliphatique, araliphatique ou aromatique, ou d'un acide carbamique ; ou R₂ représente un groupe 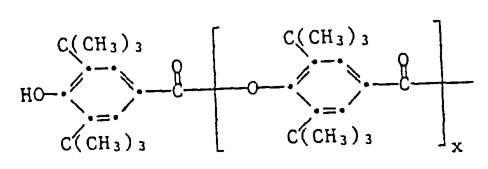 où x est 0 ou 1, ou représente un groupe 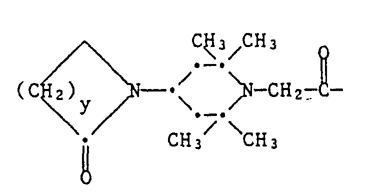 où y est de 2 à 4 ;
lorsque m est 2,
R₂ représente les alkylène en C₁-C₁₂, alcénylène en C₄-C₁₂, xylylène, un radical acyle bivalent d'un acide dicarbamique ou d'un acide carboxylique aliphatique, cycloaliphatique, araliphatique ou aromatique ; ou R₂ représente un groupe 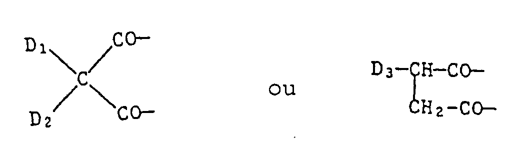 où D₁ et D₂ sont indépendamment l'un de l'autre l'hydrogène, les alkyle contenant jusqu'à 8 atomes de carbone, phényle, benzyle ou 3,5-di-t-butyl-4-hydroxybenzyle et D₃ représente un radical alkyle ou alcényle contenant jusqu'à 18 atomes de carbone ;
lorsque m est 3, R₂ représente un radical acyle trivalent d'un acide tricarboxylique aliphatique, cycloaliphatique ou aromatique ;
lorsque m est 4, R₂ représente un radical acyle tétravalent d'un acide tétracarboxylique aliphatique ou aromatique ;
p est 1, 2 ou 3,
R₃ représente l'hydrogène, les alkyle en C₁-C₁₂, cycloalkyle en C₅-C₇, aralkyle en C₇-C₉, alcanoyle en C₂-C₁₈, alcénoyle en C₃-C₅ ou benzoyle ;
lorsque p est 1,
R₄ représente l'hydrogène, les alkyle en C₁-C₁₈, cycloalkyle en C₅-C₇, alcényle en C₂-C₈ non-substitué ou substitué par un groupe cyano, carbonyle ou carbamide, ou représente les aryle, aralkyle, glycidyle, un groupe de formule -CH₂-CH(OH)-Z ou -CONH-Z dans lesquelles Z représente l'hydrogène, le méthyle ou le phényle ; ou R₄ représente un groupe de formule I 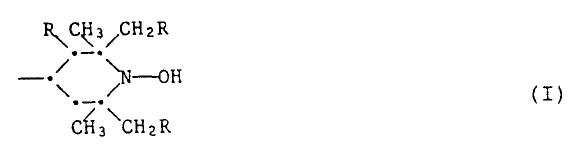 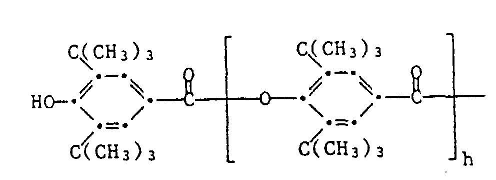 avec h étant 0 ou 1 ;
ou R₃ et R₄ ensemble, représentent un alkylène avec 4 à 6 atomes de carbone ou un 1-oxo-alkylène ou le radical acyle bivalent d'un acide 1,2- ou 1,3-dicarboxylique aliphatique ou aromatique,
lorsque p est 2,
R₄ représente les alkylène en C₂-C₁₂, arylène en C₆-C₁₂, xylylène, un groupe -CH₂CH(OH)-CH₂-, ou un groupe -CH₂-CH(OH)-CH₂-O-X-O-CH₂-CH(OH)-CH₂- où X représente les alkylène en C₂-C₁₀, arylène en C₆-C₁₅ ou cycloalkylène en C₆-C₁₂ ; ou à la condition que R₃ ne représente pas un alcanoyle, alcénoyle ou benzoyle, R₄ peut aussi représenter un radical acyle bivalent d'un acide dicarbamique ou dicarboxylique aliphatique, cycloaliphatique ou aromatique ou peut représenter le groupe -CO- ; ou R₄ représente un groupe de formule II 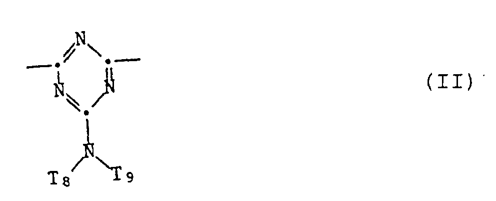 où T₈ et T₉ indépendamment l'un de l'autre représentent l'hydrogène, un alkyle avec 1 à 18 atomes de carbone ou un groupe de formule I, ou T₈ et T₉ ensemble, représentent un alkylène avec 4 à 6 atomes de carbone ou le 3-oxapentaméthylène ;
lorsque p est 3,
R₄ représente le 2,4,6-triazinetriyle,
n est 1 ou 2 et
lorsque n est 1,
R₅ et R'₅ indépendamment l'un de l'autre représentent les alkyle en C₁-C₁₂, alcényle en C₂-C₁₂, aralkyle en C₇-C₁₂ ou R₅ représente aussi l'hydrogène, ou R₅ et R'₅ ensemble représentent les hydroxyalkylène ou alkylène en C₂-C₈ ou acyloxyalkylène en C₄-C₂₂
lorsque n est 2,
R₅ et R'₅ ensemble représentent (-CH₂)₂C(CH₂-)₂ ;
R₆ représente l'hydrogène, les alkyle en C₁-C₁₂, allyle, benzyle, glycidyle ou alcoxyalkyle en C₂-C₆ ;
lorsque n est 1,
R₇ représente l'hydrogène, les alkyle en C₁-C₁₂, alcényle en C₃-C₅, aralkyle en C₇-C₉, cycloalkyle en C₅-C₇, hydroxyalkyle en C₂-C₄, alcoxyalkyle en C₂-C₆, aryle en C₆-C₁₀, glycidyle, un groupe de formule -(CH₂)ₜ-COO-Q ou de formule -(CH₂)ₜ-O-CO-Q dans lesquelles t est 1 ou 2, et Q représente un alkyle en C₁-C₄ ou le phényle ; ou
lorsque n est 2,
R₇ représente les alkylène en C₂-C₁₂, arylène en C₆-C₁₂, un groupe -CH₂CH(OH)-CH₂-O-X-O-CH₂-CH(OH)-CH₂- dans lequel X représente les alkylène en C₂-C₁₀, arylène en C₆-C₁₅ ou cycloalkylène en C₆-C₁₂ ou un groupe -CH₂CH(OZ')CH₂-(OCH₂-CH(OZ')CH₂)₂- dans lequel Z' représente l'hydrogène, les alkyle en C₁-C₁₈, allyle, benzyle, alcanoyle en C₂-C₁₂ ou benzoyle ;
Q₁ représente -N(R₈)- ou -O- ;
E représente un alkylène en C₁-C₃, le groupe -CH₂-CH(R₉)-O- dans lequel R₉ représente l'hydrogène, le méthyle ou le phényle, ou E représente le groupe -(CH₂)₃-NH- ou une liaison directe ;
R₁₀ représente l'hydrogène ou un alkyle en C₁-C₁₈,
R₈ représente l'hydrogène, les alkyle en C₁-C₁₈, cycloalkyle en C₅-C₇, aralkyle en C₇-C₁₂, cyanoéthyle, aryle en C₆-C₁₀, le groupe -CH₂-CH(R₉)-OH dans lequel R₉ a la signification donnée ci-dessus, un groupe de formule I ou un groupe de formule 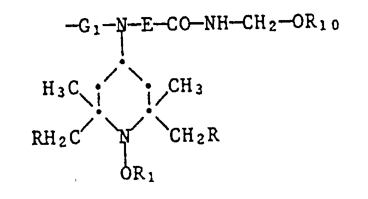 où G₁ peut représenter un alkylène en C₂-C₆ ou un arylène en C₆-C₁₂, ou R₈ représente un groupe -E-CO-NH-CH₂-OR₁₀ ;
T₃ représente l'éthylène ou le 1,2-propylène ou une unité de structure répétitive dérivée d'un copolymère α-oléfinique avec un méthacrylate ou acrylate d'alkyle ; k va de 2 à 100 ;
T₄ a la même signification que R₄ lorsque p est 1 ou 2,
T₅ représente le méthyle,
T₆ représente le méthyle ou l'éthyle, ou T₅ et T₆ ensemble représentent le tétraméthylène ou le pentaméthylène ;
M et Y indépendamment l'un de l'autre représentent le méthylène ou le carbonyle ;
T₇ a la même signification que R₇ ;
T₁₀ et T₁₁ indépendamment l'un de l'autre représentent un alkylène avec 2 à 12 atomes de carbone, ou T₁₁ représente un groupe de formule II ;
e est 2, 3 ou 4 et
T₁₂ représente un groupe de formule -N(R₅)-(CH₂)_{d}-N(R₅)-ou 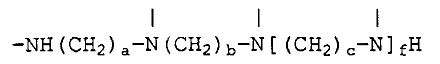 où a, b et c indépendamment l'un de l'autre sont 2 ou 3, d va de 2 à 10 et f est 0 ou 1 ;
T₁₃ a la même signification que R₄ à la condition que T₁₃ ne peut pas être l'hydrogène lorsque n est 1 ;
E₁ et E₂, étant différents, chacun représentent -CO- ou -N(E₅)- où E₅ représente l'hydrogène, un alkyle en C₁-C₁₂
ou alcoxycarbonylalkyle en C₄-C₂₂ ;
E₃ représente l'hydrogène, les alkyle avec 1 à 30 atomes de carbone, phényle, naphtyle, ledit phényle ou ledit naphtyle substitué par du chlore ou par un alkyle avec 1 à 4 atomes de carbone, ou phénylalkyle avec 7 à 12 atomes de carbone, ou ledit phénylalkyle substitué par un alkyle avec 1 à 4 atomes de carbone, et
E₄ représente l'hydrogène, un alkyle avec 1 à 30 atomes de carbone, un phényle, un naphtyle ou un phénylalkyle avec 7 à 12 atomes de carbone, ou
E₃ et E₄ ensemble représentent un polyméthylène avec 4 à 17 atomes de carbone, ou ledit polyméthylène est substitué par jusqu'à quatre groupes alkyle avec 1 à 4 atomes de carbone ;
R₂ de formule (N) est défini comme décrit précédemment, lorsque m est 1, et
G est une liaison directe, les alkylène en C₁-C₁₂, phénylène ou -NH-G'-NH où G' représente un alkylène en C₁-C₁₂.

3. Composition selon la revendication 2, qui contient un composé de formule A, B, C, D, J, K ou M dans lesquelles R représente l'hydrogène et T₅ et T₆ représentent le méthyle.

4. Composition selon la revendication 2, qui contient un composé de formule A, B, C, J ou K dans lesquelles R représente l'hydrogène et R₁ représente les alkyle en C₁-C₁₈, cycloalkyle en C₆-C₁₂, cyclohexényle ou phénylalkyle en C₇-C₉.

5. Composition selon la revendication 2, qui contient un composé de formule A où R représente l'hydrogène et R₁ représente les alkyle en C₁-C₁₈, cycloalkyle en C₆-C₁₂, cyclohexényle ou phénylalkyle en C₇-C₉, m est 1, 2 ou 4 et lorsque m est 1, R₂ représente les alkyle en C₁-C₁₂, allyle, benzyle ou un radical acyle d'un acide carboxylique aliphatique en C₂-C₁₈, ou d'un acide carboxylique cycloaliphatique en C₆-C₁₂ ou d'un acide carboxylique aromatique en C₇-C₁₅, et lorsque m est 2, R₂ représente les alkylène en C₁-C₈, butylène, xylylène ou un radical acyle bivalent d'un acide dicarboxylique aliphatique en C₂-C₁₈, d'un acide dicarboxylique cycloaliphatique ou aromatique en C₈-C₁₄, ou d'un acide dicarbamique aliphatique, cycloaliphatique ou aromatique en C₈-C₁₄, ou R₂ représente un groupe 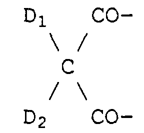 où D₁ représente un alkyle en C₁-C₈ ou le 3,5-di-tert.butyl-4-hydroxybenzyle et D₂ représente D₁ ou l'hydrogène et lorsque m est 4, R₂ représente un radical acyle tétravalent d'un acide butane- ou pentanetétracarboxylique.

6. Composition selon la revendication 5, qui contient un composé de formule A dans laquelle R représente l'hydrogène, R₁ représente les alkyle en C₁-C₁₀, cycloalkyle, cyclohexyle ou phénylalkyle en C₇-C₉, m est 1 ou 2, et lorsque m est 1, R₂ représente les benzyle, alcanoyle en C₂-C₁₈, benzoyle, ou 3,5-di-tert.butyl-4-hydroxybenzoyle et lorsque m est 2, R₂ représente le xylylène ou un radical acyle bivalent d'un acide dicarboxylique aliphatique en C₄-C₁₀ ou d'un acide benzènedicarboxylique.

7. Composition selon la revendication 2, qui contient un composé de formule B dans laquelle R représente l'hydrogène et R₁ représente les alkyle en C₁-C₁₈, cycloalkyle en C₆-C₁₂, cyclohexényle ou phénylalkyle en C₇-C₉, p est 1 ou 2, R₃ représente l'hydrogène, les alkyle en C₁-C₁₂, ou alcanoyle en C₂-C₁₂, allyle et lorsque p est 1, R₄ représente l'hydrogène, un alkyle en C₁-C₁₂ ou un groupe de formule I et lorsque p est 2, R₄ représente un alkylène en C₂-C₈ ou un xylylène et si R₃ ne représente pas un alcanoyle, R₄ peut aussi représenter un radical acyle bivalent d'un acide dicarboxylique aliphatique en C₄-C₁₀ ou d'un acide benzènedicarboxylique ou représente un groupe de formule II, dans laquelle T₈ représente l'hydrogène ou un alkyle en C₁-C₄ et T₉ représente un alkyle en C₁-C₁₂ ou un groupe de formule I.

8. Composition selon la revendication 1, dans laquelle le composé d'amine encombrée est contenu à raison de 0,1 à 10 % en poids, par rapport à la résine solide.

9. Composition selon la revendication 1, qui contient de plus un absorbeur d'UV pris dans le groupe comprenant les benzophénones, les benzotriazoles, les dérivés d'acide acrylique, les aryl-s-triazines, les composés organiques du nickel et les oxanilides.

10. Composition selon la revendication 9, qui contient un absorbeur d'UV à base de benzotriazole.

11. Composition selon la revendication 9, qui contient un absorbeur d'UV de type benzotriazole pris dans le groupe comprenant les 2-[2-hydroxy-3,5-di-α,α-diméthylbenzyl)-phényl]-benzotriazole, 2-(2-hydroxy-3,5-di-tert-octylphényl]-benzotriazole, 2-(2-hydroxy-3-α,α,-diméthylbenzyl-5-tert-octylphényl)-benzotriazole, 2-(2-hydroxy-3-tert-octyl-5-α,α-diméthylbenzylphényl)-benzotriazole, 2-(2-hydroxy-3,5-di-tert-amylphényl)-benzotriazole, 2-[2-hydroxy-3-tert.butyl-5-(2-(ω-hydroxy-octa-(éthylèneoxy)-carbonyl)-éthylphényl]-benzotriazole, 5-chloro-2-[2-hydroxy-3,5-di(α,α-diméthylbenzyl)-phényl]-benzotriazole, 5-chloro-2-(2-hydroxy-3,5-di-tert-butylphényl)-benzotriazole, 2-[2-hydroxy-3-tert-butyl-5-(2-octyloxycarbonyléthyl)-phényl]-benzotriazole, 2-(2-hydroxy-3-sec-dodécyl-5-méthyl-phényl)-benzotriazole et hexaméthylène di[β-(3-tert-butyl-4-hydroxy-5-[2-benzotriazolyl]-phényl)propionate.

12. Composition selon la revendication 9, dans laquelle le benzotriazole est le 2-[2-hydroxy-3,5-di(α,α-diméthylbenzyl)-phényl]-benzotriazole et le 2-[2-hydroxy-3-tert-butyl-5-(2-(ω-hydroxy-octa-(éthylèneoxy)-carbonyl)-éthylphényl]-benzotriazole.

13. Composition selon la revendication 9, qui contient de plus un antioxydant de type phosphite ou de type phosphonite.

14. Composition selon la revendication 1, qui contient de plus un antioxydant de type phénol encombré.

15. Composition de revêtement selon la revendication 1, qui est un système durcissable à la température ambiante à base d'une résine alkyde, d'une résine thermoplastique acrylique, d'une résine alkyde acrylique, d'une résine polyuréthanne ou d'une résine polyester ou desdites résines modifiées par des silicones, isocyanates, époxydes, isocyanurates, cétiurines ou oxazolidones, ou c'est un système à base d'un ester de cellulose ou d'une résine époxyde.

16. Composition de revêtement selon la revendication 1, qui est un système thermodurcissable catalysé par acide à base d'une résine alkyde, polyamide, polyuréthanne, polyester ou acrylique réticulable à chaud.

17. Composition selon la revendication 1, qui est un vernis-émail pour couches de finition industrielles.

18. Composition selon la revendication 1, qui est un vernis-émail pour retouches pour les automobiles.

19. Composition correspondant à une des formules de A' à M' : 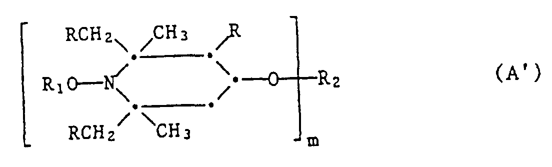 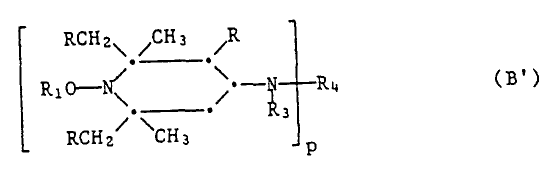 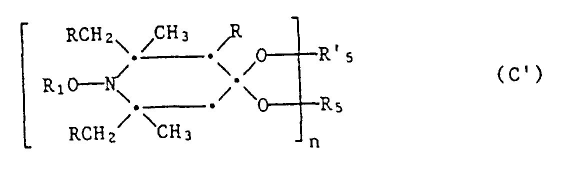 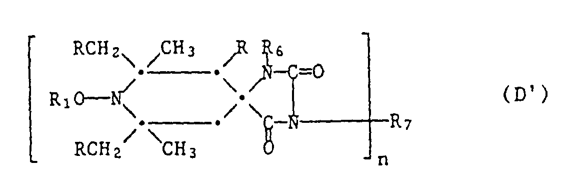 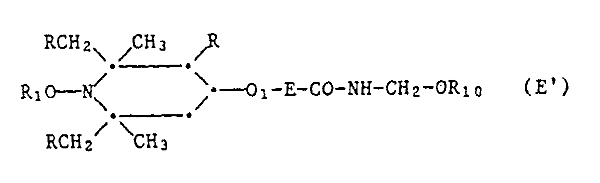 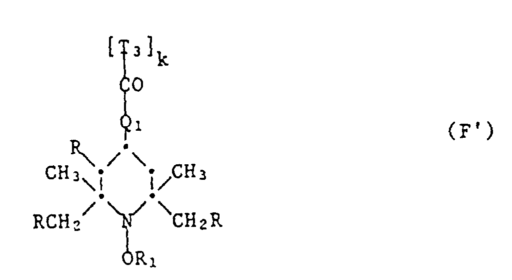 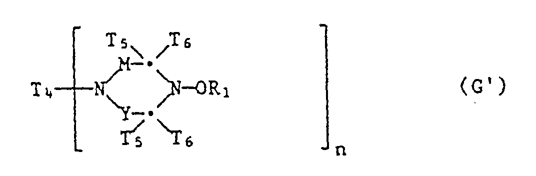 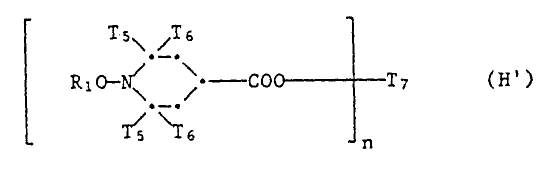 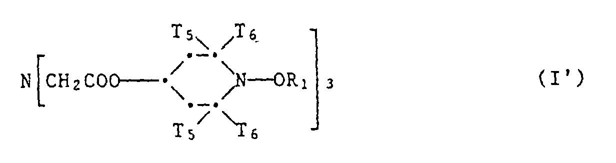 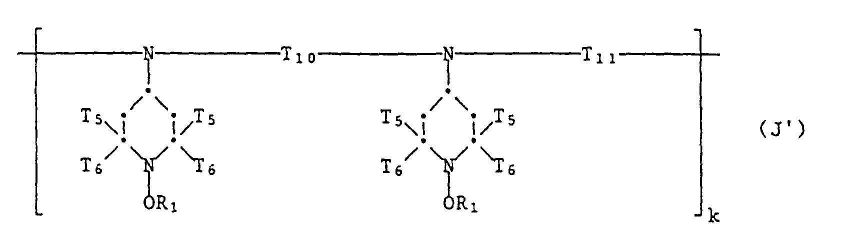 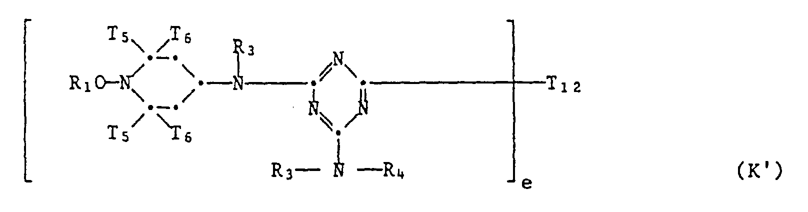 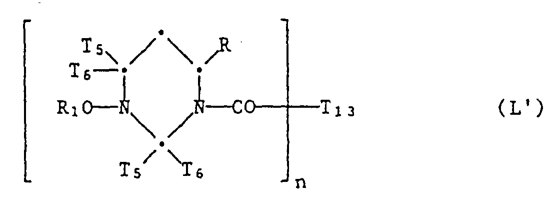 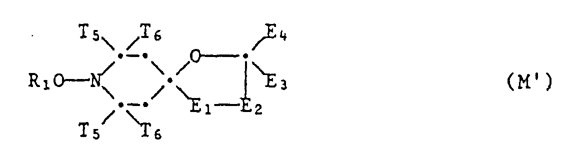 où
R représente l'hydrogène ou le méthyle ;
R₁ représente les alkyle en C₁-C₁₈, alcényle en C₂-C₁₈, alcynyle en C₂-C₁₈, cycloalcènyle en C₅-C₈, cycloalkyle en C₅-C₁₂, bicycloalkyle en C₆-C₁₀, aryle en C₆-C₁₀, aralkyle en C₇-C₉ ou aralkyle en C₇-C₉ substitué par un alkyle en C₁-C₄ ou un aryle ;
m est 2 à 4,
lorsque m est 2,
R₂ représente les alkylène en C₁-C₁₂, alcénylène en C₄-C₁₂, xylylène, un radical acyle bivalent d'un acide dicarbamique ou dicarboxylique aliphatique, cycloaliphatique, araliphatique ou aromatique avec jusqu'à 20 atomes de carbone, ou représente un groupe de formule 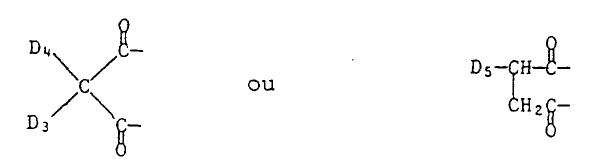 où D₃ et D₄ représentent indépendamment l'un de l'autre l'hydrogène, les alkyle en C₁-C₆, phényle, benzyle, ou 3,5-di-t-butyl-4-hydroxybenzyle, et D₅ représente un alkyle ou un alcényle avec jusqu'à 18 atomes de carbone ;
lorsque m est 3, R₂ représente un radical acyle trivalent d'un acide tricarboxylique aliphatique, cycloaliphatique ou aromatique avec jusqu'à 12 atomes de carbone ;
lorsque m est 4, R₂ représente un radical acyle tétravalent d'un acide tétracarboxylique aliphatique ou aromatique avec jusqu'à 18 atomes de carbone ;
p est 1, 2 ou 3,
R₃ représente l'hydrogène, les alkyle en C₁-C₁₂, cycloalkyle en C₅-C₈, aralkyle en C₇-C₉, alcanoyle en C₂-C₁₈, alcénoyle en C₃-C₅ ou benzoyle ;
lorsque p est 1,
R₄ représente l'hydrogène, les alkyle en C₁-C₁₈, cycloalkyle en C₅-C₈, alcényle en C₂-C₈ non-substitué ou substitué par un groupe cyano, carbonyle ou carbamide ou R₄ représente les aryle en C₆-C₁₀, aralkyle en C₇-C₉, glycidyle, un groupe de formule -CH₂-CH(OH)-Z ou -CONH-Z dans lesquelles Z représente l'hydrogène, le méthyle ou le phényle ; ou R₄ représente un groupe de formule 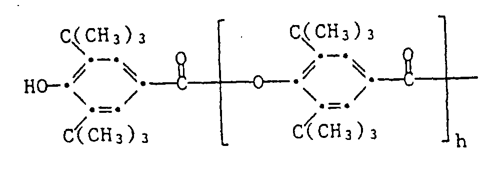 avec h étant 0 ou 1 ;
ou un groupe de formule I 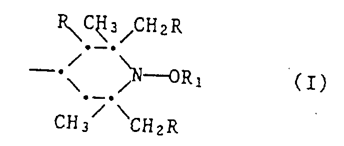 ou R₃ et R₄ ensemble représentent un alkylène avec 4 à 6 atomes de carbone ou un 1-oxo-alkylène ou le radical acyle bivalent d'un acide 1,2- ou 1,3-dicarboxylique aliphatique ou aromatique ;
lorsque p est 2,
R₄ représente les alkylène en C₁-C₁₂, arylène en C₆-C₁₂, xylylène, un groupe -CH₂CH(OH)-CH₂- ou un groupe -CH₂-CH(OH)-CH₂-O-X-O-CH₂-CH(OH)-CH₂- dans lesquels X représente les alkylène en C₂-C₁₀, arylène en C₆-C₁₅ ou cycloalkylène en C₆-C₁₂ ; ou, à la condition que R₃ ne représente pas un alcanoyle, un alcénoyle ou un benzoyle, R₄ peut aussi représenter un radical acyle bivalent d'un acide dicarbamique ou d'un acide acide dicarboxylique aliphatique, cycloaliphatique ou aromatique ou peut représenter le groupe -CO- ;
ou R₄ représente un groupe de formule II 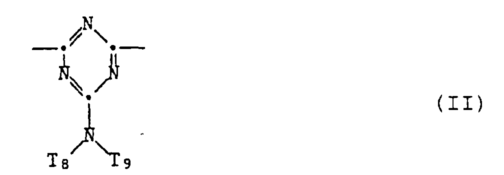 dans laquelle T₈ et T₉ indépendamment l'un de l'autre représentent l'hydrogène, un alkyle avec 1 à 18 atomes de carbone, ou T₈ et T₉ ensemble représentent un alkylène avec 4 à 6 atomes de carbone ou le 3-oxapentaméthylène ;
lorsque p est 3,
R₄ représente le 2,4,6-triazinetriyle,
n est 1 ou 2 et
lorsque n est 1,
R₅ et R'₅ indépendamment l'un de l'autre représentent les alkyle en C₁-C₁₂, alcényle en C₂-C₁₂, aralkyle en C₇-C₁₂, ou R₅ représente aussi l'hydrogène, ou R₅ et R'₅ ensemble représentent un hydroxyalkylène ou un alkylène en C₂-C₈ ou un acyloxyalkylène en C₄-C₂₂ ;
lorsque n est 2,
R₅ et R'₅ ensemble représentent (-CH₂)₂C(CH₂-)₂ ;
R₆ représente l'hydrogène, les alkyle en C₁-C₁₂, allyle, benzyle, glycidyle ou alcoxyalkyle en C₂-C₆ ;
lorsque n est 1,
R₇ représente l'hydrogène, les alkyle en C₁-C₁₂, alcényle en C₃-C₅, aralkyle en C₇-C₉, cycloalkyle en C₅-C₇, hydroxyalkyle en C₂-C₄, alcoxyalkyle en C₂-C₆, aryle en C₆-C₁₀, glycidyle, un groupe de formule -(CH₂)ₜ-COO-Q ou de formule -(CH₂)ₜ-O-CO-Q où t est 1 ou 2, et Q représente un alkyle en C₁-C₄ ou un phényle ;
et lorsque n est 2,
R₇ représente les alkylène en C₂-C₁₂, arylène en C₆-C₁₂, un groupe -CH₂CH(OH)-CH₂-O-X-O-CH₂-CH(OH)-CH₂- où X représente les alkylène en C₂-C₁₀, arylène en C₆-C₁₅ ou cycloalkylène en C₆-C₁₂, ou un groupe -CH₂CH(OZ')CH₂-(OCH₂-CH(OZ')CH₂)₂- où Z' représente l'hydrogène, les alkyle en C₁-C₁₈, allyle, benzyle, alcanoyle en C₂-C₁₂ ou benzoyle ;
Q₁ représente -N(R₈)- ou -O- ;
E représente un alkylène en C₁-C₃, le groupe -CH₂-CH(R₉)-O- où R₉ représente l'hydrogène, le méthyle ou le phényle, ou E représente le groupe -(CH₂)₃-NH- ou une liaison directe ;
R₁₀ représente l'hydrogène ou un alkyle en C₁-C₁₈,
R₈ représente l'hydrogène, les alkyle en C₁-C₁₈, cycloalkyle en C₅-C₇, aralkyle en C₇-C₁₂, cyanoéthyle, aryle en C₆-C₁₀, le groupe -CH₂-CH(R₉)-OH où R₉ a la signification définie ci-dessus, ou R₈ est un groupe de formule I ou un groupe de formule 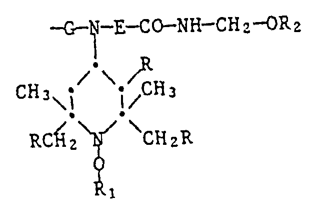 dans laquelle G représente un alkylène en C₂-C₆ ou un arylène en C₆-C₁₂ ;
ou R₈ représente un groupe -E-CO-NH-CH₂-OR₁₀ ;
T₃ représente l'éthylène ou le 1,2-propylène, ou est l'unité de structure répétitive dérivant d'un copolymère d'une α-oléfine avec un méthacrylate ou acrylate d'alkyle
k va de 2 à 10 0 ;
T₄ a la même signification que R₄ lorsque p est 1 ou 2,
T₅ représente le méthyle,
T₆ représente le méthyle ou l'éthyle, ou T₅ et T₆ ensemble représentent le tétraméthylène ou le pentaméthylène ;
M et Y sont indépendamment l'un de l'autre un méthylène
ou un carbonyle ;
T₇ est identique à R₇ ;
T₁₀ et T₁₁ indépendamment l'un de l'autre représentent un alkylène avec 2 à 12 atomes de carbone, ou T₁₁ représente un groupe de formule II,
e est 2, 3 ou 4,
T₁₂ représente un groupe de formule -N(R⁴)-(CH₂)_{d}-N(R⁴)-ou 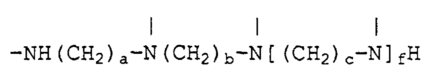 dans lesquelles a, b et c indépendamment l'un de l'autre sont 2 ou 3, d va de 2 à 10 et f est 0 ou 1, ;
lorsque n est 1, T₁₃ représente les alkyle en C₂-C₁₈, alcényle en C₂-C₁₈, cycloalkyle en C₆-C₈, aryle en C₆-C₁₂, phényle substitué par les alkyle en C₁-C₄, hydroxy ou halogène et lorsque n est 2, T₁₃ a la même signification que R₂ ;
E₁ et E₂, étant différents, chacun représente -CO- ou - N(E₅)-, où E₅ représente l'hydrogène, les alkyle en C₁-C₁₂, alcoxycarbonylalkyle avec 4 à 22 atomes de carbone ;
E₃ représente l'hydrogène, les alkyle avec 1 à 30 atomes de carbone, phényle, naphtyle, ledit phényle ou ledit naphtyle substitué par le chlore ou par un alkyle avec 1 à 4 atomes de carbone, ou phénylalkyle avec 7 à 12 atomes de carbone, ou ledit phénylalkyle substitué par un alkyle avec 1 à 4 atomes de carbone, et
E₄ représente l'hydrogène, les alkyle avec 1 à 30 atomes de carbone,phényle, naphtyle ou phénylalkyle avec 7 à 12 atomes de carbone, ou
E₃ et E₄ ensemble représentent un polyméthylène avec 4 à 17 atomes de carbone, ou ledit polyméthylène substitué par jusqu'à quatre groupes alkyle avec 1 à 4 atomes de carbone, à la condition que dans la formule B', R3 ou R₄ ne représentent pas l'hydrogène si R₁ est un groupe alkyle.

20. Composé selon la revendication 19, répondant à la formule (A') à (F') où R représente l'hydrogène.

21. Composé selon la revendication 19, répondant à la formule (G') à (M') où T₅ et T₆ représentent le méthyle.

22. Composé de la revendication 19, de formules (A') à (M') dans lesquelles R représente l'hydrogène, R₁ représente les alkyle en C₁-C₁₈, alcényle en C₂-C₆, cycloalkyle en C₅-C₈, cyclohexyle, phényle ou aralkyle en C₇-C₉ ; m est 2 à 4 et lorsque m est 2, R₂ représente les alkylène en C₂-C₈, alcénylène en C₄-C₈, xylylène, un radical acyle bivalent d'un acide dicarboxylique aliphatique, cycloaliphatique ou aromatique avec jusqu'à 12 atomes de carbone ou d'un dicarbamique aliphatique ou aromatique avec jusqu'à 12 atomes de carbone ou représente un groupe de formule 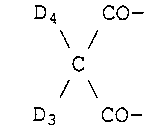 dans laquelle D₃ et D₄ indépendamment l'un de l'autre représentent l'hydrogène, les alkyle en C₁-C₆, benzyle ou 3,5-di-t-butyl-4-hydroxybenzyle et lorsque m est 3, R₂ représente un radical acyle trivalent d'un acide tricarboxylique aliphatique ou aromatique avec jusqu'à 12 atomes de carbone et lorsque m est 4, R₂ représente un radical acyle tétravalent d'un acide tétracarboxylique aliphatique ou aromatique avec jusqu'à 12 atomes de carbone, p est 1, 2 ou 3, R₃ représente l'hydrogène, les alkyle en C₁-C₁₂, cycloalkyle en C₅-C₈, aralkyle en C₇-C₉, alcanoyle en C₂-C₁₈ ou benzoyle et lorsque p est 1, R₄ représente l'hydrogène, les alkyle en C₁-C₁₈, cycloalkyle en C₅-C₈, phényle, benzyle ou un groupe de formule 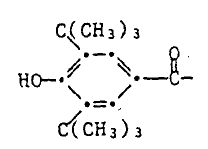 ou de formule 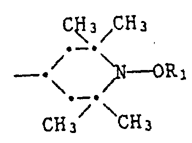 et lorsque p est 2, R₄ représente les alkylène en C₂-C₁₂, arylène en C₆-C₁₂, xylylène, ou à la condition que R₃ ne représente pas un alcanoyle ou un benzoyle, R₄ peut aussi représenter un radical acyle bivalent d'un acide dicarboxylique aliphatique ou aromatique avec jusqu'à 12 atomes de carbone ou d'un acide dicarbamique aliphatique ou aromatique avec jusqu'à 12 atomes de carbone ou peut représenter un groupe de formule II dans laquelle T₈ et T₉ indépendamment l'un de l'autre représentent l'hydrogène ou un alkyle en C₁-C₁₂ ou T₈ et T₉ ensemble représentent un alkylène en C₄-C₆ ou le 3-oxapentaméthylène, et lorsque p est 3, R₄ représente le 2,4,6-triazinetriyle; n est 1 ou 2 et lorsque n est 1, R₅ et R'₅ représentent un alkyle en C₁-C₁₂ ou le benzyle, ou R₅ et R'₅ ensemble représentent un hydroxyalkylène ou un alkylène en C₂-C₈ et lorsque n est 2, R₅ et R'₅ ensemble représentent (-CH₂)₂C(CH₂-)₂ ;
R₆ représente l'hydrogène, les alkyle en C₁-C₁₂, allyle ou benzyle, et lorsque n est 1, R₇ représente l'hydrogène, les alkyle en C₁-C₁₂, allyle, benzyle, cyclohexyle, 2-hydroxyéthyle ou un groupe de formule -CH₂CH₂-COO-Q où Q représente un alkyle en C₁-C₄, et lorsque n est 2, R₇ représente un alkylène en C₂-C₁₂ ou un arylène en C₆-C₁₂ ;
Q₁ représente -N(R₈)- ou -O- ;
E représente un alkylène en C₁-C₃ ou une liaison directe ;
R₁₀ représente l'hydrogène ou un alkyle en C₁-C₁₄;
R₈ représente l'hydrogène, les alkyle en C₁-C₁₂, cyclohexyle, benzyle, cyanoéthyle ou groupe 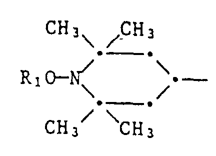 T₃ représente l'éthylène ou le 1,2-propylène, k va de 2 à 100 ;
T₄ a la même signification que R₄ lorsque p est 1 ou 2,
T₅ et T₆ représentent le méthyle, M et Y indépendamment l'un de l'autre représentent -CH₂- ou -CO- ;
T₇ a la même signification que R₇ ;
T₁₀ et T₁₁ indépendamment l'un de l'autre représentent un alkylène en C₂-C₈ ou T₁₁ représente un groupe de formule I,
e est 3 ou 4,
T₁₂ représente un groupe 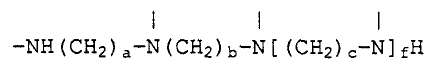 dans lequel a, b et c indépendamment l'un de l'autre sont 2 ou 3, f est 0 ou 1 ;
lorsque n est 1, T₁₃ représente un alkyle en C₂-C₁₈, un cyclohexyle ou un phényle et lorsque n est 2, T₁₃ a la même signification que R₂ ;
E₁ représente -CO- et E₂ représente -N(E₅)- où E₅ représente l'hydrogène, un alkyle en C₁-C₁₂ ou un alcoxycarbonylalkyle en C₄-C₁₈, E₃ et E₄ indépendamment l'un de l'autre représentent un alkyle en C₁-C₁₂ ou un phényle ou E₃ et E₄ ensemble représentent un polyméthylène en C₄-C₁₂.

23. Composé selon la revendication 19 de formule (A'), (B'), (C'), (J'), (K') ou (M') dans lesquels R représente l'hydrogène, R₁ représente les alkyle en C₁-C₁₈, cyclohexyle, cyclohexényle, méthylcyclohexyle ou phénylalkyle en C₇-C₉ ; m est 2, R₂ représente un alkylène en C₂-C₈, le xylylène ou un groupe -CO-R₁₁-CO-, où R₁₁ représente un alkylène en C₂-C₈, le cyclohexylène ou le phénylène ou un groupe 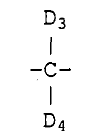 dans laquelle D₃ et D₄ indépendamment l'un de l'autre représentent l'hydrogène, les alkyle en C₁-C₄, benzyle
ou 3,5-di-t-butyl-4-hydroxybenzyle ;
p est 1 ou 2, R₃ représente l'hydrogène, un alkyle en C₁-C₁₂ ou un alcanoyle en C₂-C₈, et lorsque p est 1, R₄ représente l'hydrogène, un alkyle en C₁-C₁₂ et lorsque p est 2, R₄ représente un alkylène en C₂-C₈ ou -CO-R₁₁-CO- ;
n est 1 ou 2, et lorsque n est 1, R₅ et R'₅ ensemble représentent un alkylène en C₂-C₈ et lorsque n est 2, R₅ et R'₅ ensemble représentent (-CH₂)₂C(CH₂-)₂ ;
k va de 5 à 20, T₁₀ représente un alkylène en C₂-C₈, et T₁₁ représente un groupe de formule II, dans laquelle T₈ et T₉ indépendamment l'un de l'autre représentent l'hydrogène ou un alkyle en C₁-C₁₂, ou T₈ et T₉ ensemble représentent le pentaméthylène ou le 3-oxapentaméthylène, T₅ et T₆ représentent le méthyle ; e est 4, T₁₂ représente un groupe 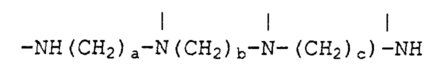 dans lequel a, b et c indépendamment l'un de l'autre valent 2 ou 3 ;
E₁ représente -CO- et E₂ représente -N(E₅)-, dans laquelle E₅ représente l'hydrogène, un alkyle en C₁-C₁₂ ou un alcoxycarbonylalkyle en C₄-C₁₅ et E₃ et E₄ indépendamment l'un de l'autre représentent un alkyle en C₁-C₁₂ ou E₃ et E₄ ensemble représentent un polyméthylène en C₅-C₁₂.

24. Sébacate de di-(1-méthoxy-2,2,6,6-tétraméthylpipéridin-4-yle) selon la revendication 19.

25. Sébacate de di-(1-cyclohexyloxy-2,2,6,6-tétraméthylpipéridin-4-yle) selon la revendication 19.

26. Isophtalate de di-(1-cyclohexyloxy-2,2,6,6-tétraméthylpipéridin-4-yle) selon la revendication 19.

27. Sébacate de di-(1-octyloxy-2,2,6,6-tétraméthylpipéridin-4-yle) selon la revendication 19.

28. 3,15-dicyclohexyloxy-2,2,4,4,14,14,16,16-octaméthyl-7,11,18,21-tétraoxa-3,15-diazatrispiro-[5.2.2.5.2.2]hénéicosane selon la revendication 19.

29. Succinate de di-(1-cyclohexyloxy-2,2,6,6-tétraméthylpipéridin-4-yle) selon la revendication 19.

30. Utilisation d'un composé selon la revendication 19 en tant que stabilisant pour matières organiques.

31. Utilisation selon la revendication 30 en tant que stabilisants pour polymères.

32. Utilisation selon la revendication 30 en tant que stabilisant pour couches photographiques.

33. Matière organique contenant une quantité efficace d'un composé de la revendication 19.

## Revendications (Revendications pour l'(les) Etat(s) contractant(s) suivant(s): ES)

1. Composition stabilisée de revêtement thermodurcissable, durcissante à la température ambiante ou catalysée par acide, contenant une quantité stabilisante efficace d'un composé d'amine encombrée contenant le groupe 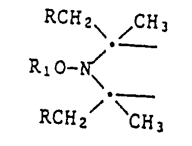 dans lequel R représente l'hydrogène ou le méthyle et R₁ représente les alkyle en C₁-C₁₈, alcényle en C₂-C₁₈, alcynyle en C₂-C₁₈, cycloalkyle en C₅-C₁₂, bicycloalkyle en C₆-C₁₀, cycloalcényle en C₅-C₈, aryle en C₆-C₁₀, aralkyle en C₇-C₉ ou aralkyle en C₇-C₉ substitué par un alkyle en C₁-C₄ ou le phényle.

2. Composition selon la revendication 1, qui contient un composé d'amine encombrée correspondant à une des formules A à N 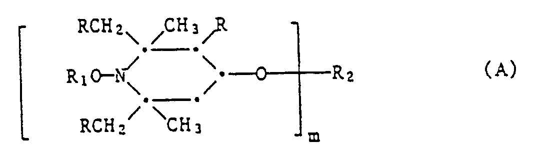 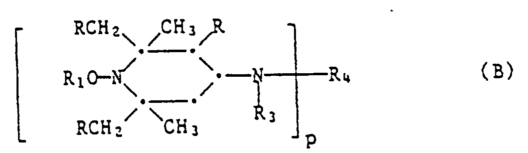 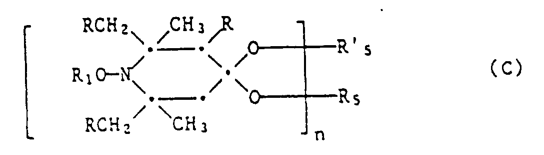 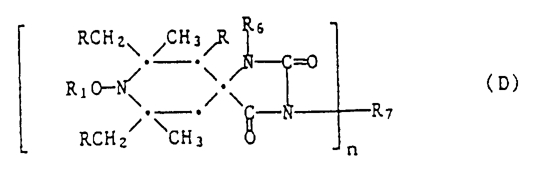 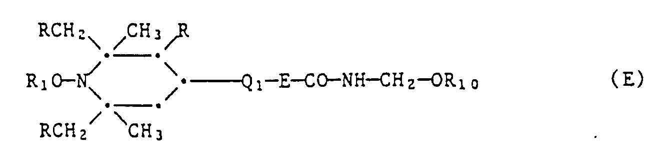 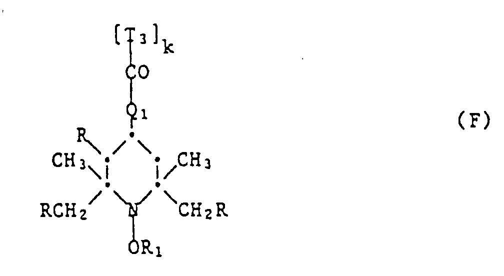 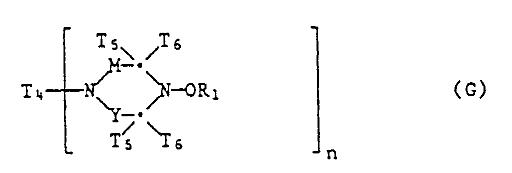 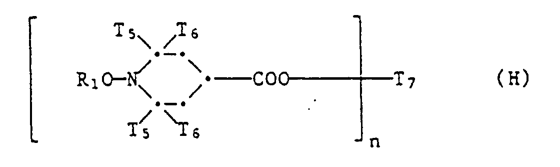 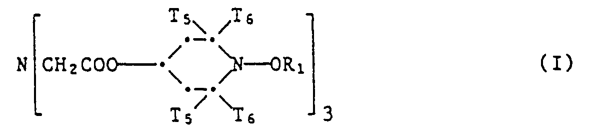 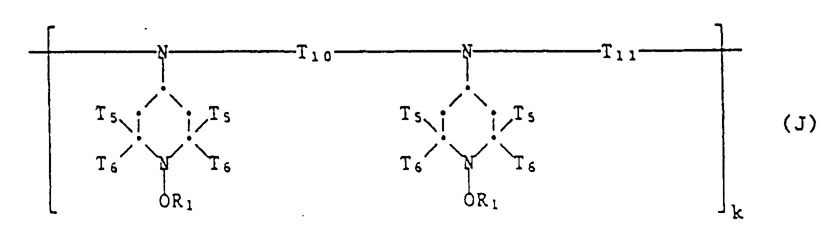 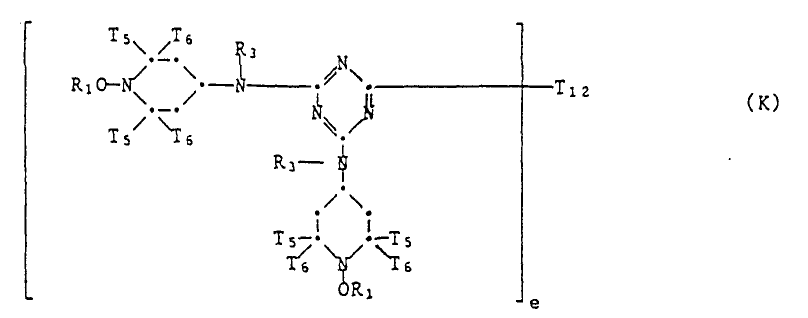 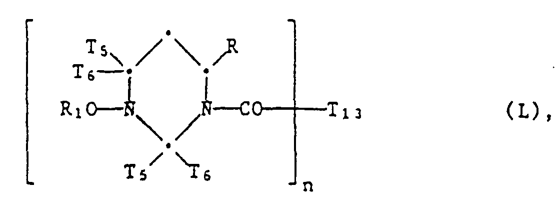 ou 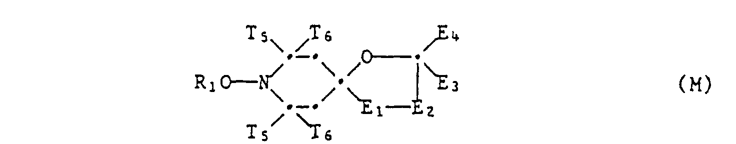 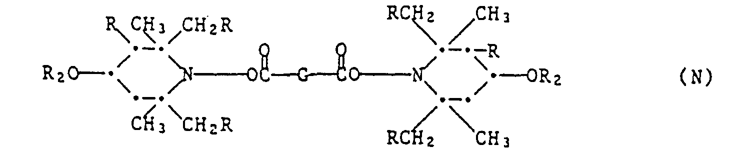 dans lesquelles
R représente l'hydrogène ou le méthyle,
R₁ représente les alkyle en C₁-C₁₈, alcényle en C₂-C₁₈, alcynyle en C₂-C₁₈, cycloalkyle en C₅-C₁₂, bicycloalkyle en C₆-C₁₀, cycloalcényle en C₅-C₈, aryle en C₆-C₁₀, aralkyle en C₇-C₉ ou aralkyle en C₇-C₉ substitué par un alkyle en C₁-C₄ ou le phényle ;
m est 1 à 4,
lorsque m est 1,
R₂ représente l'hydrogène, les alkyles en C₁-C₁₈ pouvant être éventuellement interrompus par un ou plusieurs atomes d'oxygène, les alcényle en C₂-C₁₂, aryle en C₆-C₁₀, aralkyle en C₇-C₁₈, glycidyle, un radical acyle monovalent d'un acide carboxylique aliphatique, cycloaliphatique, araliphatique ou aromatique ou d'un acide carbamique ou R₂ représente un groupe 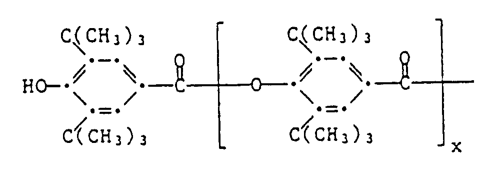 où x est 0 ou 1, ou représente un groupe 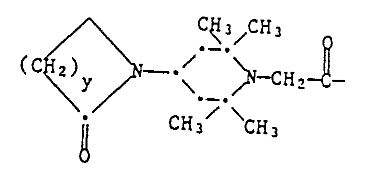 où y va de 2 à 4 ;
lorsque m est 2,
R₂ représente les alkylène en C₁-C₁₂, alcénylène en C₄-C₁₂, xylylène, un radical acyle bivalent d'un acide carboxylique aliphatique, cycloaliphatique, araliphatique ou aromatique ou d'un acide dicarbamique,
ou R₂ représente un groupe 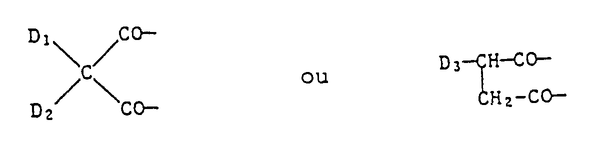 où D₁ et D₂ sont indépendamment l'un de l'autre l'hydrogène, les alkyle contenant jusqu'à 8 atomes de carbone, phényle, benzyle ou 3,5-di-t-butyl-4-hydroxybenzyle et D₃ représente un radical alkyle ou alcényle contenant jusqu'à 18 atomes de carbone ;
lorsque m est 3, R₂ représente un radical acyle trivalent d'un acide tricarboxylique aliphatique, cycloaliphatique ou aromatique ;
lorsque m est 4, R₂ représente un radical acyle tétravalent d'un acide tétracarboxylique aliphatique ou aromatique ;
p est 1, 2 ou 3,
R₃ représente l'hydrogène, les alkyle en C₁-C₁₂, cycloalkyle en C₅-C₇, aralkyle en C₇-C₉, alcanoyle en C₂-C₁₈, alcénoyle en C₃-C₅ ou benzoyle ;
lorsque p est 1,
R₄ représente l'hydrogène, les alkyle en C₁-C₁₈, cycloalkyle en C₅-C₇, alcényle en C₂-C₈ non-substitué ou substitué par un groupe cyano, carbonyle ou carbamide, ou représente les aryle, aralkyle, glycidyle, un groupe de formule -CH₂-CH(OH)-Z ou -CONH-Z dans lesquelles Z représente l'hydrogène, le méthyle ou le phényle ; ou R₄ représente un groupe de formule I 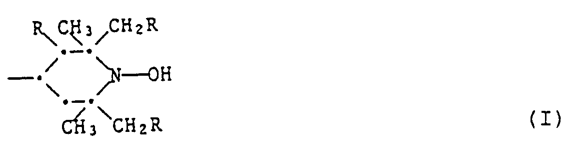 ou le groupe de formule 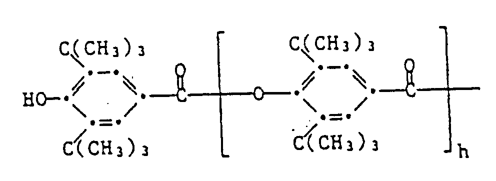 avec h étant 0 ou 1 ;
ou R₃ et R₄ ensemble, représentent un alkylène avec 4 à 6 atomes de carbone ou un 1-oxo-alkylène ou le radical acyle bivalent d'un acide 1,2- ou 1,3-dicarboxylique aliphatique ou aromatique,
lorsque p est 2,
R₄ représente les alkylène en C₂-C₁₂, arylène en C₆-C₁₂, xylylène, un groupe -CH₂CH(OH)-CH₂-, ou un groupe -CH₂-CH(OH)-CH₂-O-X-O-CH₂-CH(OH)-CH₂- où X représente les alkylène en C₂-C₁₀, arylène en C₆-C₁₅ ou cycloalkylène en C₆-C₁₂ ; ou à la condition que R₃ ne représente pas un alcanoyle, alcénoyle ou benzoyle, R₄ peut aussi représenter un radical acyle bivalent d'un acide dicarbamique ou dicarboxylique aliphatique, cycloaliphatique ou aromatique ou peut représenter le groupe -CO- ; ou R₄ représente un groupe de formule II 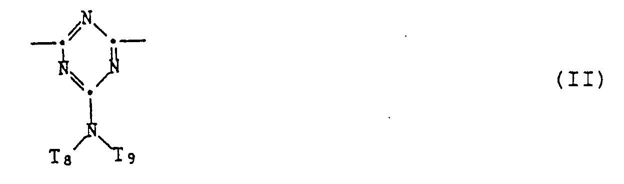 où T₈ et T₉ indépendamment l'un de l'autre représentent l'hydrogène, un alkyle avec 1 à 18 atomes de carbone ou un groupe de formule I, ou T₈ et T₉ ensemble, représentent un alkylène avec 4 à 6 atomes de carbone ou le 3-oxapentaméthylène ;
lorsque p est 3,
R₄ représente le 2,4,6-triazinetriyle,
n est 1 ou 2 et
lorsque n est 1,
R₅ et R'₅ indépendamment l'un de l'autre représentent les alkyle en C₁-C₁₂, alcényle en C₂-C₁₂, aralkyle en C₇-C₁₂ ou R₅ représente aussi l'hydrogène, ou R₅ et R'₅ ensemble représentent les hydroxyalkylène ou alkylène en C₂-C₈ ou acyloxyalkylène en C₄-C₂₂ ;
lorsque n est 2,
R₅ et R'₅ ensemble représentent (-CH₂)₂C(CH₂-)₂ ;
R₆ représente l'hydrogène, les alkyle en C₁-C₁₂, allyle, benzyle, glycidyle ou alcoxyalkyle en C₂-C₆ ;
lorsque n est 1,
R₇ représente l'hydrogène, les alkyle en C₁-C₁₂, alcényle en C₃-C₅, aralkyle en C₇-C₉, cycloalkyle en C₅-C₇, hydroxyalkyle en C₂-C₄, alcoxyalkyle en C₂-C₆, aryle en C₆-C₁₀, glycidyle, un groupe de formule -(CH₂)ₜ-COO-Q ou de formule -(CH₂)ₜ-O-CO-Q dans lesquelles t est 1 ou 2, et Q représente un alkyle en C₁-C₄ ou le phényle ; ou
lorsque n est 2,
R₇ représente les alkylène en C₂-C₁₂, arylène en C₆-C₁₂, un groupe -CH₂CH(OH)-CH₂-O-X-O-CH₂-CH(OH)-CH₂- dans lequel X représente les alkylène en C₂-C₁₀, arylène en C₆-C₁₅ ou cycloalkylène en C₆-C₁₂ ou un groupe - CH₂CH(OZ') CH₂-(OCH₂-CH(OZ')CH₂)₂- dans lequel Z' représente l'hydrogène, les alkyle en C₁-C₁₈, allyle, benzyle, alcanoyle en C₂-C₁₂ ou benzoyle ;
Q₁ représente -N(R₈)- ou -O- ;
E représente un alkylène en C₁-C₃, le groupe -CH₂-CH(R₉)-O- dans lequel R₉ représente l'hydrogène, le méthyle ou le phényle, ou E représente le groupe -(CH₂)₃-NH- ou une liaison directe ;
R₁₀ représente l'hydrogène ou un alkyle en C₁-C₁₈,
R₈ représente l'hydrogène, les alkyle en C₁-C₁₈, cycloalkyle en C₅-C₇, aralkyle en C₇-C₁₂, cyanoéthyle, aryle en C₆-C₁₀, le groupe -CH₂-CH(R₉)-OH dans lequel R₉ a la signification donnée ci-dessus, un groupe de formule I ou un groupe de formule 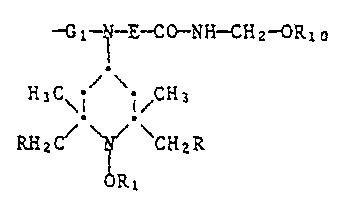 où G₁ peut représenter un alkylène en C₂-C₆ ou un arylène en C₆-C₁₂, ou R₈ représente un groupe -E-CO-NH-CH₂-OR₁₀ ;
T₃ représente l'éthylène ou le 1,2-propylène, ou représente une unité de structure répétitive dérivée d'un copolymère α-oléfinique avec un méthacrylate ou acrylate d'alkyle ;
k va de 2 à 100 ;
T₄ a la même signification que R₄ lorsque p est 1 ou 2,
T₅ représente le méthyle ;
T₆ représente le méthyle ou l'éthyle, ou T₅ et T₆ ensemble représentent le tétraméthylène ou le pentaméthylène ;
M et Y indépendamment l'un de l'autre représentent le méthylène ou le carbonyle;
T₇ a la même signification que R₇,
T₁₀ et T₁₁ indépendamment l'un de l'autre représentent un alkylène avec 2 à 12 atomes de carbone, ou T₁₁ représente un groupe de formule II ;
e est 2, 3 ou 4 et
T₁₂ représente un groupe de formule -N(R₅)-(CH₂)_{d}-N(R₅)-ou 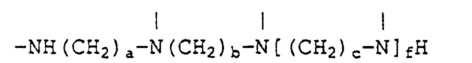 où a, b et c indépendamment l'un de l'autre sont 2 ou 3, d va de 2 à 10 et f est 0 ou 1 ;
T₁₃ a la même signification que R₄ à la condition que T₁₃ ne peut pas être l'hydrogène lorsque n est 1 ;
E₁ et E₂, étant différents, chacun représentent -CO- ou -N(E₅)- où E₅ représente l'hydrogène, un alkyle en C₁-C₁₂
ou alcoxycarbonylalkyle en C₁-C₂₂ ;
E₃ représente l'hydrogène, les alkyle avec 1 à 30 atomes de carbone, phényle, naphtyle, ledit phényle ou ledit naphtyle substitué par du chlore ou par un alkyle avec 1 à 4 atomes de carbone, ou phénylalkyle avec 7 à 12 atomes de carbone, ou ledit phénylalkyle substitué par un alkyle avec 1 à 4 atomes de carbone, et
E₄ représente l'hydrogène, un alkyle avec 1 à 30 atomes de carbone, un phényle, un naphtyle ou un phénylalkyle avec 7 à 12 atomes de carbone, ou
E₃ et E₄ ensemble représentent un polyméthylène avec 4 à 17 atomes de carbone, ou ledit polyméthylène est substitué jusqu'à quatre groupes alkyle avec 1 à 4 atomes de carbone ; R₂ de formule (N), est défini comme décrit précédemment, lorsque m est 1, G est une liaison directe, un alkylène en C₁-C₁₂, un phénylène ou -NH-G'-NH où G' représente un alkylène en C₁-C₁₂.

3. Composition selon la revendication 2, qui contient un composé de formule A, B, C, D, J, K ou M dans lesquelles R représente l'hydrogène et T₅ et T₆ représentent le méthyle.

4. Composition selon la revendication 2, qui contient un composé de formule A, B, C, J ou K dans lesquelles R représente l'hydrogène et R₁ représente les alkyle en C₁-C₁₈, cycloalkyle en C₆-C₁₂, cyclohexényle ou phénylalkyle en C₇-C₉.

5. Composition selon la revendication 2, qui contient un composé de formule A où R représente l'hydrogène et R₁ représente les alkyle en C₁-C₁₈, cycloalkyle en C₆-C₁₂, cyclohexényle ou phénylalkyle en C₇-C₉, m est 1, 2 ou 4 et lorsque m est 1, R₂ représente les alkyle en C₁-C₁₂, allyle, benzyle ou un radical acyle d'un acide carboxylique aliphatique en C₂-C₁₈, ou d'un acide carboxylique cycloaliphatique en C₆-C₁₂ ou d'un acide carboxylique aromatique en C₇-C₁₅, et lorsque m est 2, R₂ représente les alkylène en C₁-C₈, butylène, xylylène ou représente un radical acyle bivalent d'un acide dicarboxylique aliphatique en C₂-C₁₈, d'un acide dicarboxylique cycloaliphatique ou aromatique en C₈-C₁₄, ou d'un acide dicarbamique aliphatique, cycloaliphatique ou aromatique en C₈-C₁₄, ou R₂ représente un groupe 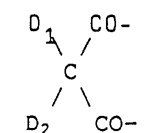 où D₁ représente un alkyle en C₁-C₈ ou le 3,5-di-tert.butyl-4-hydroxybenzyle et D₂ représente D₁ ou l'hydrogène et lorsque m est 4, R₂ représente un radical acyle tétravalent d'un acide butane- ou pentanetétracarboxylique.

6. Composition selon la revendication 5, qui contient un composé de formule A dans laquelle R représente l'hydrogène, R₁ représente les alkyle en C₁-C₁₀, cycloalkyle, cyclohexyle ou phénylalkyle en C₇-C₉, m est 1 ou 2, et lorsque m est 1, R₂ représente les benzyle, alcanoyle en C₂-C₁₈, benzoyle, ou 3,5-di-tert.butyl-4-hydroxybenzoyle et lorsque m est 2, R₂ représente le xylylène ou un radical acyle bivalent d'un acide dicarboxylique aliphatique en C₄-C₁₀ ou d'un acide benzènedicarboxylique.

7. Composition selon la revendication 2, qui contient un composé de formule B dans laquelle R représente l'hydrogène et R₁ représente les alkyle en C₁-C₁₈, cycloalkyle en C₆-C₁₂, cyclohexényle ou phénylalkyle en C₇-C₉, p est 1 ou 2, R₃ représente l'hydrogène, un alkyle en C₁-C₁₂, ou un alcanoyle en C₂-C₁₂, un allyle et lorsque p est 1, R₄ représente l'hydrogène, un alkyle en C₁-C₁₂ ou un groupe de formule I et lorsque p est 2, R₄ représente un alkylène en C₂-C₈ ou un xylylène et si R₃ ne représente pas un alcanoyle, R₄ peut représenter aussi un radical acyle bivalent d'un acide dicarboxylique aliphatique en C₄-C₁₀ ou d'un acide benzènedicarboxylique ou représente un groupe de formule II dans laquelle T₈ représente l'hydrogène ou un alkyle en C₁-C₄ et T₉ représente un alkyle en C₁-C₁₂ ou un groupe de formule I.

8. Composition selon la revendication 1, dans laquelle le composé d'amine encombrée est contenu à raison de 0,1 à 10 % en poids, par rapport à la résine solide.

9. Composition selon la revendication 1, qui contient de plus un absorbeur d'UV pris dans le groupe comprenant les benzophénones, les benzotriazoles, les dérivés d'acide acrylique, les aryl-s-triazines, les composés organiques du nickel et les oxanilides.

10. Composition selon la revendication 9, qui contient un absorbeur d'UV à base de benzotriazole.

11. Composition selon la revendication 9, qui contient un absorbeur d'UV de type benzotriazole pris dans le groupe comprenant les 2-[2-hydroxy-3,5-di-α,α-diméthylbenzyl)-phényl]-benzotriazole, 2-(2-hydroxy-3,5-di-tert-octylphényl]-benzotriazole, 2-(2-hydroxy-3-α,α,-diméthylbenzyl-5-tert-octylphényl)-benzotriazole, 2-(2-hydroxy-3-tert-octyl-5-α,α-diméthylbenzylphényl)-benzotriazole, 2-(2-hydroxy-3,5-di-tert-amylphényl)-benzotriazole, 2-[2-hydroxy-3-tert.butyl-5-(2-(ω-hydroxy-octa-(éthylèneoxy)-carbonyl)-éthylphényl]-benzotriazole, 5-chloro-2-[2-hydroxy-3,5-di(α,α-diméthylbenzyl)-phényl]-benzotriazole, 5-chloro-2-(2-hydroxy-3,5-di-tert-butylphényl)-benzotriazole, 2-[2-hydroxy-3-tert-butyl-5-(2-octyloxycarbonyléthyl)-phényl]-benzotriazole, 2-(2-hydroxy-3-sec-dodécyl-5-méthyl-phényl)-benzotriazole et hexaméthylène di[β-(3-tert-butyl-4-hydroxy-5-[2-benzotriazolyl]-phényl)propionate.

12. Composition selon la revendication 9, dans laquelle le benzotriazole est le 2-[2-hydroxy-3,5-di(α,α-diméthylbenzyl)-phényl]-benzotriazole et le 2-[2-hydroxy-3-tert-butyl-5-(2-(ω-hydroxy-octa-(éthylèneoxy)-carbonyl)-éthylphényl]-benzotriazole.

13. Composition selon la revendication 9, qui contient de plus un antioxydant de type phosphite ou de type phosphonite.

14. Composition selon la revendication 1, qui contient de plus un antioxydant de type phénol encombré.

15. Composition de revêtement selon la revendication 1, qui est un système durcissable à la température ambiante à base d'une résine alkyde, d'une résine thermoplastique acrylique, d'une résine alkyde acrylique, d'une résine polyuréthanne ou d'une résine polyester ou desdites résines modifiées par des silicones, isocyanates, époxydes, isocyanurates, cétiurines ou oxazolidones, ou un système à base d'un ester de cellulose ou d'une résine époxyde.

16. Composition de revêtement selon la revendication 1, qui est un système thermodurcissable catalysé par acide, à base d'une résine alkyde, polyamide, polyuréthanne, polyester ou acrylique réticulable à chaud.

17. Composition selon la revendication 1, qui est un vernis-émail pour couches de finition industrielles.

18. Composition selon la revendication 1, qui est un vernis-émail pour retouches pour automobiles.
